# EUROPÄISCHE PATENTSCHRIFT

(11) **EP 0 733 680 B1**
(45) Veröffentlichungstag und Bekanntmachung des Hinweises auf die Patenterteilung: **05.01.2000**
(21) Anmeldenummer: 96104230.6
(22) Anmeldetag: 16.03.1996
(51) Int. Cl.: C09B 62/503, C09B 62/08, C07D 209/46

(54) **Reaktivfarbstoffe mit einem benzoanellierten Heterocyclus als Anker**
Reactive dyes with a benzo-anellated heterocycle as reactive group
Colorants réactifs ayant un hétérocycle benzo-condensé comme groupe réactif

(30) Priorität: 24.03.1995 DE 19510888
(43) Veröffentlichungstag der Anmeldung: 25.09.1996
(73) Patentinhaber: BASF AKTIENGESELLSCHAFT, 67056 Ludwigshafen (DE)
(72) Erfinder: Patsch, Manfred, Dr., 67157 Wachenheim (DE); Löffler, Hermann, 67346 Speyer (DE); Marschner, Claus, Dr., 67346 Speyer (DE)

(56) Entgegenhaltungen:
- EP-A- 0 680 951
- EP-A- 0 685 464
- EP-A- 0 688 832
- GB-A- 1 113 370

## Beschreibung

Die vorliegende Erfindung betrifft neue Reaktivfarbstoffe der Formel I 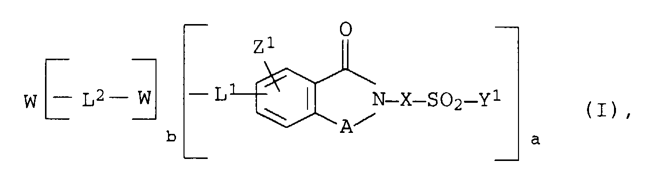 in der
- a: 1 oder 2,
- b: 0 oder 1,
- Z¹: Wasserstoff, C₁-C₆-Alkyl, C₁-C₆-Alkoxy, Halogen, Nitro, Amino, Hydroxysulfonyl, C₁-C₆-Alkoxycarbonyl, Carbamoyl oder C₁-C₆-Mono- oder Dialkylcarbamoyl,
- A: Methylen, Sulfonyl oder einen Rest der Formel CH₂-CO oder CH₂-SO₂, wobei die Methylengruppe jeweils an den Benzolring geknüpft ist,
- X: eine direkte Bindung, C₁-C₈-Alkylen, das gegebenenfalls durch 1 bis 3 Sauerstoffatome in Etherfunktion, 1 bis 3 Iminogruppen oder 1 bis 3 C₁-C₄-Alkyliminogruppen unterbrochen ist, oder einen Rest der Formel L³-CO-NZ²-L⁴, worin L³ und L⁴ unabhängig voneinander jeweils für C₁-C₄-Alkylen und Z² für Wasserstoff, C₁-C₆-Alkyl oder Phenyl stehen, und
- Y¹: Vinyl oder einen Rest der Formel C₂H₄Q, worin Q für eine unter alkalischen Reaktionsbedingungen abspaltbare Gruppe steht,
- W: im Fall 1) den Rest eines Chromophors, der gegebenenfalls weitere reaktive Gruppen aufweist und der sich von einem gegebenenfalls metallisierten Mono- oder Disazofarbstoff, einem Triphendioxazin, einem Anthrachinon, einem metallisierten Formazan oder einem metallisierten Phthalocyanin ableitet, oder
im Fall 2) den Rest einer Kupplungskomponente, an den gegebenenfalls zusätzlich der Rest einer Diazokomponente über eine Azobrücke gebunden ist und der gegebenenfalls zusätzliche reaktive Gruppen aufweist,
- L¹: im Fall 1) ein Brückenglied der Formel

O₂S-NZ³, OC-NZ³, Z³N-O₂S, Z³N-OC, Z³N-OC-NZ⁴, NZ³

oder 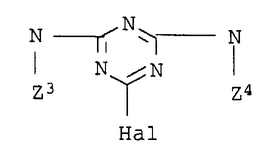 worin Z³ und Z⁴ unabhängig voneinander jeweils für Wasserstoff, C₁-C₆-Alkyl oder Phenyl und Hal für Fluor, Chlor oder Brom oder NZ³ oder NZ⁴ auch für Piperazin-1,4-diyl stehen, oder
im Fall 2) eine Azobrücke und
- L²: einen Rest der Formel 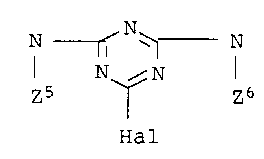 oder 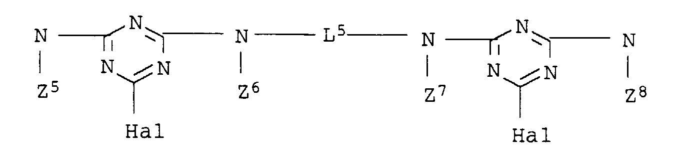 bedeuten, worin Z⁵, Z⁶, Z⁷ und Z⁸ unabhängig voneinander jeweils für Wasserstoff, C₁-C₆-Alkyl oder Phenyl und L⁵ für C₂-C₈-Alkylen oder gegebenenfalls durch C₁-C₄-Alkyl, C₁-C₄-Alkoxy oder Hydroxysulfonyl substituiertes Phenylen stehen und Hal jeweils die obengenannte Bedeutung besitzt,
deren Zwischenprodukte sowie ein Verfahren zum Färben oder Bedrucken von Hydroxygruppen oder Stickstoffatome aufweisenden Substraten mittels der neuen Farbstoffe.

Die EP-A-688 832 lehrt Reaktivfarbstoffe mit einem Phthalimidanker. Amine als Zwischenprodukte dieser Farbstoffe werden in der EP-A-685 464 offenbart.

Aufgabe der vorliegenden Erfindung war es, neue Reaktivfarbstoffe mit vorteilhaften anwendungstechnischen Eigenschaf.ten bereitzustellen. Die neuen Farbstoffe sollten sich insbesondere für das Auszieh- und Kaltverweilverfahren eignen und sollten sich vor allem durch hohe Ausgiebigkeit, hohe Naßechtheiten und brillante Färbungen auszeichnen. Außerdem sollten die nicht auf der Faser fixierten Anteile leicht auswaschbar sein.

Demgemäß wurden die eingangs näher bezeichneten Reaktivfarbstoffe der Formel I gefunden.

Alle in der obengenannten Formel auftretenden Alkyl- und Alkylengruppen können sowohl geradkettig als auch verzweigt sein.

Reste Z¹, Z², Z³, Z⁴, Z⁵, Z⁶, Z⁷ und Z⁸ sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl oder 2-Methylpentyl.

Reste Z¹ sind weiterhin z.B.Methoxy, Ethoxy, Propoxy, Isopropoxy, Butoxy, Isobutoxy, sec-Butoxy, tert-Butoxy, Pentyloxy, Isopentyloxy, Neopentyloxy, tert-Pentyloxy, Hexyloxy, 2-Methylpentyloxy, Fluor, Chlor, Brom, Methoxycarbonyl, Ethoxycarbonyl, Propoxycarbonyl, Isopropoxycarbonyl, Butoxycarbonyl, Isobutoxycarbonyl, sec-Butoxycarbonyl, Pentyloxycarbonyl, Isopentyloxycarbonyl, Neopentyloxycarbonyl, tert-Pentyloxycarbonyl, Hexyloxycarbonyl, Mono- oder Dimethylcarbamoyl, Mono- oder Diethylcarbamoyl, Mono- oder Dipropylcarbamoyl, Mono- oder Diisopropylcarbamoyl, Mono- oder Dibutylcarbamoyl, Mono- oder Dipentylcarbamoyl, Mono- oder Dihexylcarbamoyl oder N-Methyl-N-ethylcarbamoyl.

Reste X, L³, L⁴ und L⁵ sind z.B. (CH₂)₂, (CH₂)₃, (CH₂)₄, CH(CH₃)CH₂ oder CH(CH₃)CH(CH₃).

Reste X und L⁵ sind weiterhin z.B. (CH₂)₅, (CH₂)₆, (CH₂)₇ oder (CH₂)₈.

Reste X, L³ und L⁴ sind weiterhin z.B. Methylen.

Reste L⁵ sind weiterhin z.B. 1,2-, 1,3- oder 1,4-Phenylen, das jeweils ein- oder zweifach durch Methyl, Methoxy oder Hydroxysulfonyl substituiert sein kann,

Reste X sind weiterhin z.B.
C₂H₄-O-C₂H₄, C₂H₄-NH-C₂H₄, C₂H₄-N(CH₃)-C₂H₄, C₂H₄-O-C₂H₄-O-C₂H₄, C₂H₄-NH-C₂H₄-NH-C₂H₄, C₂H₄-N(CH₃)-C₂H₄-N(CH₃)-C₂H₄, C₂H₄-O-C₂H₄-N(CH₃)-C₂H₄, C₂H₄-O-C₂H₄-O-C₂H₄-O-C₂H₄, C₂H₄-NH-C₂H₄-NH-C₂H₄-NH-C₂H₄, C₂H₄-N(CH₃)-C₂H₄-N(CH₃)-C₂H₄-N(CH₃)-C₂H₄, C₂H₄-CO-NH-C₂H₄, C₂H₄-CO-N(CH₃)-C₂H₄, C₃H₆-CO-NH-C₂H₄, C₂H₄-CO-NH-C₃H₆, C₃H₆-CO-NH-C₃H₆ oder C₃H₆-CO-N(CH₃)-C₃H₆.

Der Rest Q steht für eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(D)(DH)₂, C₁-C₄-Alkylsulfonyloxy, Phenylsulfonyloxy, C₁-C₄-Alkanoyloxy, C₁-C₄-Dialkylamino oder ein Rest der Formel 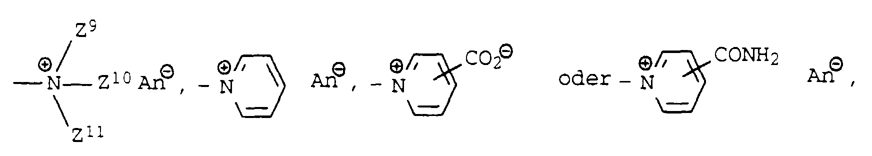 wobei Z⁹, Z¹⁰ und Z¹¹ gleich oder verschieden sind und unabhängig voneinander jeweils die Bedeutung von C₁-C₄-Alkyl oder Benzyl und An^{⊖} jeweils die Bedeutung eines Äquivalents eines Anions besitzen. Als Anionen können dabei z.B. Fluorid, Chlorid, Bromid, Iodid, Mono-, Di- oder Trichloracetat, Methylsulfonat, Phenylsulfonat oder 2- oder 4-Methylphenylsulfonat in Betracht kommen.

Wenn a 2 bedeutet, können die Reste 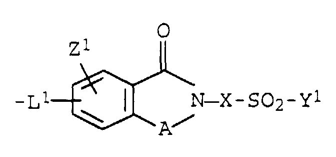 gleich oder verschieden sein.

Wenn b 1 bedeutet, können die Reste W ebenfalls gleich oder verschieden sein.

Der faserreaktive Ankerrest der Formel II 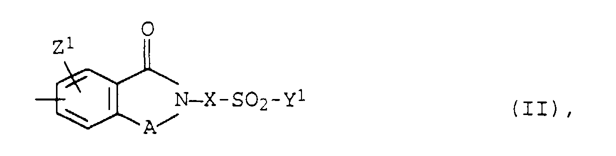 In der A, X, Y¹ und Z- jeweils die obengenannte Bedeutung besitzen, wird im folgenden als "E" bezeichnet.

Bevorzugt sind Reaktivfarbstoffe der Formel I, in der A Methylen oder den Rest der Formel CH₂-CO, insbesondere Methylen, bedeutet.

Bevorzugt sind weiterhin Reaktivfarbstoffe der Formel I, in der X C₁-C₈-Alkylen, das gegebenenfalls durch 1 bis 3 Sauerstoffatome in Etherfunktion unterbrochen ist, bedeutet, wobei solche Reaktivfarbstoffe der Formel I, in der X C₂- bis C₄-Alkylen oder einen Rest der Formel X¹-O-X² bedeutet, worin X¹ und X² unabhängig voneinander jeweils für C₂- oder C₃-Alkylen stehen, von besonderer Bedeutung sind.

Bevorzugt sind weiterhin Reaktivfarbstoffe der Formel I, in der Z¹ Wasserstoff bedeutet.

Neben dem Ankersystem E kann der Rest W noch weitere faserreaktive Reste tragen. Solche Reste sind z.B. heterocyclische Ankerreste oder Ankerreste aus der aliphatischen Reihe.

Heterocyclische Ankerreste sind z.B. halogenhaltige Reste, die sich von folgenden heterocyclischen Grundkörpern ableiten: 1,3,5-Triazin, Chinoxalin, Phthalazin, Pyrimidin oder Pyridazin, oder 2-Alkylsulfonylbenzthiazol.

Besonders seien folgende heterocyclische Reste genannt: 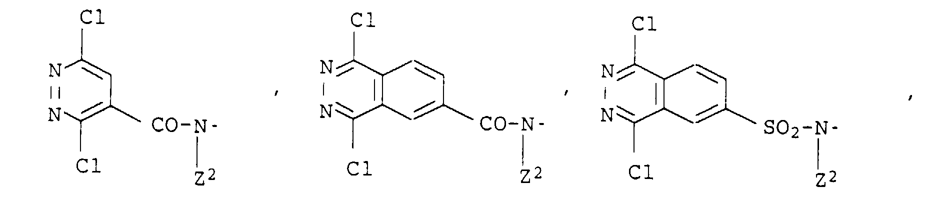 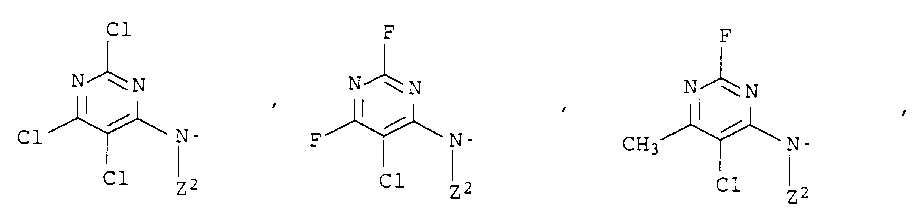 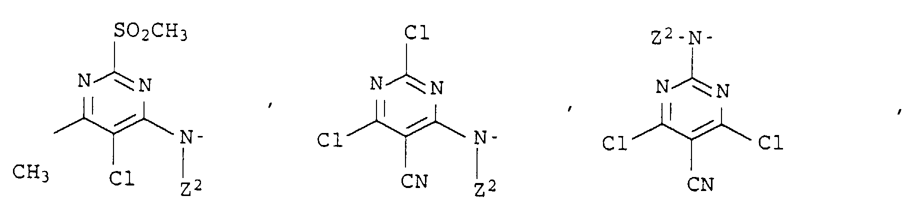 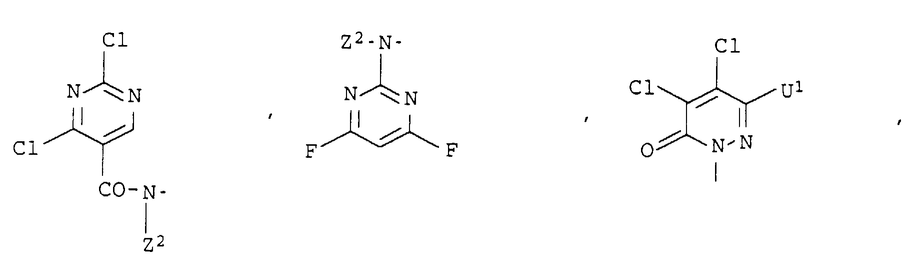 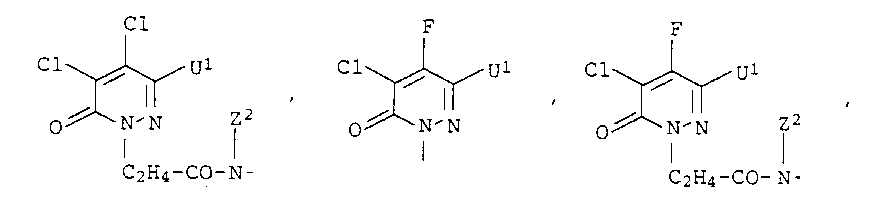 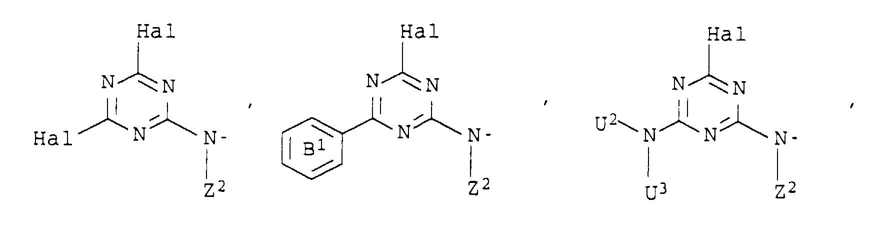 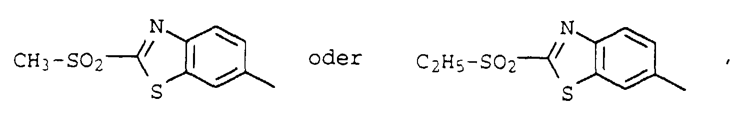 worin
- Z²: und Hal jeweils die obengenannte Bedeutung besitzen und
- U¹: Wasserstoff oder Nitro und
- U² und U³: unabhängig voneinander jeweils Wasserstoff, C₁-C₆-Alkyl, das gegebenenfalls durch Hydroxy, Halogen, Cyano, Hydroxysulfonyl oder einen Rest der Formel -SO₂-Y¹, worin Y¹ die obengenannte Bedeutung besitzt, substituiert ist und jeweils durch 1 oder 2 Sauerstoffatome in Etherfunktion, Imino- oder C₁-C₄-Alkyliminogruppen unterbrochen sein kann, oder U² und U³ zusammen mit dem sie verbindenden Stickstoffatom, Pyrrolidinyl, Piperidinyl, Morpholinyl, Piperazinyl oder N-(C₁-C₄-Alkyl)piperazinyl oder U² auch einen Rest der Formel 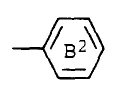 bedeuten,
wobei die Ringe B¹ und B² jeweils ein- oder zweifach durch Hydroxysulfonyl substituiert und benzoanelliert sein können und der Ring B² davon unabhängig ein- oder zweifach durch Chlor, Nitro, C₁-C₄-Alkyl, C₁-C₄-Alkoxy, Cyano, Carboxyl, Acetylamino, Hydroxysulfonylmethyl oder einen Rest der Formel CH₂-SO₂-Y¹, SO₂-Y¹, NH-CO-Y¹ oder NU²-CO-NU²-L⁰-SO₂-Y¹, worin Y¹ und U² jeweils die obengenannte Bedeutung besitzen und L⁰ für C₂-C₆-Alkylen, das gegebenenfalls durch Hydroxy, Chlor, Cyano, Carboxyl, C₁-C₄-Alkoxycarbonyl, C₁-C₄-Alkanoyloxy oder Sulfato substituiert ist und durch jeweils 1 oder 2 Sauerstoffatome in Etherfunktion oder Imino- oder C₁-C₄-Alkyliminogruppen unterbrochen sein kann, steht, substituiert sein kann.

Ankerreste aus der aliphatischen Reihe sind beispielsweise Acryloyl-, Mono-, Di- oder Trichloracryloyl, Mono-, Di- oder Tribromacryloyl, -CO-CCl=CH-COOH, -CO-CH=CCl-COOH, 2-Chlorpropionyl, 1,2-Dichlorpropionyl, 1,2-Dibrompropionyl, 3-Phenylsulfonylpropionyl, 3-Methylsulfonylpropionyl, 2-Sulfatoethylaminosulfonyl, 2-Chlor-2,3,3-trifluorcyclobutylcarbonyl, 2,2,3,3-Tetrafluorcyclobutylcarbonyl, 2,2,3,3-Tetrafluorcyclobutylsulfonyl, 2-(2,2,3,3-Tetrafluorcyclobutyl)acryloyl, 1- oder 2-Alkyl- oder 1- oder 2-Arylsulfonylacryloyl, wie 1- oder 2-Methylsulfonylacryloyl, oder ein Rest der Formel SO₂-Y¹, CONH-L⁶-SO₂-Y¹ oder NHCONH-L⁶-SO₂-Y¹, worin Y¹ die obengenannte Bedeutung besitzt und L⁶ für C₁-C₄-Alkylen oder Phenylen steht.

W in Formel I stellt z.B. den Rest einer Kupplungskomponente dar, an den gegebenenfalls zusätzlich der Rest einer Diazokomponente über eine Azobrücke gebunden ist und der gegebenenfalls zusätzliche reaktive Gruppen aufweist. In diesem Fall ist der Ankerrest E über eine Azobrücke (-N=N-) an den Rest W geknüpft.

Farbstoffe dieser Klasse gehorchen z.B. der Formel IIIa oder IIIb

(E-N=N-)ₐK (IIIa)

E-N=N-K-N=N-D (IIIb),

worin K den Rest einer Kupplungskomponente, D den Rest einer Diazokomponente und a 1 oder 2 bedeuten und E die obengenannte Bedeutung besitzt. Wenn in Formel IIIa der Rest E doppelt auftritt (a=2), können die Reste E entweder gleich oder verschieden voneinander sein.

Wertvolle Farbstoffe dieser Klasse sind z.B. wasserlösliche Azofarbstoffe, insbesondere Monoazofarbstoffe der Formel IIIa (a=1) oder Disazofarbstoffe der Formel IIIa (a=2) oder IIIb, die Hydroxysulfonyl- und/oder Carboxylgruppen aufweisen.

Wichtige Kupplungskomponenten HK leiten sich z.B. von Verbindungen aus der Benzol-, Naphthalin-, Pyrazol-, Pyridin-, Pyrimidin-, Indol- oder N-Arylacetoacetamidreihe ab.

Wichtige Diazokomponenten D-NH₂ leiten sich z.B. von Verbindungen aus der Anilin- oder Aminonaphthalinreihe ab.

Besonders bevorzugt sind Farbstoffe der Formel IV

E-N=N-K¹ (IV),

in der E die obengenannte Bedeutung besitzt und K¹ für den Rest einer Kupplungskomponente der Benzol-, Naphthalin-, Pyrazol- oder Pyridinreihe steht, der gegebenenfalls weitere faserreaktive Gruppen aufweist, insbesondere die Gruppe E, die Gruppe der Formel SO₂-Y¹, worin Y¹ die obengenannte Bedeutung besitzt, oder solche der Halogentriazinreihe.

Weiterhin besonders bevorzugt sind Farbstoffe der Formel V 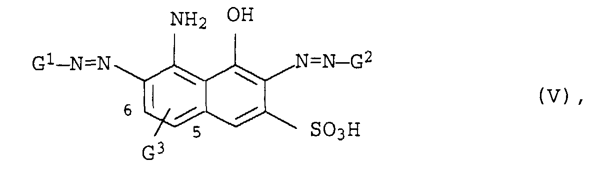 in der einer der beiden Reste G¹ und G² für den Rest E, wobei dieser die obengenannte Bedeutung besitzt, und der andere für den Rest D¹, wobei dieser die Bedeutung eines Rest einer Diazokomponente der Anilin- oder Naphthalinreihe besitzt, der gegebenenfalls weitere faserreaktive Gruppen, insbesondere die Gruppe E, die Gruppe der Formel SO₂-Y¹, worin Y¹ die obengenannte Bedeutung besitzt, oder solche der Halogentriazinreihe aufweist, und G³ für Hydroxysulfonyl in Ringposition 5 oder 6 stehen.

W in Formel I stellt weiterhin z.B. den gegebenenfalls metallisierten Rest eines Azofarbstoffs dar. Geeignete Azofarbstoffe, von denen sich solche Reste ableiten, sind an sich bekannt und in großer Zahl beschrieben, z.B. in K. Venkataraman "The Chemistry of Synthetic Dyes", Vol. VI, Academic Press, New York, London, 1972. Die Azofarbstoffe gehorchen der Formel VI

D-N=N-K(-N=N-D)₁ (VI),

in der D den Rest einer Diazokomponente, K den Rest einer Kupplungskomponente und 1 0 oder 1 bedeuten, worin, wenn 1 1 ist, die Reste D gleich oder verschieden voneinander sind.

Wertvolle Farbstoffe, von denen sich der Rest W ableitet, sind z.B. wasserlösliche Azofarbstoffe, insbesondere Monoazofarbstoffe der Formel VI (1 = 0), die Hydroxysulfonyl- und/oder Carboxylgruppen aufweisen können.

Bevorzugt leitet sich der Rest W von nichtmetallisierten Azofarbstoffen ab, insbesondere von solchen, die Sulfonsäure- und/oder Carboxylgruppen enthalten, wobei jene, die 1 bis 6 Sulfonsäuregruppen aufweisen, besonders hervorzuheben sind.

Wichtige Azofarbstoffe, von denen sich der Rest W ableitet, sind beispielsweise solche der Phenyl-azo-naphthalin-, Phenyl-azo-1-phenylpyrazol-5-on-, Phenyl-azo-benzol-, Naphthyl-azo-benzol-, Phenyl-azo-aminonaphthalin-, Naphthyl-azo-naphthalin-, Naphthyl-azo-1-phenylpyrazol-5-on-, Phenyl-azo-pyridon-, Phenyl-azo-amino-pyridin-, Naphthyl-azo-pyridon-, Naphthyl-azo-aminopyridin- oder Stilbyl-azo-benzolreihe.

Besonders bevorzugt sind Reaktivfarbstoffe der Formel VII 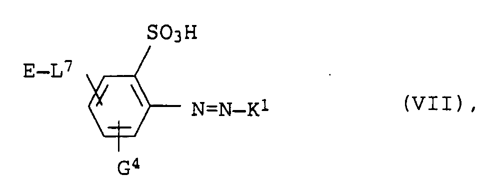 in der E und K¹ jeweils die obengenannte Bedeutung besitzen,

L⁷ einen Rest der Formel O₂S-NZ³, OC-NZ³, Z³N-O₂S, Z³N-OC, Z³N-OC-NZ⁴, NZ³ oder 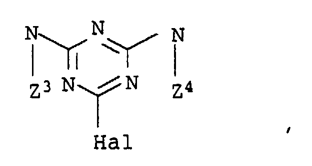 worin Z³, Z⁴ und Hal jeweils die obengenannte Bedeutung besitzen und G⁴ Wasserstoff, C₁-C₄-Alkyl, C₁-C₄-Alkoxy, Chlor oder Hydroxysulfonyl bedeuten.

Weiterhin besonders bevorzugt sind Reaktivfarbstoffe der Formel VIII 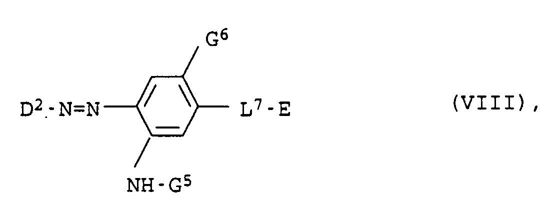 in der E und L⁷ jeweils die obengenannte Bedeutung besitzen, G⁵ für C₁-C₄-Alkanoyl, Carbamoyl, C₁-C₄-Mono- oder Dialkylcarbamoyl, Phenylcarbamoyl oder Cyclohexylcarbamoyl, G⁶ für Wasserstoff, C₁-C₄-Alkyl, C₁-C₄-Alkoxy, Hydroxysulfonyl oder Chlor und D² für den Rest einer Diazokomponente der Anilin- oder Naphthalinreihe, der keine weitere faserreaktive Gruppe trägt, stehen.

Weiterhin besonders bevorzugt sind Reaktivfarbstoffe der Formel IX 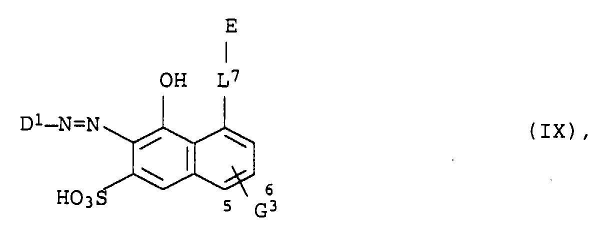 in der D¹, E und L⁷ jeweils die obengenannte Bedeutung besitzen und G³ für Hydroxysulfonyl in Ringposition 5 oder 6 steht.

Weiterhin besonders bevorzugt sind Reaktivfarbstoffe der Formel X 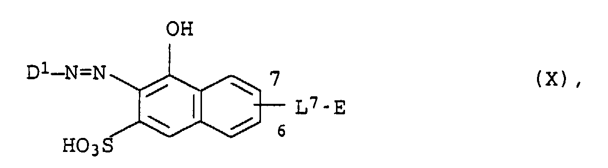 in der D¹, E und L⁷ jeweils die obengenannte Bedeutung besitzen und die Gruppe -L⁷-E in Ringposition 6 oder 7 steht.

Weiterhin wertvolle Verbindungen sind solche der Formel XI 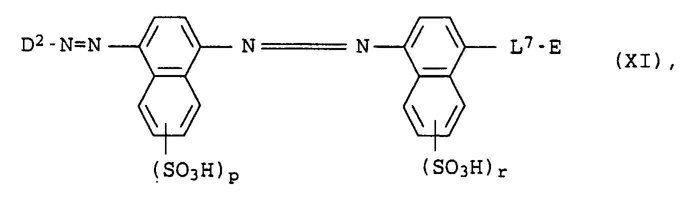 in der D², E und L⁷ jeweils die obengenannte Bedeutung besitzen und p und r unabhängig voneinander jeweils für 0,1 oder 2 stehen.

Weiterhin sind wertvolle Verbindungen solche der Formel XII 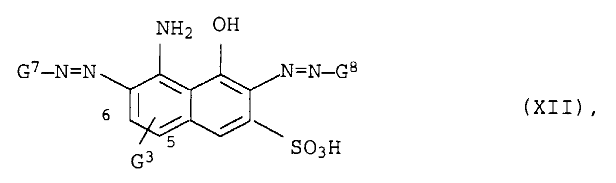 in der G³ die obengenannte Bedeutung besitzt und einer der beiden Reste G⁷ und G⁸ für den Rest D¹, wobei dieser die obengenannte Bedeutung besitzt,
und der andere für den Rest 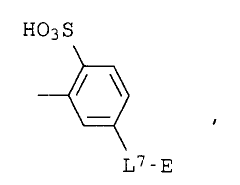 oder auch beide Reste G⁷ und G⁸ für den Rest 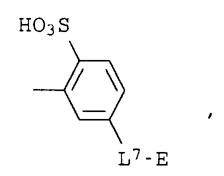 wobei L⁷ und E jeweils die obengenannte Bedeutung besitzen, stehen.

Solche aromatischen Reste D¹ und D² von Diazokomponenten der Anilin- oder Aminonaphthalinreihe, die keine faserreaktiven Gruppen tragen, leiten sich beispielsweise von Aminen der Formeln XIII a-f 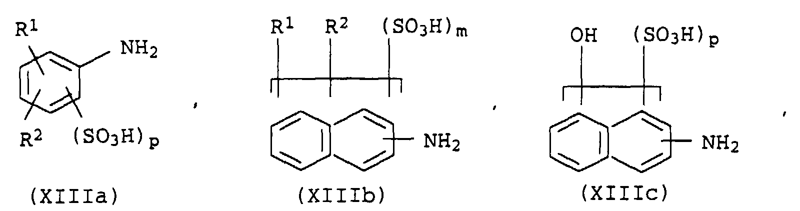 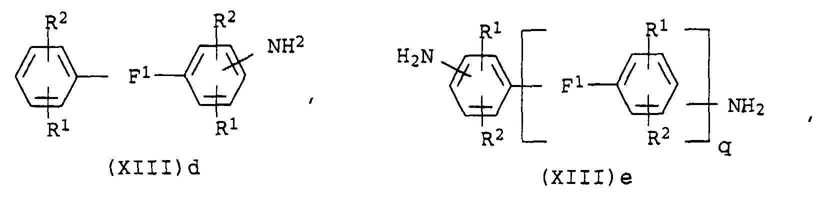 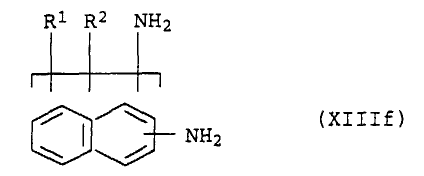
- ab,: worin
- m: 0, 1, 2 oder 3,
- p: 0, 1 oder 2,
- q: 0 oder 1,
- R¹: Wasserstoff, C₁-C₄-Alkyl, C₁-C₄-Alkoxy, Acetyl, Cyano, Carboxyl, Hydroxysulfonyl, C₁-C₄-Alkoxycarbonyl, Hydroxy, Carbamoyl, C₁-C₄-Mono- oder Dialkylcarbamoyl, Fluor, Chlor, Brom oder Trifluormethyl,
- R²: Wasserstoff, C₁-C₄-Alkyl, C₁-C₄-Alkoxy, Cyano, Carboxyl, Hydroxysulfonyl, Acetylamino, C₁-C₄-Alkoxycarbonyl, Carbamoyl, C₁-C₄-Mono- oder Dialkylcarbamoyl, Fluor, Chlor, Nitro, Sulfamoyl, C₁-C₄-Mono- oder Dialkylsulfamoyl, C₁-C₄-Alkylsulfonyl, Phenylsulfonyl oder Phenoxy und
- F¹: eine direkte Bindung, Sauerstoff, Schwefel oder einen Rest der Formel -NHCO-, -NHCONH-, -CONH-, -CO-, -NHSO₂-, -SO₂NH-, -SO₂-, -CH=CH-, -CH₂-CH₂-, -CH₂-, -NH-, oder -N=N- bedeuten.

Bevorzugt sind dabei solche Komponenten, in denen R¹ Wasserstoff, Methyl, Methoxy, Carboxyl, Hydroxysulfonyl, Hydroxy oder Chlor, R² Wasserstoff, Methyl, Methoxy, Carboxyl, Hydroxysulfonyl, Acetylamino oder Chlor und F¹ einen Rest der Formel -CO-, -SO₂-, -CH=CH-, -CH₂-CH₂-, -CH₂- oder -N=N- bedeuten.

Aromatische Amine, die sich als Diazokomponenten eignen und die der Formel XIIIa, XIIIb, XIIIc oder XIIId entsprechen, sind beispielsweise Anilin, 2-Methoxyanilin, 2-Methylanilin, 4-Chlor-2-aminoanisol, 4-Methylanilin, 4-Methoxyanilin, 2-Methoxy-5-methylanilin, 2,5-Dimethoxyanilin, 2,5-Dimethylanilin, 2,4-Dimethylanilin, 2,5-Diethoxyanilin, 2-Chloranilin, 3-Chloranilin, 4-Chloranilin, 2,5-Dichloranilin, 4-Chlor-2-nitroanilin, 4-Chlor-2-methylanilin, 3-Chlor-2-methylanilin, 4-Chlor-2-aminotoluol, 4-Phenylsulfonylanilin, 2-Ethoxy-1-naphthylamin, 1-Naphthylamin, 2-Naphthylamin, 4-Methylsulfonylanilin, 2,4-Dichloranilin-5-carbonsäure, 2-Aminobenzoesäure, 4-Aminobenzoesäure, 3-Aminobenzoesäure, 3-Chloranilin-6-carbonsäure, Anilin-2- oder -3- oder -4-sulfonsäure, Anilin-2,5-disulfonsäure, Anilin-2,4-disulfonsäure, Anilin-3,5-disulfonsäure, 2-Aminotoluol-4-sulfonsäure, 2-Aminoanisol-4-sulfonsäure, 2-Aminoanisol-5-sulfonsäure, 2-Ethoxyanilin-5-sulfonsäure, 2-Ethoxyanilin-4-sulfonsäure, 4-Hydroxysulfonyl-2-aminobenzoesäure, 2,5-Dimethoxyanilin-4-sulfonsäure, 2,4-Dimethoxyanilin-5-sulfonsäure, 2-Methoxy-5-methylanilin-4-sulfonsäure, 4-Aminoanisol-3-sulfonsäure, 4-Aminotoluol-3-sulfonsäure, 2-Aminotoluol-5-sulfonsäure, 2-Chloranilin-4-sulfonsaure, 2-Chloranilin-5-sulfonsäure, 2-Bromanilin-4-sulfonsäure, 2,6-Dichloranilin-4-sulfonsäure, 2,6-Dimethylanilin-3- oder -4-sulfonsäure, 3-Acetylaminoanilin-6-sulfonsäure, 4-Acetylaminoanilin-2-sulfonsäure, 1-Aminonaphthalin-4-sulfonsäure, 1-Aminonaphthalin-3-sulfonsäure, 1-Aminonaphthalin-5-sulfonsäure, 1-Aminonaphthalin-6-sulfonsäure, 1-Aminonaphthalin-7-sulfonsäure, 1-Aminonaphthalin-3,7-disulfonsäure, 1-Aminonaphthalin-3,6,8-trisulfonsäure, 1-Aminonaphthalin-4,6,8-trisulfonsäure, 2-Napththylamin-5- oder -6- oder -8-sulfonsäure, 2-Aminonaphthalin-3,6,8-trisulfonsäure, 2-Aminonaphthalin-6,8-disulfonsäure, 2-Aminonaphthalin-1,6-disulfonsäure, 2-Aminonaphthalin-1-sulfonsäure, 2-Aminonaphthalin-1,5-disulfonsäure, 2-Aminonaphthalin-3,6-disulfonsäure, 2-Aminonaphthalin-4,8-disulfonsäure, 2-Aminophenol-4-sulfonsäure, 2-Aminophenol-5-sulfonsäure, 3-Aminophenol-6-sulfonsäure, 1-Hydroxy-2-aminonaphthalin-5,8-oder -4,6-disulfonsäure, 4-Aminodiphenylamin, 4-Amino-4'-methoxydiphenylamin, 4-Amino-4'-methoxydiphenylamin-3-sulfonsäure, 4-(2'-Methylphenylazo)-2-methylanilin, 4-Aminoazobenzol, 4'-Nitrophenylazo-1-aminonaphthalin, 4-(6'-Hydroxysulfonylnaphthylazo)-1-aminonaphthalin, 4-(2',5'-Dihydroxysulfonylphenylazo)-1-aminonaphthalin, 4'-Amino-3'-methyl-3-nitrobenzophenon, 4-Aminobenzophenon, 4-(4'-Aminophenylazo)benzolsulfonsäure, 4-(4'-Amino-3'-methoxyphenylazo)benzolsulfonsäure oder 2-Ethoxy-1-naphthylamin-6-sulfonsäure.

Aromatische Diamine, die sich als Tetraazokomponenten oder auch der Verdoppelung (z.B. mit Cyanurchlorid) eignen und die der Formel XIIIe oder XIIIf entsprechen, sind beispielsweise 1,3-Diaminobenzol, 1,3-Diaminobenzol-4-sulfonsäure, 1,4-Diaminobenzol, 1,4-Diaminobenzol-2-sulfonsäure, 1,4-Diamino-2-methylbenzol, 1,4-Diamino-2-methoxybenzol, 1,3-Diamino-4-methylbenzol, 1,3-Diaminobenzol-5-sulfonsäure, 1,3-Diamino-5-methylbenzol, 1,6-Diaminonaphthalin-4-sulfonsäure, 2,6-Diaminonaphthalin-4,8-disulfonsäure, 3,3'-Diaminodiphenylsulfon, 4,4'-Diaminodiphenylsulfon, 4,4'-Diaminostilben-2,2'-disulfonsäure, 2,2-Diaminodiphenylsulfon, 2,2'-Diaminodiphenylsulfon-4,5-disulfonsäure, 4,4'-Diaminobenzophenon, 4,4'-Diamino-3,3'-dinitrobenzophenon, 3,3'-Diamino-4,4'-dichlorbenzophenon, 4,4'- oder 3,3'-Diaminodiphenyl, 4,4'-Diamino-3,3'-dichlordiphenyl, 4,4'-Diamino-3,3'-dimethoxy- oder -3,3'-dimethyl- oder -2,2'-dimethyl- oder -2,2'-dichlor- oder -3,3'-diethoxydiphenyl, 4,4'-Diamino-3,3'-dimethyl-6,6'-dinitrodiphenyl, 4,4'-Diaminodiphenyl-2,2'- oder -3,3'-disulfonsäure, 4,4'-Diamino-3,3'-dimethyl- oder -3,3'-dimethoxy oder -2,2'-dimethoxydiphenyl-6,6'-disulfonsäure, 4,4'-Diamino-2,2', 5,5'-tetrachlordiphenyl, 4,4'-Diamino-3,3'-dinitrodiphenyl, 4,4'-Diamino-2,2'-dichlor-5,5'-dimethoxydiphenyl, 4,4'-Diaminodiphenyl-2,2'- oder -3,3'-dicarbonsäure, 4,4'-Diamino-3,3'-dimethyldiphenyl-5,5'-disulfonsäure, 4,4'-Diamino-2-nitrodiphenyl, 4,4'-Diamino-3-ethoxy- oder -3-hydroxysulfonyldiphenyl, 4,4'-Diamino-3, 3'-dimethyldiphenyl-5-sulfonsäure, 4,4'-Diaminodiphenylmethan, 4,4'-Diamino-3,3'-dimethyldiphenylmethan, 4,4'-Diamino-2,2',3,3'-tetramethyldiphenylmethan, 4,4'-Diaminodiphenylethan, 4,4'-Diaminostilben oder 4,4'-Diaminodiphenylmethan-3,3'-dicarbonsäure.

Solche aromatischen Reste D¹ von Diazokomponenten aus der Anilin- oder Aminonaphthalinreihe, die weitere faserreaktive Reste tragen können, leiten sich beispielsweise von Aminen der Formeln XIVa-c 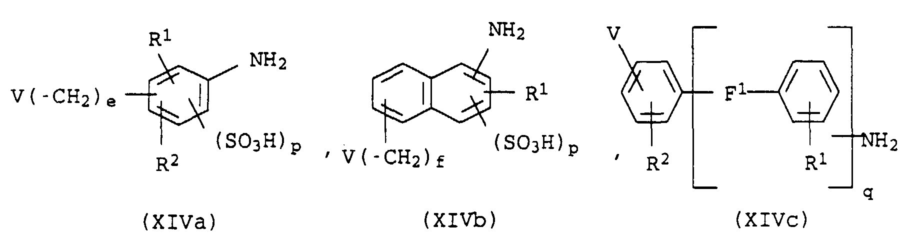 ab, in denen R¹, R², p, q und F¹ jeweils die obengenannte Bedeutung besitzen und e und f gleich oder verschieden sind und unabhängig voneinander jeweils 0 oder 1 und V einen faserreaktiven Rest bedeuten.

Faserreaktive Reste V leiten sich beispielsweise vom Rest E ab oder sind, wie oben bereits ausgeführt, heterocyclische Ankerreste oder Ankerreste aus der aliphatischen Reihe.

Aromatische Amine, die den, den faserreaktiven Rest V aufweisenden, Derivaten der Formel XIVa, XIVb oder XIVc zugrundeliegen, sind beispielsweise 1,3-Diaminobenzol, 1,3-Diaminobenzol-4-sulfonsäure, 1,3-Diaminobenzol-4,6-disulfonsäure, 1,4-Diaminobenzol, 1,4-Diaminobenzol-2-sulfonsäure, 1,4-Diaminobenzol-2,5-disulfonsäure, 1,4-Diamino-2-methylbenzol, 1,4-Diamino-2-methoxybenzol, 1,3-Diamino-4-methylbenzol, 1,4-Diaminobenzol-2,6-disulfonsäure, 1,5-Diamino-4-methylbenzol-2-sulfonsäure, 1,5-Diamino-4-methoxybenzol-2-sulfonsäure, 1,6-Diaminonaphth-2-ol-4-sulfonsäure, 1,6-Diaminonaphthalin-4-sulfonsäure, 2,6-Diaminonaphthalin-4,8-disulfonsäure, 2,6-Diaminonaphth-1-ol-4,8-disulfonsäure, 1,3-Diaminobenzol-5-sulfonsäure, 1,3-Diamino-5-methylbenzol, 2,6-Diaminophenol-4-sulfonsäure, 5-Aminomethyl-2-aminonaphthalin-1-sulfonsäure, 5-(N-Methylaminomethyl)-2-aminonaphthalin-1-sulfonsäure, 4,4-Diaminostilben-3,3-dicarbonsäure, 4-(N-Methylaminomethyl)anilin-2-sulfonsäure oder 3-(N-Methylaminomethyl)-anilin-6-sulfonsäure.

Die Reste K der Kupplungskomponente entstammen vorzugsweise der Benzol-, Naphthalin-, Pyrazol-, Pyridin-, Pyrimidin-, Indol- oder N-Arylacetoacetamidreihe und können auch faserreaktive Gruppen tragen.

Faserreaktivgruppenfreie Kupplungskomponenten der Anilin- oder Naphthalinreihe entsprechen beispielsweise den Verbindungen der Formeln XVa-g 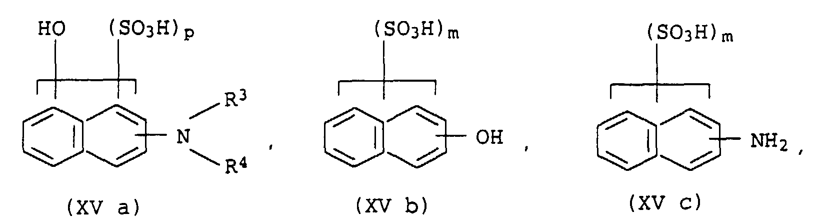 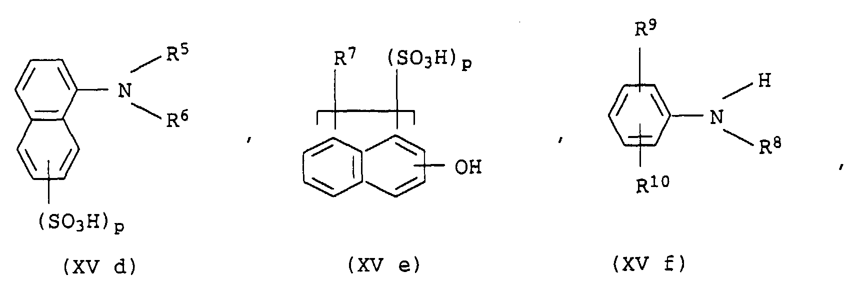 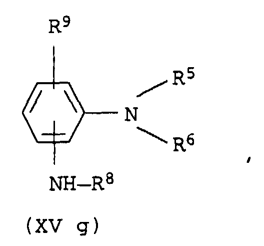 worin
- R³: Wasserstoff oder C₁-C₄-Alkyl,
- R⁴: Wasserstoff, C₁-C₄-Alkyl oder Phenyl, das durch C₁-C₄-Alkyl, C₁-C₄-Alkoxy, Chlor, Brom oder Hydroxysulfonyl ein- oder zweifach substituiert sein kann,
- R⁵: Wasserstoff oder C₁-C₄-Alkyl, das durch Hydroxy, Cyano, Carboxyl, Hydroxysulfonyl, Sulfato, Methoxycarbonyl, Ethoxycarbonyl oder Acetoxy substituiert sein kann,
- R⁶: Wasserstoff, C₁-C₄-Alkyl, das durch Hydroxy, Cyano, Carboxyl, Hydroxysulfonyl, Sulfato, Methoxycarbonyl, Ethoxycarbonyl oder Acetoxy substituiert sein kann, Benzyl oder Phenyl, das durch C₁-C₄-Alkyl, C₁-C₄-Alkoxy, Chlor oder Hydroxysulfonyl substituiert sein kann,
- R⁷: C₁-C₆-Alkylureido, Phenylureido, das durch Chlor, Methyl, Methoxy, Nitro, Hydroxysulfonyl oder Carboxyl substituiert sein kann, C₁-C₆-Alkanoylamino, das durch Hydroxysulfonyl oder Chlor substituiert sein kann, Cyclohexancarbonylamino, Benzoylamino, das durch Chlor, Methyl, Methoxy, Nitro, Hydroxylsulfonyl oder Carboxyl substituiert sein kann, oder Hydroxy,
- R⁸: Wasserstoff, C₁-C₆-Alkyl, insbesondere C₁-C₄-Alkyl, das jeweils durch Phenyl, C₁-C₄-Alkoxy, Hydroxy, Phenoxy oder C₁-C₄-Alkanoyloxy substituiert sein kann, C₅-C₇-Cycloalkyl, Hydroxysulfonylphenyl, C₁-C₄-Alkanoyl, Carbamoyl, C₁-C₄-Mono- oder Dialkylcarbamoyl, Phenylcarbamoyl oder Cyclohexylcarbamoyl,
- R⁹: C₁-C₄-Alkoxy, Chlor, Brom, Hydroxysulfonyl, C₁-C₄-Alkanoylamino, Amino, Ureido, Methylsulfonylamino, Ethylsulfonylamino, Dimethylaminosulfonylamino, Methylamino, Ethylamino, Dimethylamino oder Diethylamino und
- R¹⁰: Wasserstoff, C₁-C₄-Alkyl, C₁-C₄-Alkoxy, Hydroxysulfonyl, Chlor oder Brom bedeuten und
- p und m: jeweils die obengenannte Bedeutung besitzen.

Im einzelnen sind beispielsweise o- oder m-Toluidin, o- oder m-Anisidin, Kresidin, 2,5-Dimethylanilin, 2,5-Dimethoxyanilin, m-Aminoacetanilid, 3-Amino-4-methoxyacetanilid, 3-Amino-4-methylacetanilid, m-Aminophenylharnstoff, N-Methylanilin, N-Methyl-m-toluidin, N-Ethylanilin, N-Ethyl-m-toluidin, N-(2-Hydroxyethyl)anilin oder N-(2-Hydroxyethyl)-m-toluidin zu nennen.

Naphtholsulfonsäuren sind beispielsweise 1-Naphthol-3-sulfonsäure, 1-Naphthol-4-sulfonsäure, 1-Naphthol-5-sulfonsäure, 1-Naphthol-8-sulfonsäure, 1-Naphthol-3,6-disulfonsäure, 1-Naphthol-3,8-disulfonsäure, 2-Naphthol-5-sulfonsäure, 2-Naphthol-6-sulfonsäure, 2-Naphthol-7-sulfonsäure, 2-Naphthol-8-sulfonsäure, 2-Naphthol-3,6-disulfonsäure, 2-Naphthol-6,8-disulfonsäure, 2-Naphthol-3,6,8-trisulfonsäure, 1,8-Dihydroxynaphthalin-3,6-disulfonsäure, 2,6-Dihydroxynapthalin-8-sulfonsäure oder 2,8-Dihydroxynaphthalin-6-sulfonsäure.

Weiterhin sind beispielsweise 1-Naphthylamin, N-Phenyl-1-naphthylamin, N-Ethyl-1-naphthylamin, N-Phenyl-2-naphthylamin, 1-Naphthol, 2-Naphthol, 1,5-Dihydroxynaphthalin, 1,6-Dihydroxynaphthalin, 1,7-Dihydroxynaphthalin oder 2,7-Dihydroxynaphthalin zu nennen.

Aminonaphthalinsulfonsäuren sind beispielsweise 1-Naphthyl-amin-6-sulfonsäure, 1-Naphthylamin-7-sulfonsäure, 1-Naphthylamin-8-sulfonsäure, 2-Naphthylamin-3,6-disulfonsäure, 2-Naphthylamin-5,7-disulfonsäure oder 2-Naphthylamin-6,8-disulfonsäure.

Als Aminonaphtholsulfonsäuren sind z.B. 1-Amino-5-hydroxynaphthalin-7-sulfonsäure, 1-Amino-8-hydroxynaphthalin-4-sulfonsäure, 1-Amino-8-hydroxynaphthalin-2,4-disulfonsäure, 1-Amino-8-hydroxynaphthalin-3,6-disulfonsäure, 1-Amino-8-hydroxynaphthalin-4,6-disulfonsäure, 2-Amino-5-hydroxy- naphthalin-7-sulfonsäure, 2-Amino-8-hydroxynaphthalin-6-sulfonsäure, 2-Amino-8-hydroxynaphthalin-3,6-disulfonsäure, 2-Amino-5-hydroxynaphthalin-1,7-disulfonsäure, 1-Acetylamino-8-hydroxynaphthalin-3,6-disulfonsäure, 1-Benzoylamino-8-hydroxynaphthalin-3,6-disulfonsäure 1-Acetylamino-8-hydroxynaphthalin-4,6-disulfonsäure, 1-Benzoylamino-8-hydroxynaphthalin-4,6-disulfonsäure, 1-Acetylamino-5-hydroxynaphthalin-7-sulfonsäure, 2-Methylamino-8-hydroxynaphthalin-6-sulfonsäure oder 2-(3'-oder 4'-Hydroxysulfonylphenylamino)-8-hydroxynaphthalin-6-sulfonsäure zu nennen.

Von besonderer Bedeutung sind Kupplungskomponenten, die Sulfonsäure- und/oder Carboxylgruppen aufweisen und die in ortho- oder para-Stellung zu einer Hydroxy- und/oder Aminogruppe kuppeln.

Als Beispiele für solche Kupplungskomponenten seien 2-Acetylamino-5-hydroxynaphthalin-7-sulfonsäure, 2-Acetylamino-8-hydroxynaphthalin-6-sulfonsäure, 1-Acetylamino-8-hydroxynaphthalin-3,6-disulfonsäure, 1-Benzoylamino-8-hydroxynaphthalin-3,6-disulfonsäure, 1-Acetylamino-8-hydroxynaphthalin-4,6-disulfonsäure oder 1-Benzoylamino-8-hydroxynaphthalin-4,6-disulfonsäure genannt.

Kupplungskomponenten der weiteren Reihen sind beispielsweise: Pyrazolone, Aminopyrazole, 2,6-Diaminopyridine, Pyridone, Hydroxy- oder Aminopyrimidine, Indole oder N-Arylacetoacetamide.

Faserreaktivgruppenfreie Kupplungskomponenten dieser Reihe entsprechen beispielsweise den Formeln XVIa-f 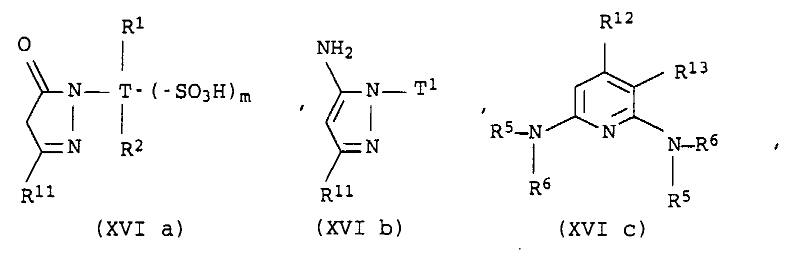 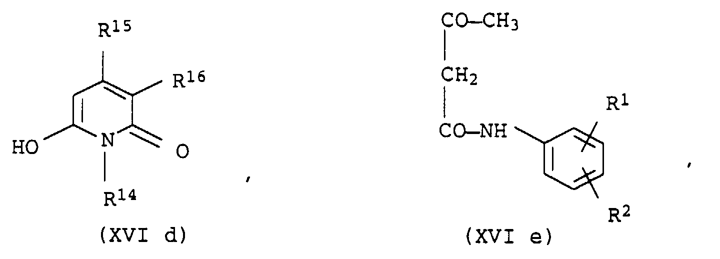 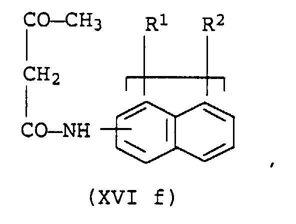 worin
- T: für den Rest eines Benzol- oder Naphthalinrings,
- T¹: für C₁-C₄-Alkyl, Cyclohexyl, Benzyl oder Phenyl, das ein- bis dreifach durch Fluor, Chlor, Brom, Methoxy, Nitro, Hydroxysulfonyl, Carboxyl, Acetyl, Acetylamino, Methylsulfonyl, Sulfamoyl oder Carbamoyl substituiert ist,
- R¹¹: für Methyl, Carboxyl, C₁-C₄-Alkoxycarbonyl oder Phenyl,
- R¹²: für Wasserstoff oder C₁-C₄-Alkyl, das durch Methoxy, Ethoxy oder Cyano substituiert sein kann,
- R¹³: für Wasserstoff, Methyl, Hydroxysulfonylmethyl, Hydroxysulfonyl, Cyano oder Carbamoyl,
- R¹⁴: für Wasserstoff, C₁-C₄-Alkyl, das durch Phenyl, Hydroxysulfonylphenyl, Hydroxy, Amino, Methoxy, Ethoxy, Carboxyl, Hydroxysulfonyl, Acetylamino, Benzoylamino oder Cyano substituiert sein kann, Cyclohexyl, Phenyl, das gegebenenfalls durch Carboxyl, Hydroxysulfonyl, Benzoylamino, Acetylamino, Methyl, Methoxy, Cyano oder Chlor substituiert ist, oder Amino, das durch Phenyl, C₁-C₄-Alkyl, C₁-C₄-Alkanoyl oder Benzoyl substituiert ist,
- R¹⁵: für C₁-C₄-Alkyl, Phenyl, Hydroxy, Cyano, Acetyl, Benzoyl, Carboxyl, Methoxycarbonyl, Carbamoyl oder Hydroxysulfonylmethyl und
- R¹⁶: für Wasserstoff, Chlor, Brom, Acetylamino, Amino, Nitro, Hydroxysulfonyl, Sulfamoyl, Methylsulfonyl, Phenylsulfonyl, Carboxyl, C₁-C₄-Alkoxycarbonyl, C₁-C₄-Alkanoyl, Benzoyl, Carbamoyl, Cyano oder Hydroxysulfonylmethyl stehen und R¹, R², R⁵, R⁶ und m jeweils die obengenannte Bedeutung besitzen.

Als Pyrazolon-Kupplungskomponenten sind beispielsweise 3-Methyl-, 3-Carboxy- oder 3-(C₁-C₄-Alkoxycarbonyl)pyrazol-5-one zu nennen, die in 1-Stellung Wasserstoff, gegebenenfalls durch Methyl, Ethyl, Fluor, Chlor, Brom, Trifluormethyl, Methoxy, Ethoxy, Cyano, Phenoxy, Phenylsulfonyl, Methylsulfonyl, Hydroxysulfonyl, Acetylamino, Nitro, Hydroxyl, Carboxyl, Carbamoyl oder Sulfamoyl substituiertes Phenyl oder durch Hydroxysulfonyl substituiertes 1- oder 2-Naphthyl tragen können. Beispielsweise sind 1-Phenyl-, 1-(2'-Chlorphenyl)-, 1-(2'-Methoxyphenyl)-, 1-(2'Methylphenyl)-, 1-(1',5'-Dichlorphenyl)-, 1-(2',6'Dichlorphenyl)-, 1-(2'- Methyl-6'-chlorphenyl)-, 1-(2'-Methoxy-5'-methylphenyl)-, 1-(2'-Methoxy- 5'-hydroxysulfonylphenyl)-, 1-(2',5'-Dichlor-4'-hydroxysulfonylphenyl)-, 1-(2',5'-Dihydroxysulfonylphenyl)-, 1-(2'-Carboxyphenyl)-, 1-(3'-Hydroxysulfonylphenyl)-, 1-(4'-Hydroxysulfonylphenyl)- oder 1-(3'-Sulfamoylphenyl)-3-carboxylpyrazol-5-on, 1-(3'- oder 4'-Hydroxysulfonylphenyl)-, 1-(2'-Chlor-4'- oder -5'-hydroxysulfonylphenyl)-, 1-(2'-Methyl-4'-hydroxysulfonylphenyl)-, 1-(2',5'-Dichlorphenyl)-, 1-(4',8'-Dihydroxysulfonyl-1-naphthyl)-, 1-(6'-Hydroxysulfonyl-1-naphthyl)-3-methylpyrazol-5-on, 1-Phenylpyrazol-5-on-3-carbonsäureethylester, Pyrazol-5-on-3-carbonsäureethylester oder Pyrazol-5-on-3-carbonsäure zu nennen.

Andere aus der Pyrazolreihe stammende Kupplungskomponenten sind beispielsweise 1-Methyl-, 1-Ethyl-, 1-Propyl-, 1-Butyl-, 1-Cyclohexyl-, 1-Benzyl- oder 1-Phenyl-5-aminopyrazol, 1-(4'-Chlorphenyl)-, l-(4'-Methylphenyl)-5-aminopyrazol oder l-Phenyl-3-methyl-5-aminopyrazol.

Acetoacetanilide sind vor allem Acetessiganilid oder dessen im Phenylkern durch Chlor, Methyl, Ethyl, Methoxy, Ethoxy, Acetylamino, Hydroxysulfonyl, Carboxyl, Carbamoyl oder Sulfamoyl ein- oder mehrfach substituierte Derivate.

Vom Pyridin abgeleitete Kupplungskomponenten sind beispielsweise die in der DE-A-2 260 827 beschriebenen Derivate.

Als Pyrimidinkupplungskomponenten sind z. B. die in der DE-A-2 202 820, DE-A-2 308 663 oder DE-A-3 119 349 aufgeführten Verbindungen geeignet. Weiterhin sind Barbitursäure und deren N-Substitutionsprodukte zu nennen. Als N-Substituenten kommen dabei insbesondere C₁-C₄-Alkyl oder Phenyl in Betracht.

Als Indolkupplungskomponenten sind beispielsweise 2-Methylindol, 2-Phenylindol, 2-Phenylindol-5-sulfonsäure, 1-Methyl-2-phenylindol, 1-(2'-Hydroxyethyl)-, 1-(2'-Carboxyethyl)-, 1-(2'Carbamoylethyl)-2-methylindol- oder -2-phenylindol zu nennen.

Als Pyridonkupplungskomponenten sind beispielsweise 1-Ethyl-2-hydroxy-4-methyl-5-carbamoylpyrid-6-on, 1-(2'Hydroxyethyl)-2-hydroxy-4-methyl-5-carbamoylpyrid-6-on, 1-Phenyl-2-hydroxy-4-methyl-5-carbamoylpyrid-6-on, 1-Ethyl-2-hydroxy-4-methyl-5-cyanopyrid-6-on, 1-Ethyl-2-hydroxy-4-methyl-5-hydroxysulfonylmethylpyrid-6-on, 1-Methyl-2-hydroxy-4-methyl-5-cyanopyrid-6-on, 1-Methyl-2-hydroxy-5-acetylpyrid-6-on, 1,4-Dimethyl-2-hydroxy-5-cyanopyrid-6-on, 1,4-Dimethyl-5-carbamoylpyrid-6-on, 2,6-Dihydroxy-4-ethyl-5-cyanopyridin, 2-Hydroxy-4-ethyl-5-carbamoylpyrid-6-on, 1-Ethyl-2-hydroxy-4-methyl-5-hydroxysulfonylmethylpyrid-6-on, 1-Methyl-2-hydroxy-4-methyl-5-methylsulfonylpyrid-6-on oder 1-Carboxymethyl-2-hydroxy-4-ethyl-5-phenylsulfonylpyrid-6-on zu nennen.

Faserreaktivgruppenhaltige Kupplungskomponenten K der Benzol- oder Naphthalinreihe sind beispielsweise Verbindungen der Formeln XVII a - f 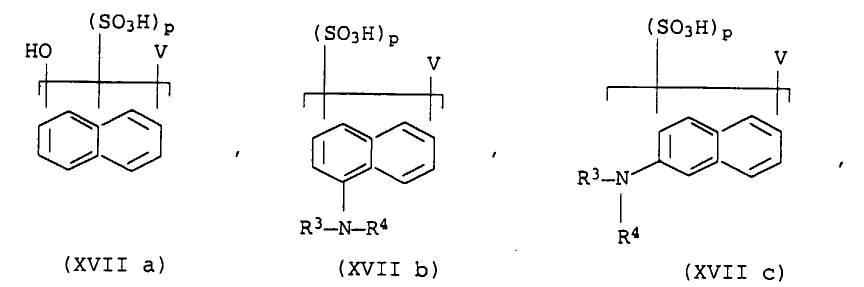 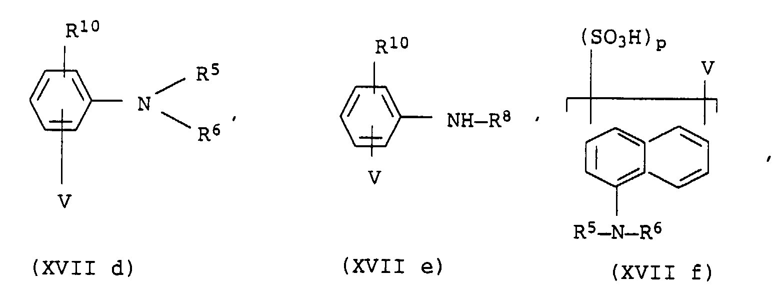 worin R³, R⁴, R⁵, R⁶, R⁸, R¹⁰, V und p jeweils die obengenannte Bedeutung besitzen.

Faserreaktivgruppenhaltige Kupplungskomponenten der Pyrazolon-, Aminopyrazol-, 2,6-Diaminopyridin-, Pyridon-, Hydroxy- oder Aminopyrimidin-, Indol-, oder N-Arylacetoacetamid entsprechen beispielsweise den Formel XVIII a - f 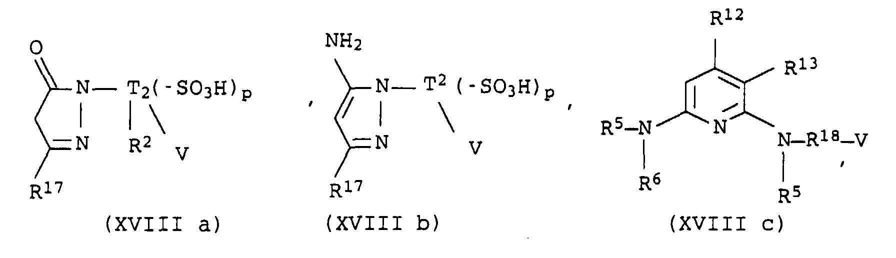 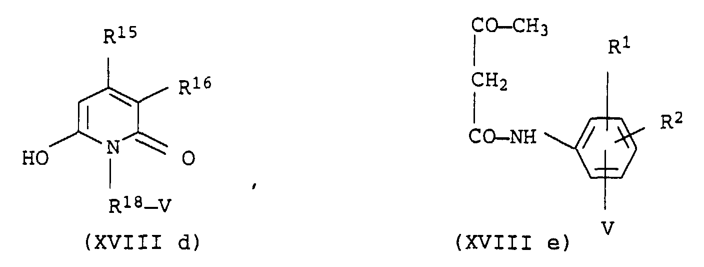 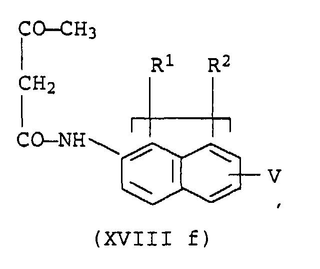 worin
T² für den Rest eines Benzol- oder Naphthalinrings,
R¹⁷ für Methyl, Carboxyl, C₁-C₄-Alkoxycarbonyl oder Phenyl und
R¹⁸ für C₁-C₆-Alkylen stehen und
R¹, R², R⁵, R⁶, R¹², R¹³, R¹⁵, R¹⁶, p und V jeweils die obengenannte Bedeutung besitzen.

Faserreaktive Reste V tragende Pyrazolonkupplungskomponenten leiten sich beispielsweise von folgenden Pyrazolonen ab: 1-(3'- oder 4'-Aminophenyl)-, 1-(2'-Hydroxysulfonyl-5'-aminophenyl)- oder 1-(2'-Methoxy-5'-aminophenyl)- 3-carboxylpyrazol-5-on, 1-(3'-oder 4'-Aminophenyl)- oder 1-(6'-Amino- 4',8'-dihydroxysulfonylnaphth-2'-yl)-3-carboxylpyrazol-5-on.

Anstelle der Azofarbstoffreste können die Farbstoffe der Formel I auch entsprechende Metallkomplexazofarbstoffreste enthalten. Als komplexierende Metalle kommen dabei insbesondere Kupfer, Kobalt, Chrom, Nickel oder Eisen in Betracht, wobei Kupfer, Kobalt oder Chrom bevorzugt sind.

Dabei befinden sich die metallisierten Gruppen vorzugsweise jeweils in ortho-Stellung zur Azogruppe, z. B. in Form von o,o'-Dihydroxy-, o-Hydroxy-o'-carboxy-, o-Carboxy-o'-amino- oder o-Hydroxy-o'-amino-azogruppierungen.

W in Formel I stellt weiterhin z.B. den Rest eines metallisierten Formazanfarbstoffs dar, wobei insbesondere Kupferformazane zu nennen sind. Kupferformazane sind an sich bekannt und beispielsweise in K. Venkataraman "The Chemistry of Synthetic Dyes", Vol. III, Academic Press, New York, London, 1970, beschrieben.

Besonders bevorzugt sind Kupfer-Formazanfarbstoffe der Formel XIX 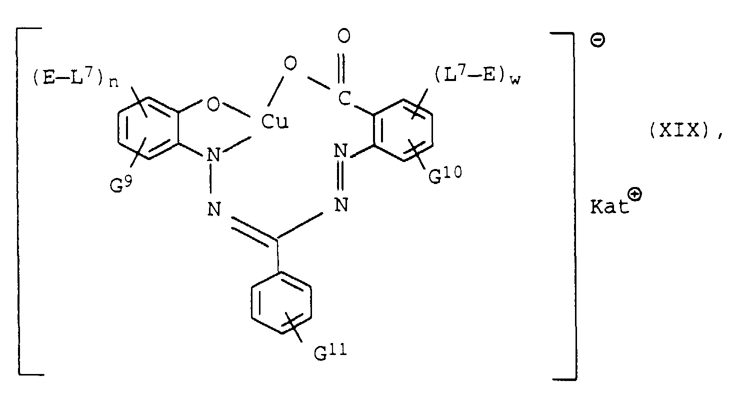 in der
- Kat^{⊕}: das Äquivalent eines Kations ist
- G⁹, G¹⁰ und G¹¹: gleich oder verschieden sind und unabhängig voneinander jeweils Wasserstoff oder Hydroxysulfonyl-
- n: 0 oder 1 und
- w: 0 oder 1 bedeuten und
- E und L⁷: jeweils die obengenannte Bedeutung besitzen, mit der Maßgabe, daß n und w nicht gleichzeitig 0 bedeuten.

Kat^{⊕} in Formel XIX stellt das Äquivalent eines Kations dar. Es stellt entweder ein Proton dar oder leitet sich von Metall- oder Ammoniumionen ab. Metallionen sind insbesondere die Lithium-, Natrium- oder Kaliumionen. Unter Ammoniumionen im erfindungsgemäßen Sinne sind unsubstituierte oder substituierte Ammoniumkationen zu verstehen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium- oder Piperaziniumkationen oder deren N-monoalkyl- oder N,N-dialkylsubstituierte Produkte. Unter Alkyl ist dabei im allgemeinen geradkettiges oder verzweigtes C₁-C₂₀-Alkyl zu verstehen, das durch 1 oder 2 Hydroxylgruppen substituiert und/oder durch 1 bis 4 Sauerstoffatome in Etherfunktion unterbrochen sein kann.

Besonders als Kationen hervorzuheben sind Protonen oder Lithium-, Natrium- oder Kaliumionen, wobei die genannten Metallkationen auch bevorzugte Kationen sind, wenn die Reaktivfarbstoffe XIX in Salzform vorliegen.

Eine Methode zur Herstellung der diesen Farbstoffen zugrundeliegenden Formazane ist beispielsweise in der EP-A-315 046 beschrieben.

W in Formel I stellt weiterhin z.B. den Rest eines Anthrachinonfarbstoffs dar. Anthrachinone sind an sich bekannt und beispielsweise in K. Venkataraman "The Chemistry of Synthetic Dyes", Vol. II, Academic Press, New York, 1952, beschrieben.

Besonders bevorzugt sind Anthrachinonfarbstoffe der Formel XX 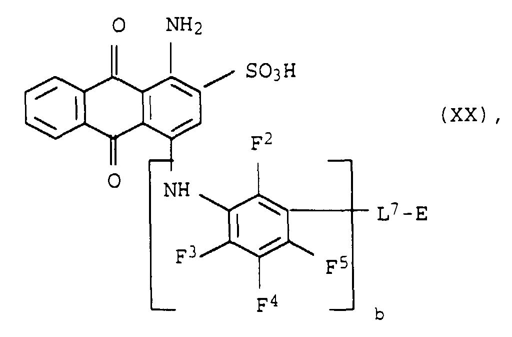 worin E und L⁷ jeweils die obengenannte Bedeutung besitzen, b 0 oder 1 bedeutet und F² und F³ unabhängig voneinander jeweils Wasserstoff oder Methyl und einer der beiden Reste F⁴ und F⁵ Wasserstoff oder Methyl und der andere Hydroxysulfonyl bedeuten.

W in Formel I stellt weiterhin z.B. den Rest eines Triphendioxazinfarbstoffs dar. Triphendioxazine sind an sich bekannt und beispielsweise in der EP-A-141 359 oder EP-A-311 969 beschrieben.

Besonders bevorzugt sind Triphendioxazinfarbstoffe der Formel XXI 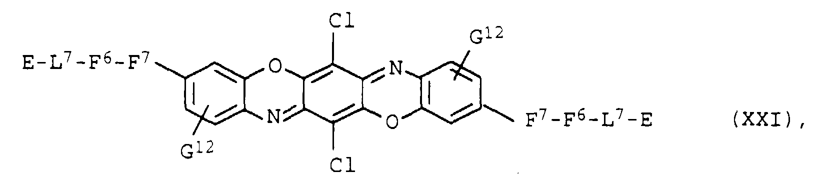 in der
E und L⁷ jeweils die obengenannte Bedeutung besitzen und
G¹² für Hydroxysulfonyl oder den Rest SO₂-C₂H₄-SO₃H,
F⁷ für Sauerstoff, Imino oder C₁-C₄-Alkylimino und
F⁶ für C₂-C₄-Alkylen oder Phenylen stehen.

W in Formel I stellt weiterhin z.B. den Rest eines metallisierten Phthalocyaninfarbstoffs dar. Phthalocyanine sind an sich bekannt und beispielsweise in F.H. Moser, D.L. Thomas "The Phthalocyanines", Vol. II, CRC Press, Boca Raton, Florida 1983, beschrieben.

Besonders bevorzugt sind Phthalocyaninfarbstoffe der Formel XXII 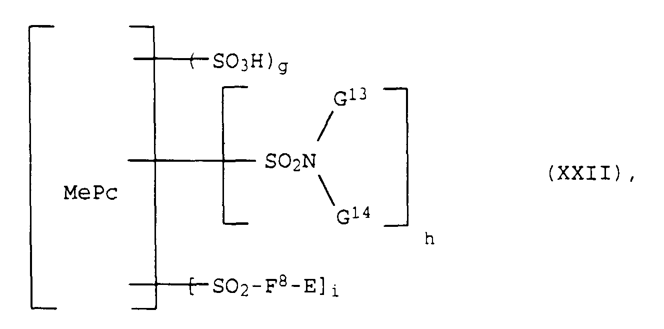 in der
- Pc: den Phthalocyaninrest,
- G¹³ und G¹⁴: unabhängig voneinander jeweils Wasserstoff oder C₁-C₄-Alkyl,
- F⁸: Imino oder C₁-C₄-Alkylimino,
- Me: Kupfer oder Nickel,
- g: 0, 1 oder 2,
- h: 0, 1 oder 2 und
- i: 1 oder 2 bedeuten
und E die obengenannte Bedeutung besitzt.

Die Herstellung der neuen Reaktivfarbstoffe der Formel I kann nach an sich bekannten Methoden erfolgen.

Beispielsweise kann man einen geeigneten Farbstoff der Formel XXIII

W¹-G¹⁵ (XXIII),

in der W¹ den Rest eines Chromophors, der gegebenenfalls weitere reaktive Gruppen aufweist und der sich von einem gegebenenfalls metallisierten Mono- oder Disazofarbstoff, einem Triphendioxazin, einem Anthrachinon, einem metallisierten Formazan oder einem metallisierten Phthalocyanin ableitet und G¹⁵ einen Rest der Formel NHZ³, COHal oder SO₂Hal, worin Z³ und Hal jeweils die obengenannte Bedeutung besitzen, bedeuten, mit einer faserreaktiven Verbindung der Formel XXIV

G¹⁵-E (XXIV),

in der G¹⁵ und E jeweils die obengenannte Bedeutung besitzen, umsetzen.

Wenn L¹ (Fall 1) eine Harnstoffbrücke oder einen Triazinrest darstellt, fügt man die bei diesen Stoffklassen üblichen Syntheseschritte ein.

Es ist auch möglich, von solchen Zwischenprodukten der Verbindungen der Formel XXIII auszugehen, die noch über keinen Chromophorrest verfügen, und diese zunächst mit der faserreaktiven Verbindung XXIV umzusetzen und anschließend den Chromophorrest W¹ aufzubauen.

Wenn der Rest W eine Kupplungskomponente darstellt, gelangt man zu den erfindungsgemäßen Farbstoffen, in dem man z.B. die faserreaktive Verbindung der Formel XXV

H₂N-E (XXV),

in der E die obengenannte Bedeutung besitzt, auf an sich bekannte Weise diazotiert und mit einer Kupplungskomponente der Formel XXVI

W²-H (XXVI)

kuppelt, in der W² den Rest einer Kupplungskomponente, an den gegebenenfalls zusätzlich der Rest einer Diazokomponente über eine Azobrücke gebunden ist und der gegebenenfalls zusätzliche reaktive Gruppen aufweist, bedeutet.

Wenn b in Formel I den Wert 1 bedeutet, so kann man zu den verdoppelten Chromophoren z.B. auf die Weise gelangen, daß man entweder die fertigen Einzelchromophore verdoppelt oder aber zunächst geeignete Zwischenprodukte verdoppelt und danach die jeweiligen chromophoren Systeme aufbaut.

Die Herstellung der faserreaktiven Verbindungen der Formeln XXIV und XXV ist an sich bekannt und beispielsweise in der EP-A 680 951 beschrieben.

Ein weiterer Gegenstand der vorliegenden Anmeldung sind Schwefelverbindungen der Formel XXVII 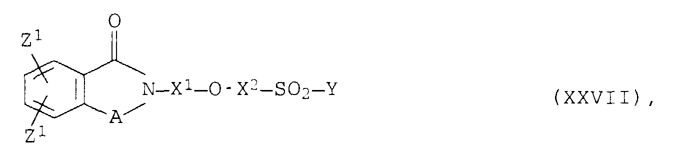 in der
- die Reste Z¹: unabhängig voneinander jeweils Wasserstoff, C₁-C₆-Alkyl, C₁-C₆-Alkoxy, Halogen, Nitro, Amino, Hydroxysulfonyl, C₁-C₆-Alkoxycarbonyl, Carbamoyl oder C₁-C₆-Mono- oder Dialkylcarbamoyl,
- A: Methylen, Sulfonyl oder einen Rest der Formel CH₂-CO oder CH₂-SO₂, wobei die Methylengruppe jeweils an den Benzolring geknüpft ist,
- X¹ und X²: unabhängig voneinander jeweils C₂-C₄-Alkylen und
- Y: Vinyl oder einen Rest der Formel C₂H₄OH oder C₂H₄Q, worin Q für eine unter alkalischen Reaktionsbedingungen abspaltbare Gruppe steht, bedeuten.

Bevorzugt sind Schwefelsverbindungen der Formel XXVII, in der A Methylen oder den Rest der Formel CH₂-CO bedeutet.

Bevorzugt sind weiterhin Schwefelverbindungen der Formel XXVII, in der X¹ und X² jeweils C₂- oder C₃-Alkylen, insbesondere jeweils C₂-Alkylen bedeutet.

Bevorzugt sind weiterhin Schwefelverbindungen der Formel XXVII, in der ein Rest Z¹ Wasserstoff oder Amino und der andere Wasserstoff bedeuten.

Besonders bevorzugt sind Schwefelverbindungen der Formel XXVII, in der ein Rest Z¹ Amino und der andere Wasserstoff bedeuten.

Besonders bevorzugt sind weiterhin Schwefelverbindungen der Formel XXVII, in der A Methylen bedeutet.

Die neuen Schwefelverbindungen der Formel XXVII können auf an sich bekannte Weise erhalten werden. Beispielsweise kann man ein Benzolderivat der Formel XXVIII 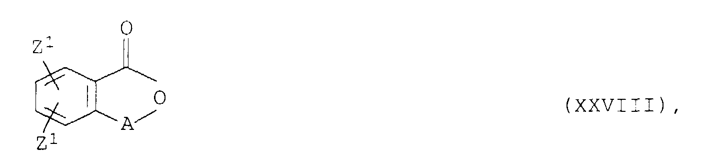 in der Z¹ und A jeweils die obengenannte Bedeutung besitzen, mit einem Amin der Formel XXIX

H₂N-X¹-O-X²-SO₂-Y (XXIX),

in der X¹ und X² und Y jeweils die obengenannte Bedeutung besitzen, umsetzen.

Vorteilhaft ist die Umsetzung der Benzolderivate der Formel XXVIII zunächst mit einem Thioether der Formel XXIXa

H₂N-X¹-O-X²-S-Y (XXIXa),

in der X¹, X² und Y jeweils die obengenannte Bedeutung besitzen, und anschließende Oxidation des Sulfids zur Sulfonylgruppe, z.B. mit Wasserstoffperoxid.

Die Reste Z¹, sofern sie von Wasserstoff verschieden sind, können auf an sich bekanntem Weg auch nachträglich in den Benzolring eingeführt werden. Insbesondere bei der Einführung der Hydroxysulfonylgruppe oder der Nitrogruppe, die anschließend zur Aminogruppe reduziert werden kann, ist dieser Weg bevorzugt.

Die neuen Schwefelverbindungen der Formel XXVII sind wertvolle Zwischenprodukte für die Herstellung von Farbstoffen, insbesondere für die hier näher bezeichneten Reaktivfarbstoffe.

Die neuen Reaktivfarbstoffe der Formel I eignen sich in vorteilhafter Weise zum Färben oder Bedrucken von Hydroxygruppen oder Stickstoffatome aufweisenden organischen Substraten. Solche Substrate sind beispielsweise Leder oder Fasermaterial, das überwiegend natürliche oder synthetische Polyamide oder natürliche oder regenerierte Cellulose enthält. Vorzugsweise eignen sich die neuen Farbstoffe zum Färben und Bedrucken von Textilmaterial auf der Basis von Wolle oder insbesondere von Baumwolle.

Insbesondere auf Substraten auf Basis von Cellulose werden farbstarke Färbungen mit sehr hoher Fixierausbeute erhalten, die eine sehr gute Lichtechtheit sowie vorzügliche Naßechtheiten, wie Wasch-, Chlorbleich-, Peroxidbleich-, Alkali-, Meerwasser- oder Schweißechtheit, aufweisen.

Die folgenden Beispiele sollen die Erfindung näher erläutern.

### Beispiel 1

a) 94 g Cyanurchlorid wurden in einer Mischung aus 400 ml Eiswasser, 1 ml 30 gew.-%iger Salzsäure und 0,5 g eines Dispergiermittels dispergiert und bei 0° bis 5°C mit einer Lösung von 126 g Anilin-2,5-disulfonsäure in 250 ml Wasser und 35 ml 50 gew.-%iger Natronlauge versetzt. Nach vierstündigem Rühren bei 0 bis 5°C und einem pH-Wert von 4 bis 5, der mit 47 g Natriumhydrogencarbonat eingehalten wurde, war die Reaktion beendet und das Reaktionsgemisch wurde klärfiltriert.
   Dem Filtrat ließ man eine Lösung von 170 g 1-Amino-8-hydroxynaphthalin-3,6-disulfonsäure (Mononatriumsalz) in 500 ml Wasser und 25 ml 50 gew.-%iger Natronlauge zufließen und hielt dabei ohne weitere Kühlung mit Natriumhydrogencarbonat einen pH-Wert von 4,5 bis 5 ein. Nach Rühren über Nacht bei Raumtemperatur wurde das Kondensat mit 600 g Kochsalz gefällt, abgesaugt, mit 20 gew.%iger wäßriger Kochsalzlösung gewaschen und bei 50°C unter vermindertem Druck getrocknet.
b) 18,2 g der Ankerverbindung der Formel 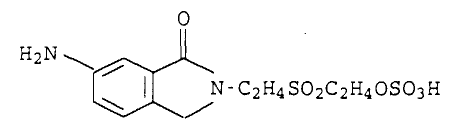 wurden in 200 ml Wasser mit 4 g Natriumhydrogencarbonat bei einem pH-Wert von 4,4 gelöst. Nach Kühlung mit 150 g Eis wurden 18 ml 30 gew.-%ige Salzsäure zugegeben und durch Eintropfen von 15 ml 3,33 N wäßriger Natriumnitritlösung diazotiert. Nach 30-minütigem Rühren bei 0° bis 5°C wurde ein leichter Nitritüberschuß mit Amidosulfonsäure entfernt und das Reaktionsgemisch mit einer Lösung von 52 g der unter a) beschriebenen Verbindung (Reinheit: 95 % Kochsalzgehalt: 20 %) in 200 ml Wasser versetzt. Die Kupplung erfolgte durch Eintropfen einer wäßrigen Natriumacetatlösung bis zu einem pH-Wert von 5.
Durch Fällung mit 200 g Kaliumchlorid, Absaugen und Trocknen wurde der Farbstoff der Formel 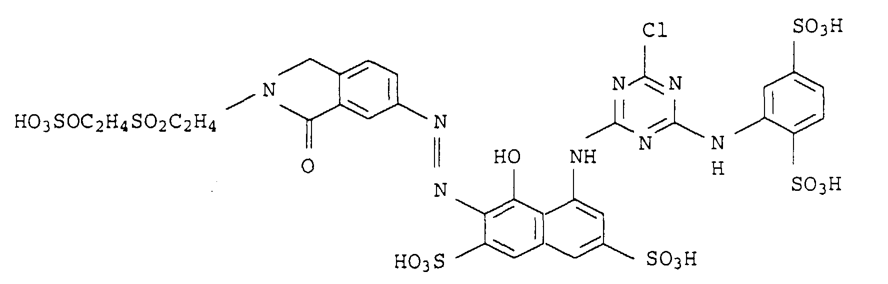 als Pentanatriumsalz isoliert. Es färbt Baumwolle entweder nach dem Ausziehverfahren bei 40 bis 60°C oder nach dem Kaltverweilverfahren bei Raumtemperatur in blaustichigen Rottönen. Die Färbungen zeichnen sich durch sehr gute Gebrauchsechtheiten und hohe Fixiergrade aus und sind sehr unempfindlich gegen Färbetemperaturschwankungen.

In analoger Weise können die in der folgenden Tabelle 1 aufgeführten Farbstoffe erhalten werden.

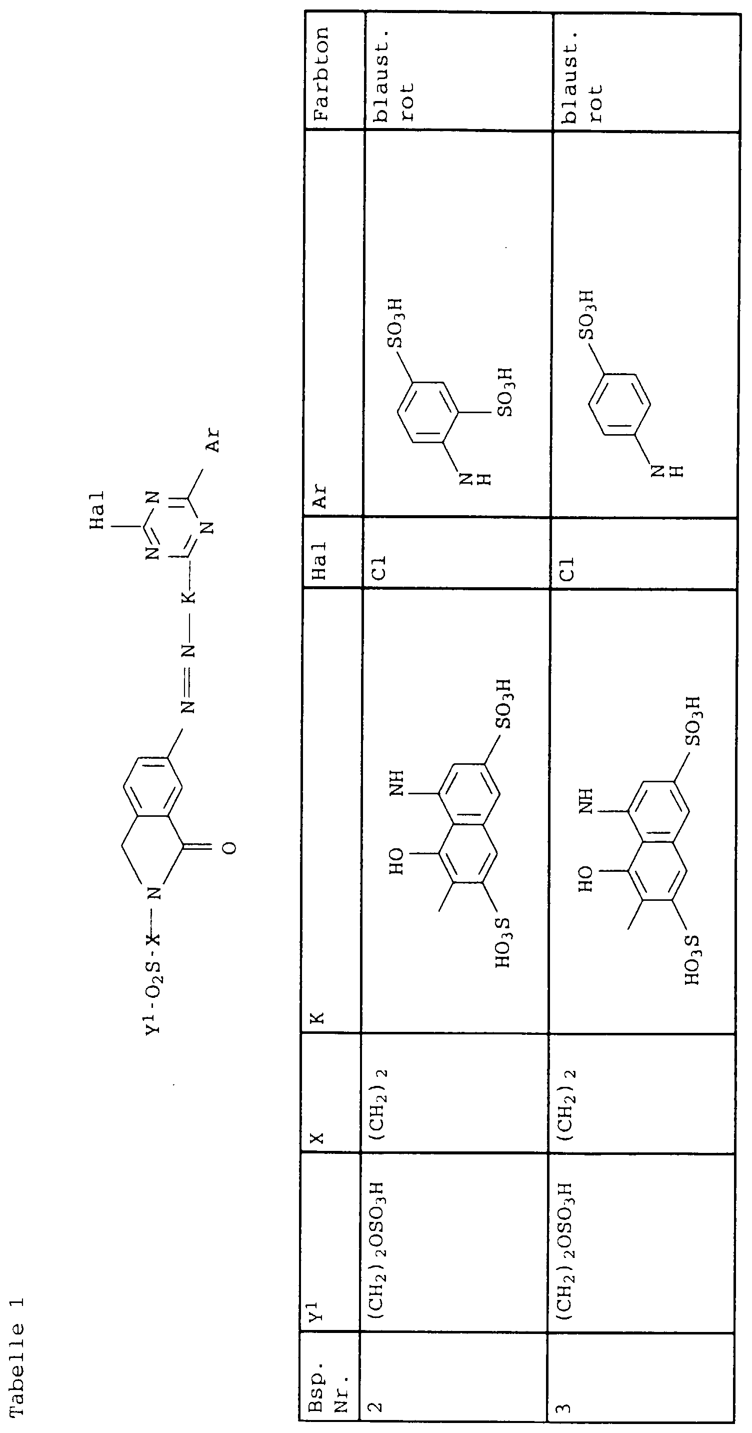

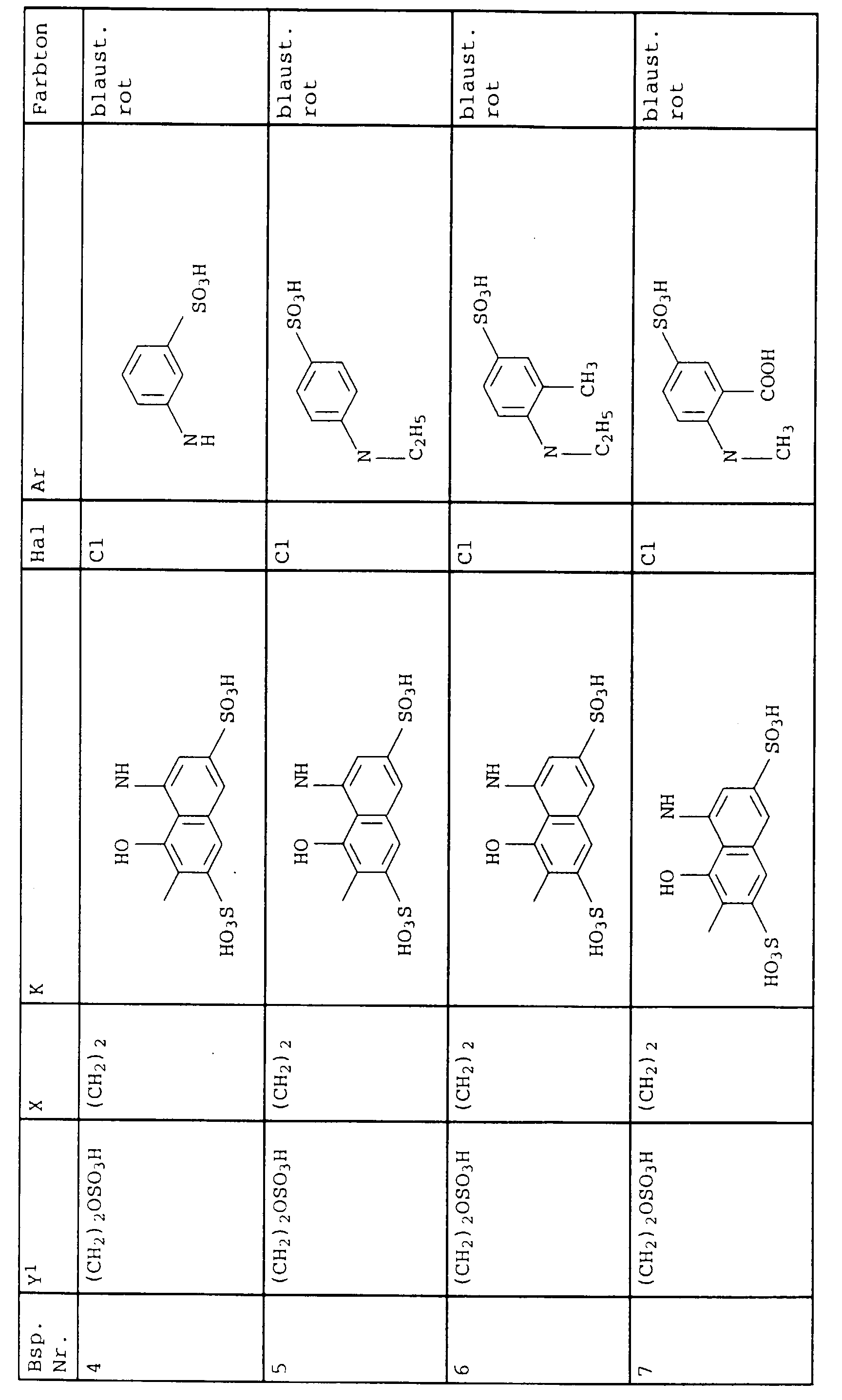

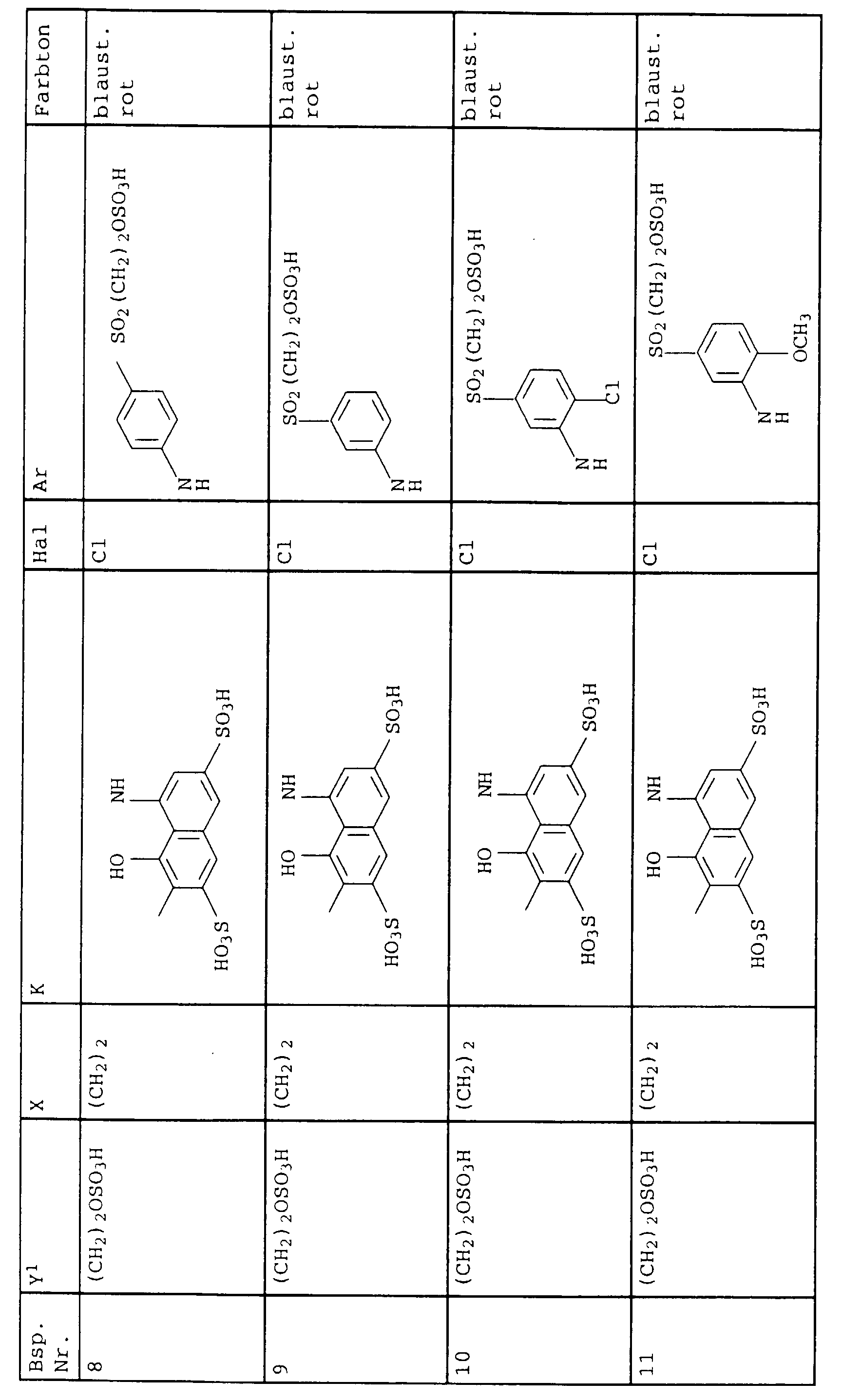

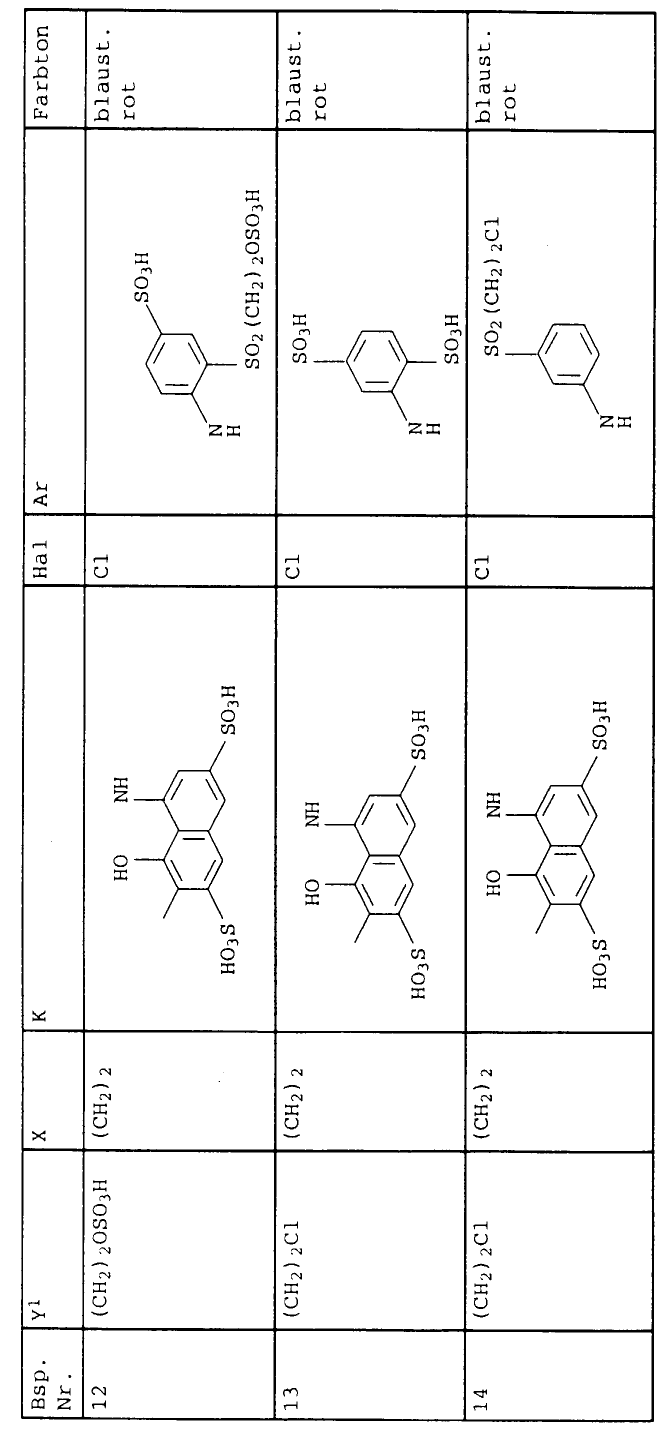

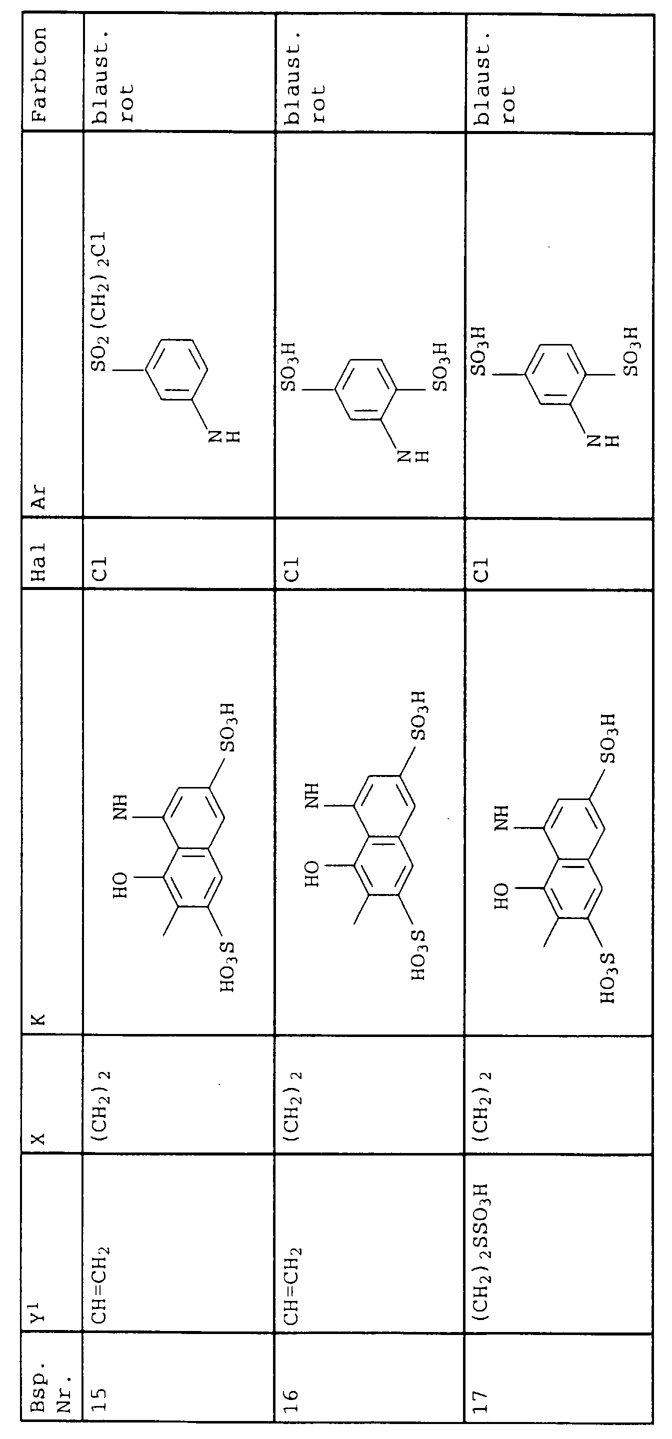

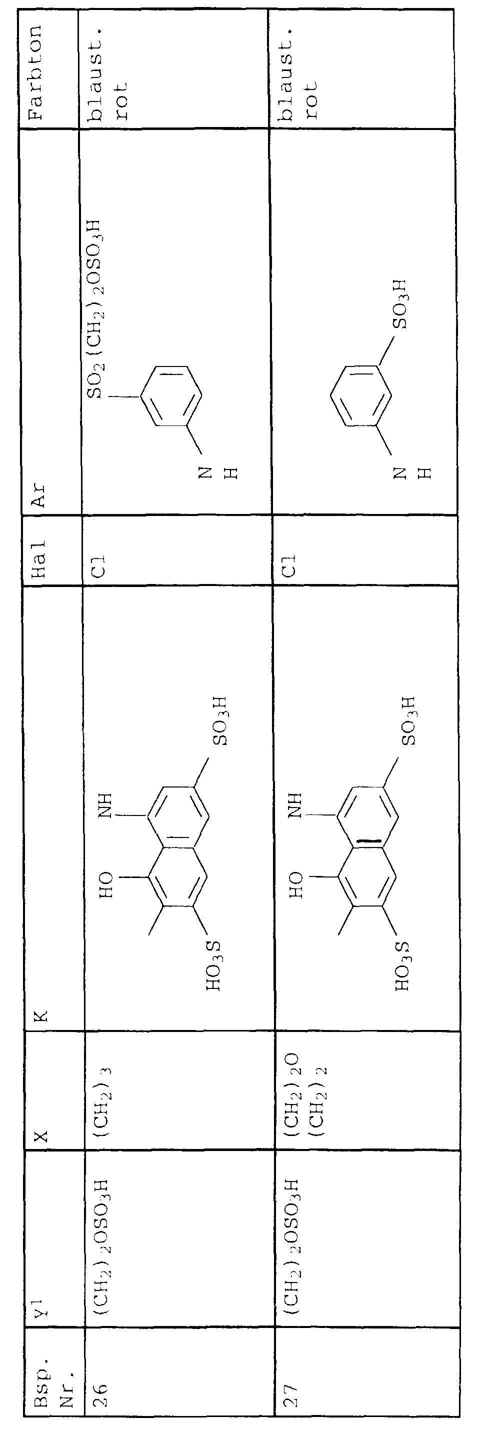

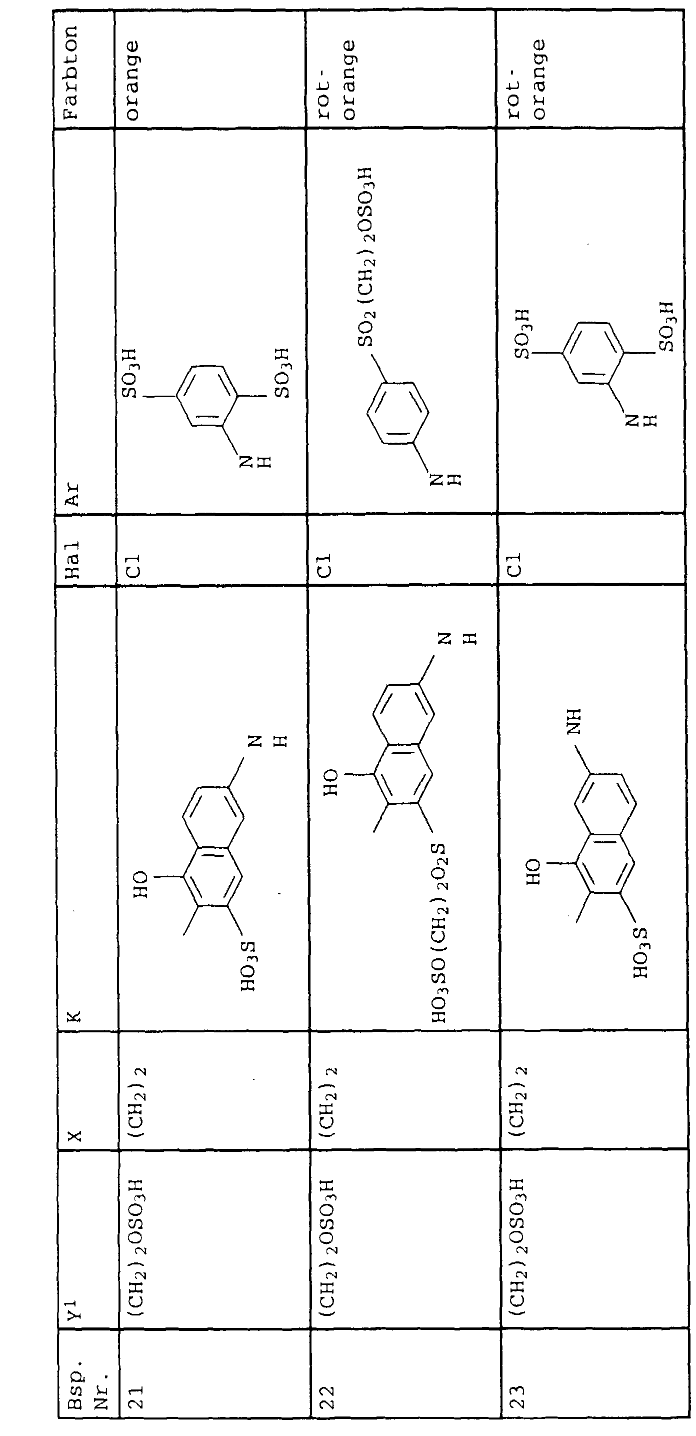

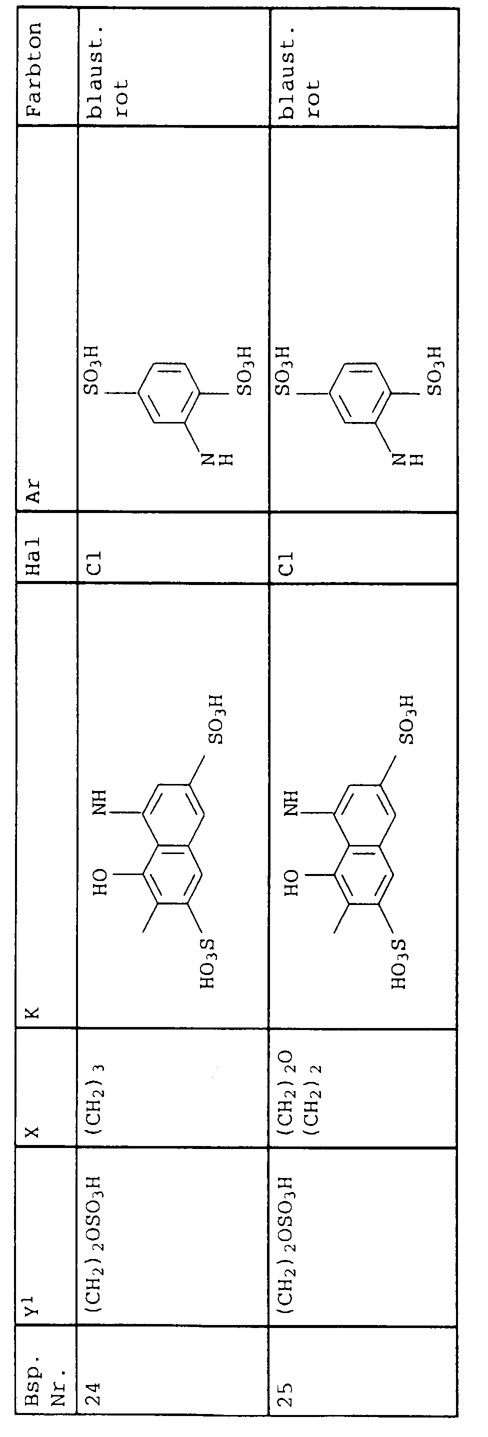

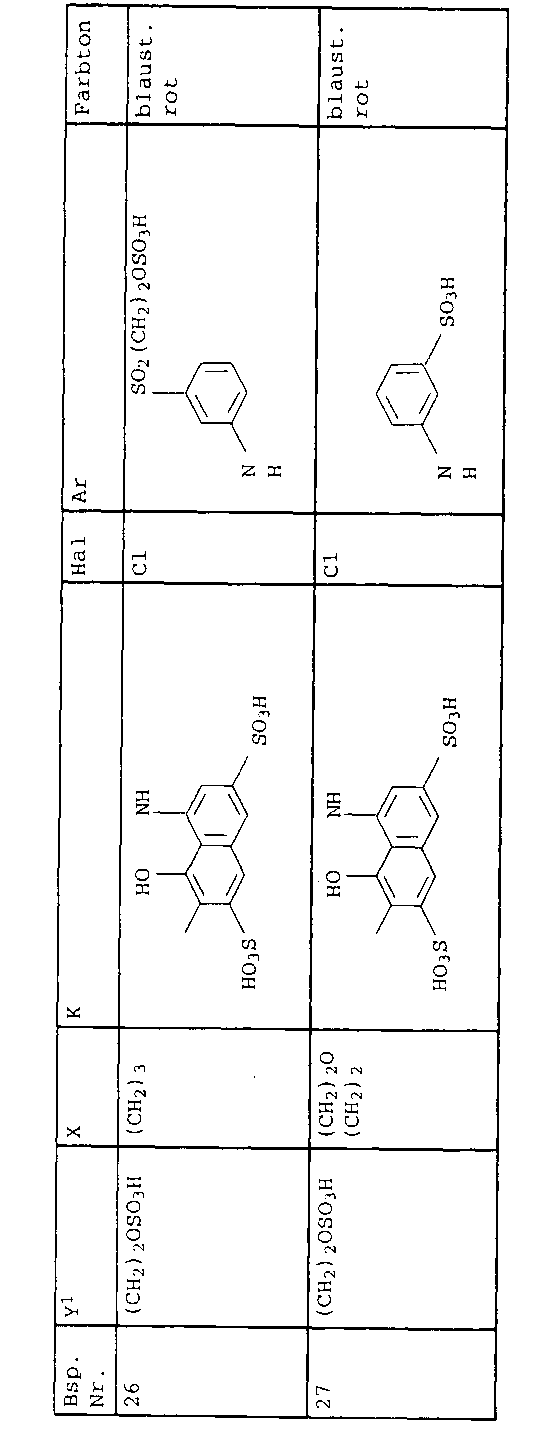

### Beispiel 28

Die unter Beispiel 1b) beschriebene Ankerverbindung wurde nach den dortigen Bedingungen mit N-Acetyl-H-Säure gekuppelt, die zuvor aus H-Säure und Acetanhydrid in wäßriger Lösung bereitet wurde.

Der durch Aussalzen mit Kochsalz erhaltene Farbstoff der Formel 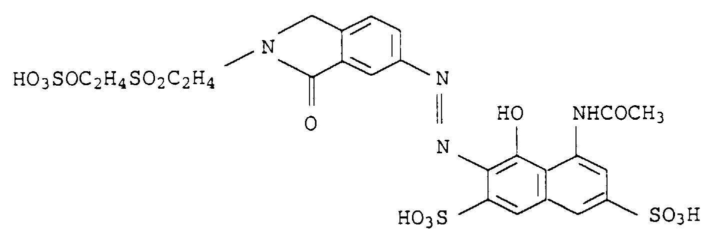 färbt Baumwolle in blaustichig rotem, klaren Ton mit gutem Echtheitsprofil.

Vergleichbare Eigenschaften besitzen die in der folgenden Tabelle 2 aufgeführten Farbstoffe.

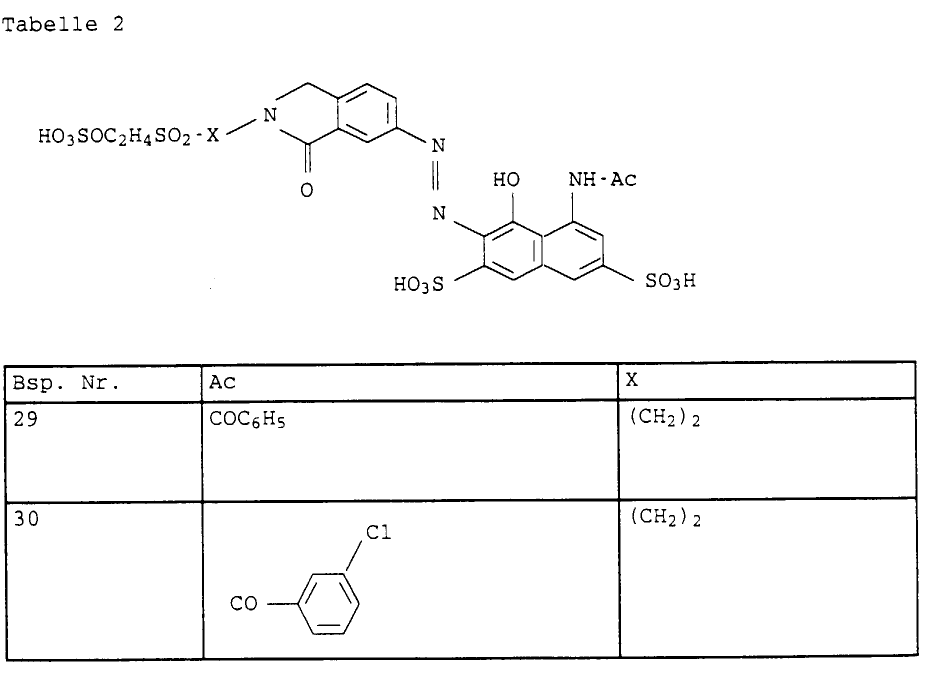

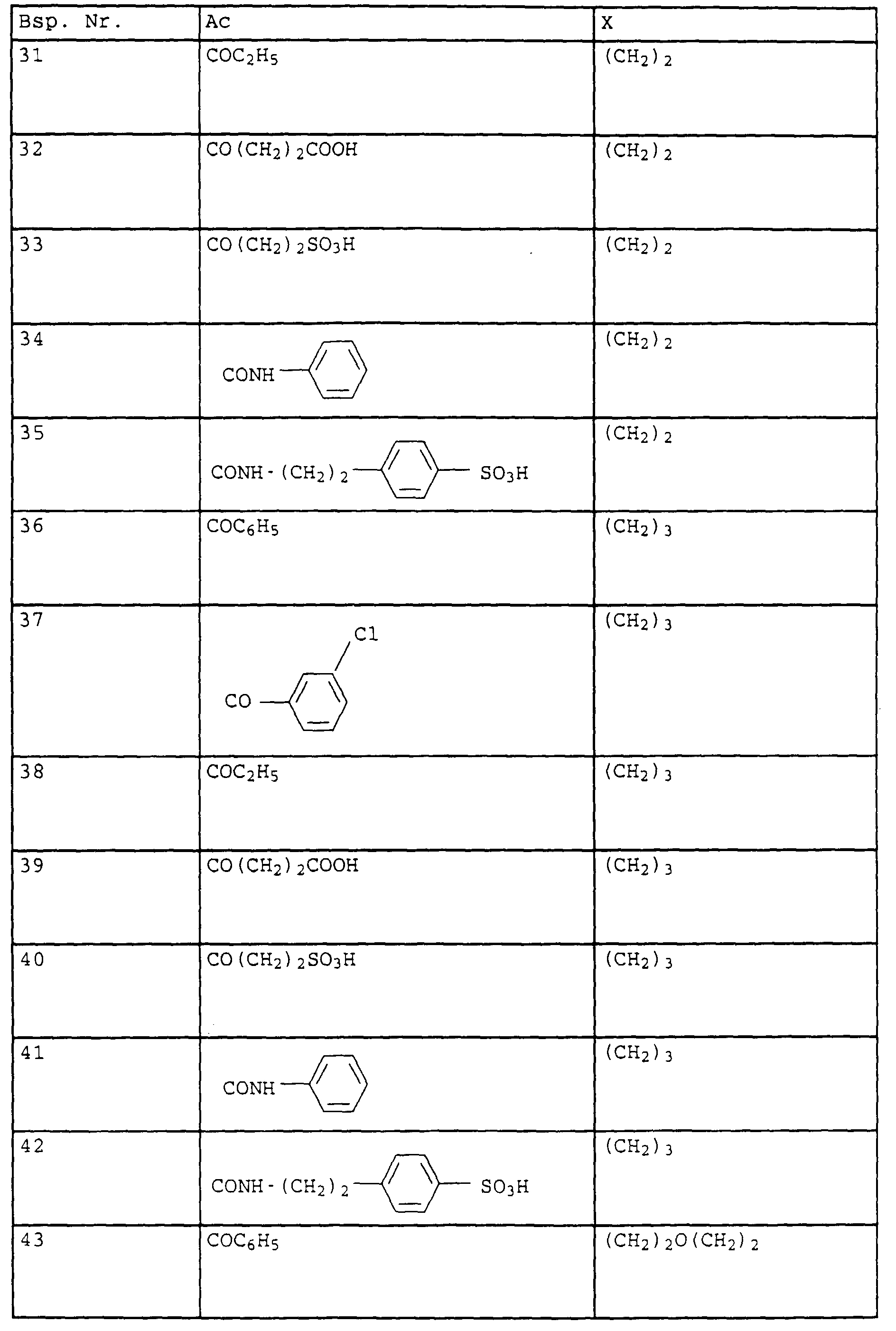

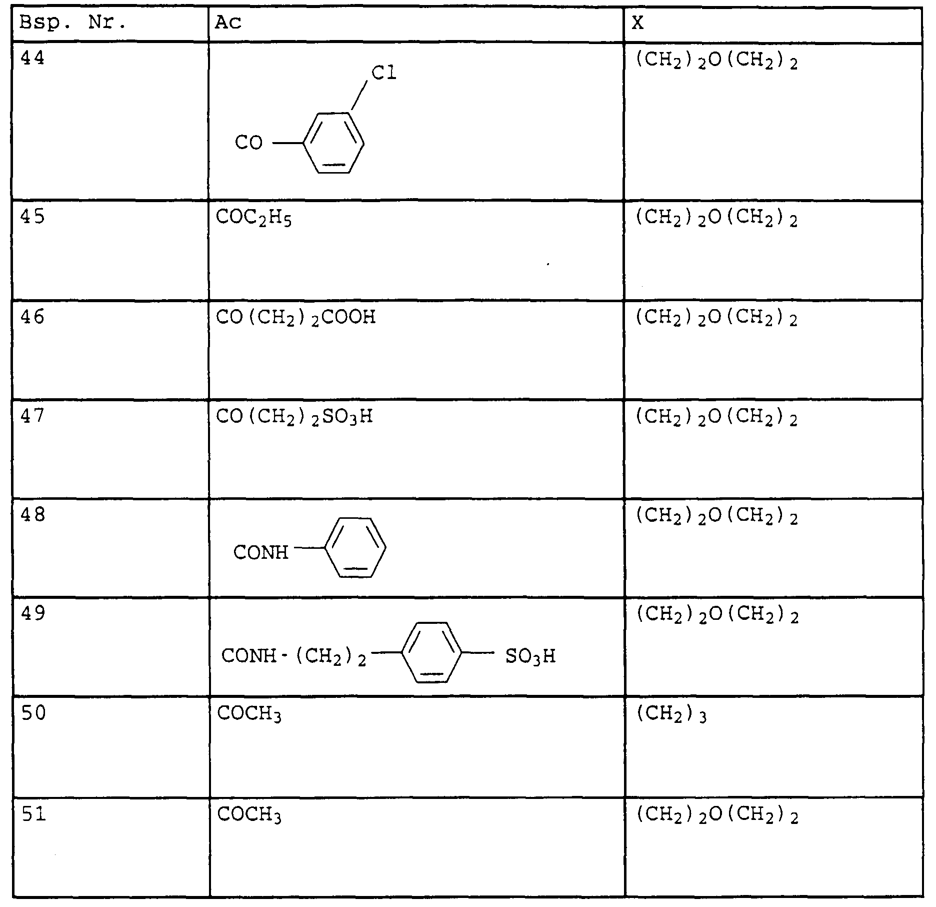

### Beispiel Nr. 52

a) 18,5 g Cyanurchlorid wurden in 60 ml einer Mischung aus 60 ml Eiswasser, 1 ml 30 gew.-%iger Salzsäure und 0,5 g eines Dispergiermittels dispergiert. Durch Zugabe einer Lösung von 26,3 g H-Säure-Dinatriumsalz in Wasser, die 1 g Dinatriumphosphat enthielt, bei 5° bis 10°C fand im stark sauren pH-Bereich vollständige Umsetzung statt. Dem resultierenden Reaktionsgemisch setzte man die Lösung von 37,5 g der Verbindung der Formel 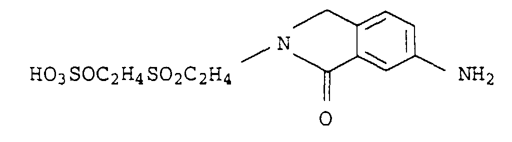 in 200 ml Wasser und 80 ml 10 gew.-%iger wäßriger Natriumhydrogencarbonatlösung zu und rührte unter Einhalten des pH-Wertes von 5 bei Raumtemperatur über Nacht.
   Das Reaktionsprodukt der Formel 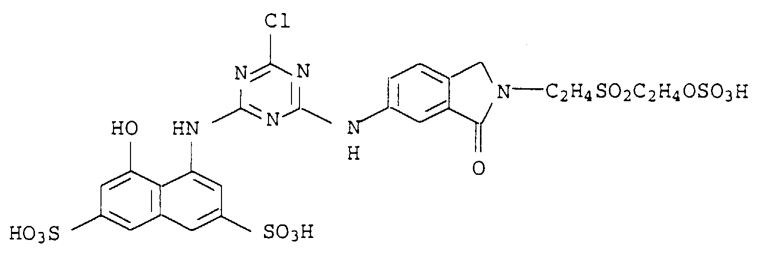 wurde mit 200 g Kochsalz gefällt, abgesaugt und mit 20 gew.-%iger wäßriger Kochsalzlösung gewaschen.
b) 17 g 2-Aminonaphthalin-1,5-disulfonsäure wurden in einer Mischung aus 50 ml Wasser, 6 ml 30 gew.-%iger Salzsäure und 50 g Eis verrührt und mit 16,5 ml 3,33 N wäßriger Natriumnitritlösung versetzt. Nach einstündigem Rühren bei 5 bis 10°C wurde der geringe Überschuß salpetriger Säure mit Amidosulfonsäure zerstört und das gesamte Reaktionsgemisch mit der Hälfte der Lösung des unter a) beschriebenen Monochlortriazins in 350 ml Wasser vereinigt. Durch Zugabe von Natriumhydrogencarbonat wurde bei 15 bis 20°C ein pH-Wert von 7 bis 8 eingehalten bis die Umsetzung beendet war. Der Farbstoff der Formel 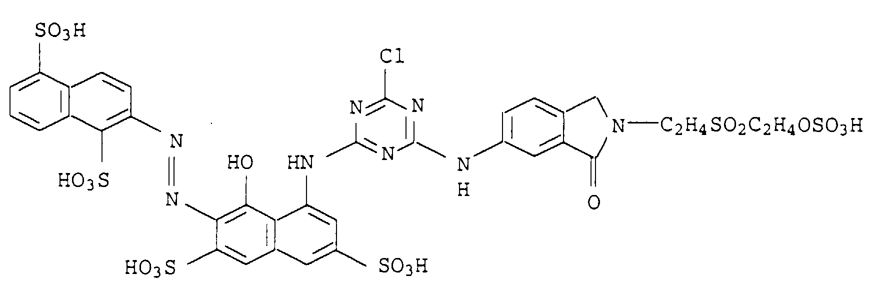 wurde mit 250 g Kaliumchlorid ausgefällt und abgesaugt. Er färbt Baumwolle aus alkalischem Bad in klarer blaustichig roter Nuance bei sehr guten Gebrauchsechtheiten.

In analoger Weise werden die in der folgenden Tabelle 3 aufgeführten Farbstoffe erhalten.

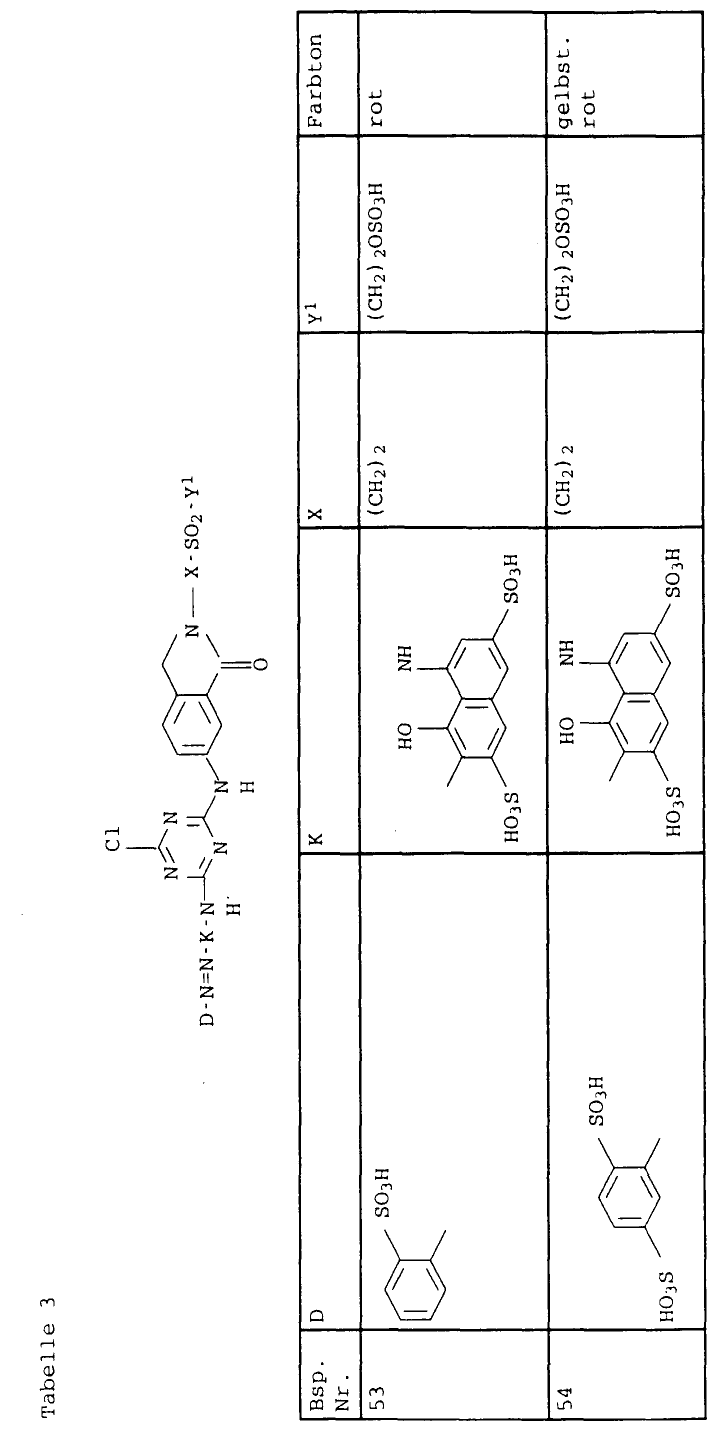

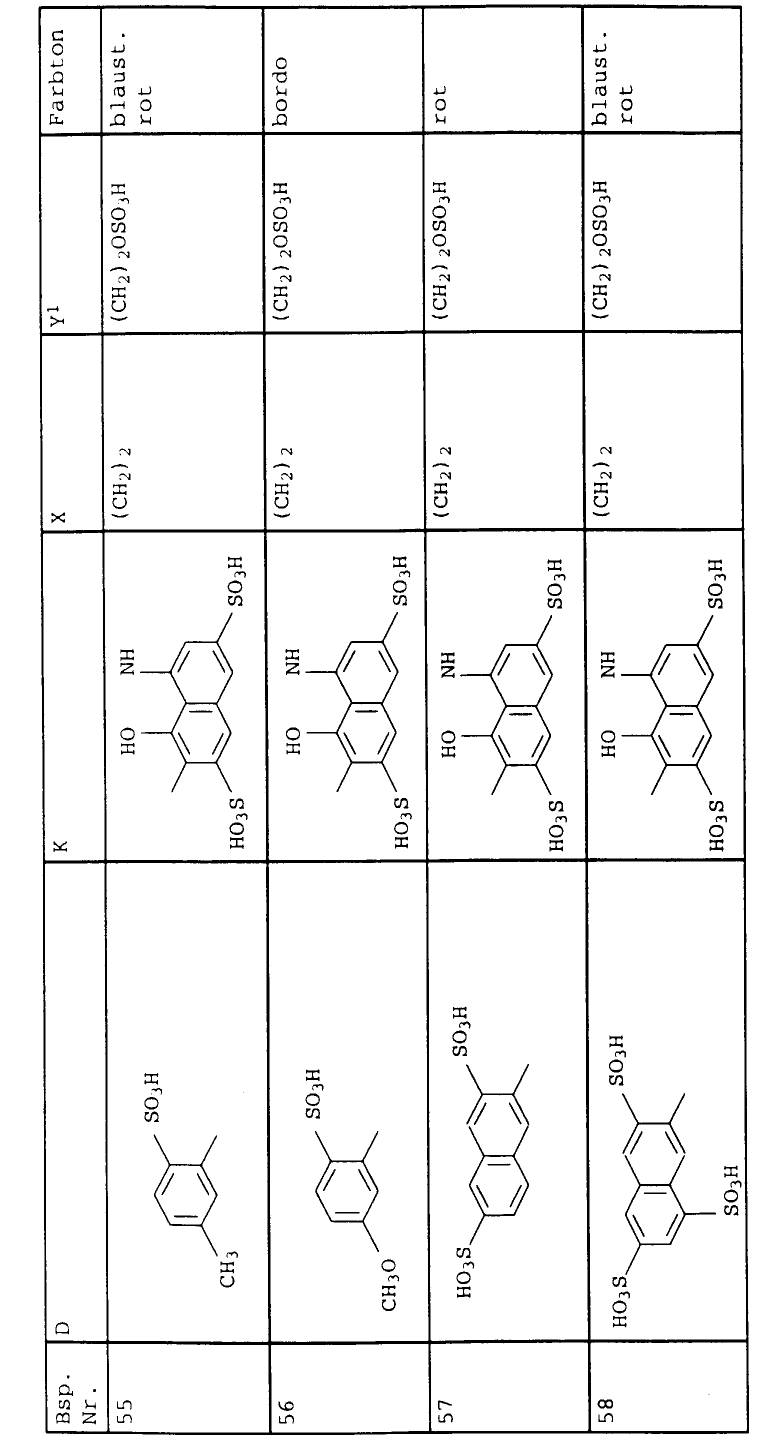

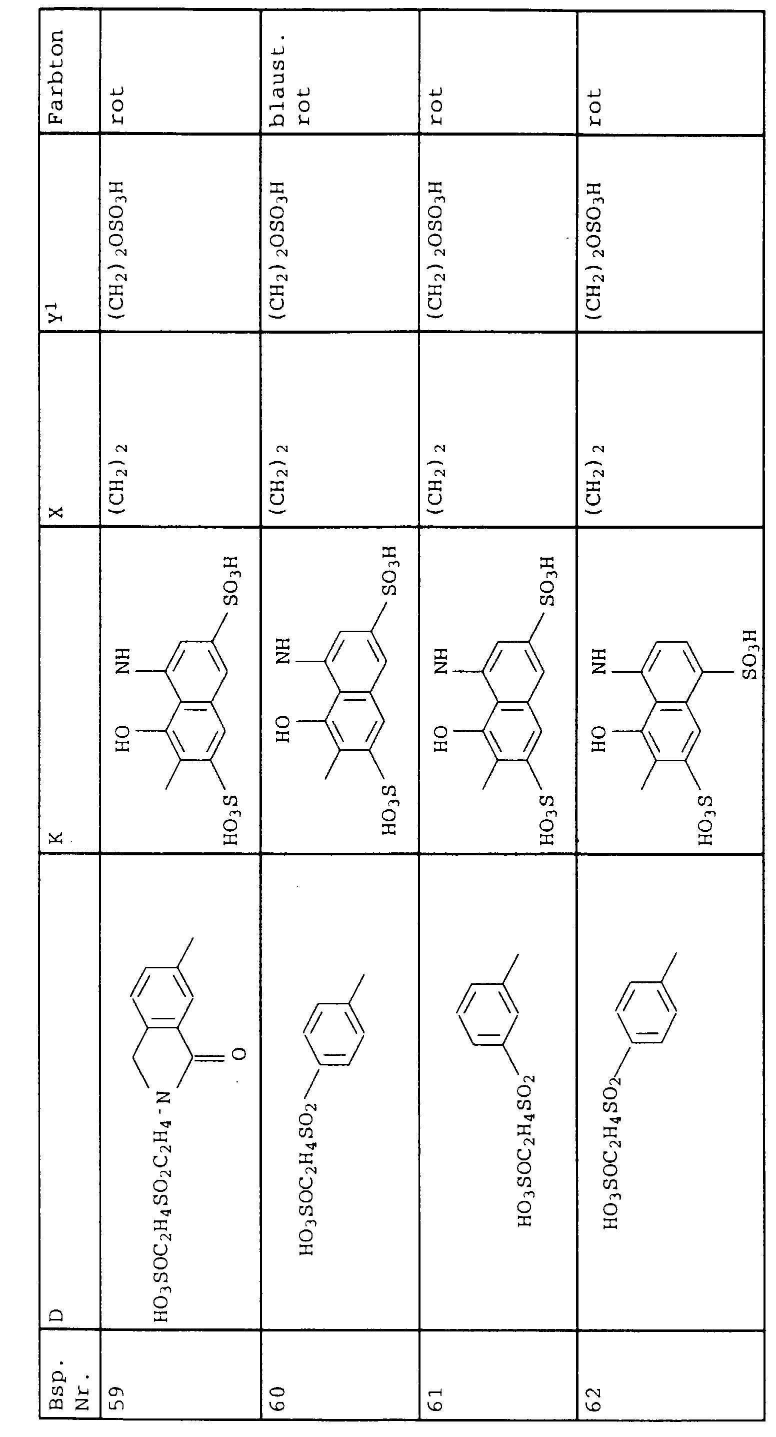

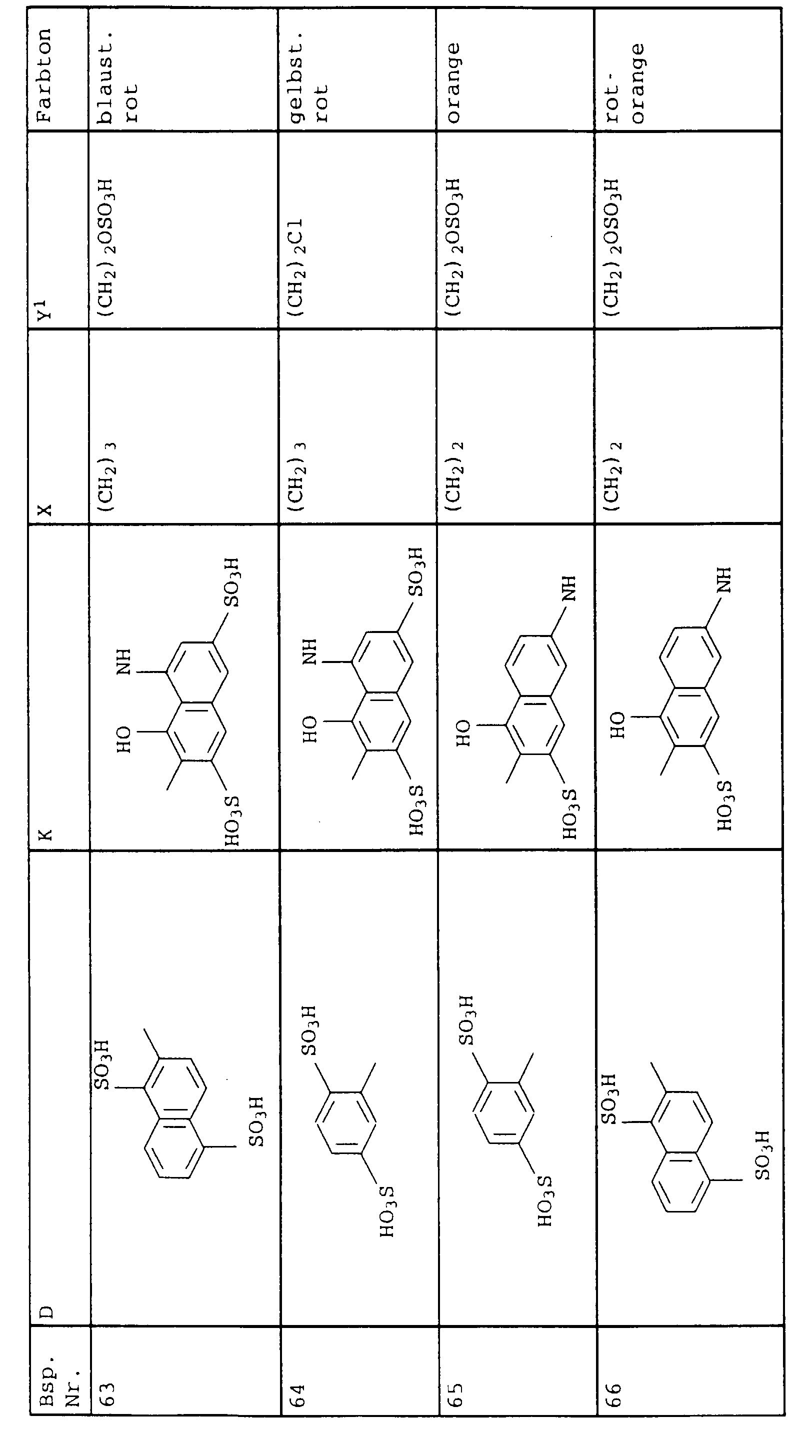

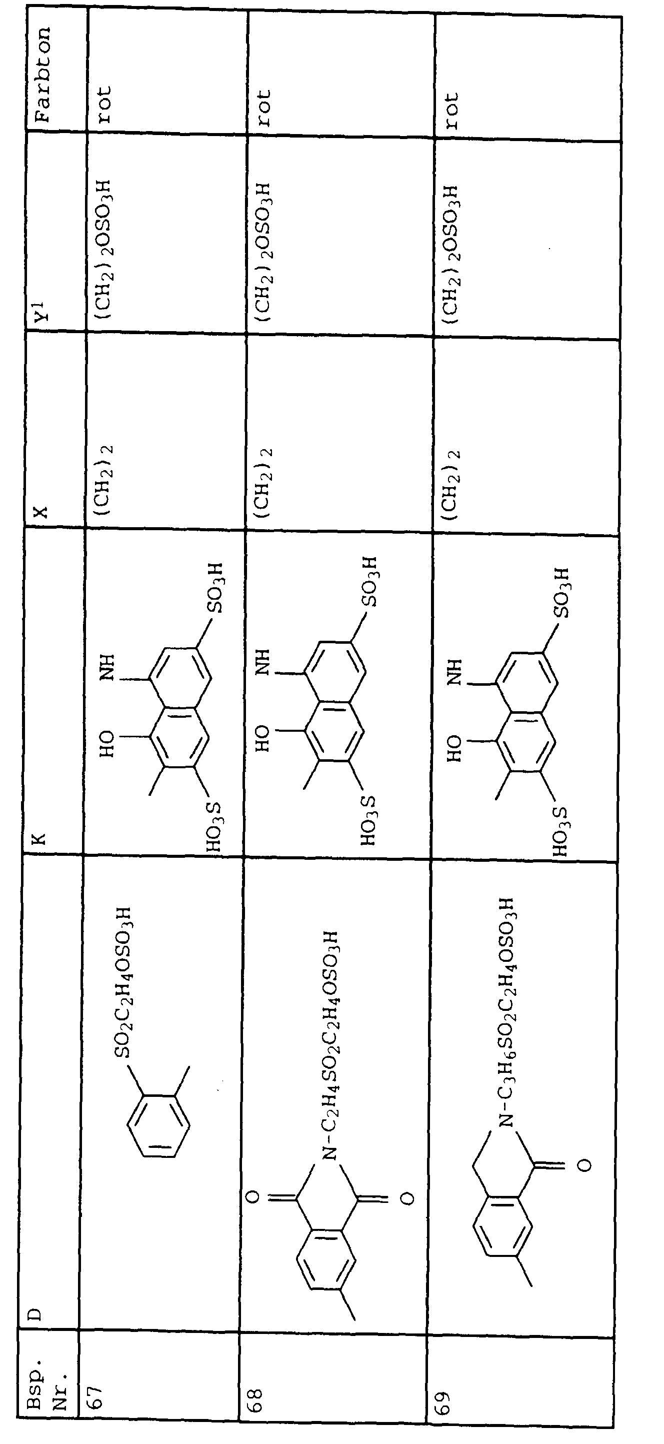

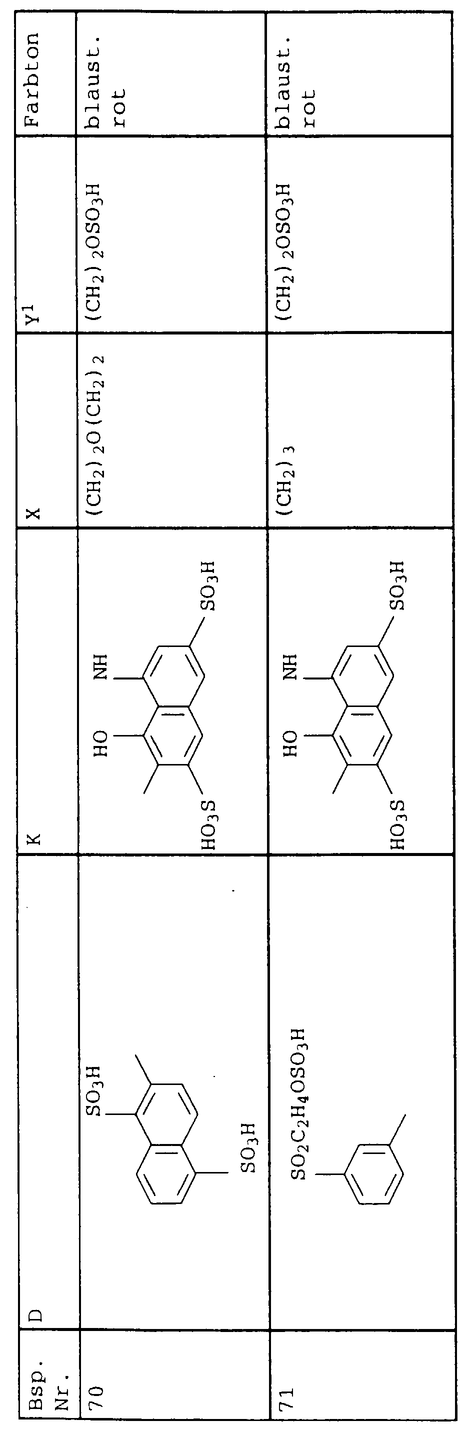

### Beispiel 72

a) 30,3 g 4-(2-Sulfatoethylsulfonyl)anilin-Natriumsalz wurden in 500 ml Eiswasser gelöst und mit 30 ml 3,33 N wäßriger Natriumnitritlösung versetzt. Durch Zugabe von 30 ml 30 gew.-%iger Salzsäure und einstündiges Nachrühren bei 0 bis 5°C wurde diazotiert. Nachdem überschüssige salpetrige Säure mit Amidosulfonsäure zerstört wurde, vereinigte man die erhaltene Dispersion des Diazoniumsalzes mit einer Lösung von 1-(4,6-Dichlor-1,3,5-triazin-2-yl)amino-8-hydroxynaphthalin-4,6-disulfonsäure, die wie folgt bereitet wurde:
   Man ließ eine Lösung von 36,6 g K-Säure Dinatriumsalz und 25 g Natriumformiat in 150 ml Wasser unter intensivem Rühren zu einer eiskalten Dispersion aus 18,5 g Cyanurchlorid, 0,1 g eines Dispergiermittels, 1 ml 30 gew.-%iger Salzsäure und 200 ml Eiswasser fließen und rührte 2 h bei 5 bis 10°C und bei einem pH-Wert von 1 nach, bis die Umsetzung beendet war.
   Durch Pufferung mit Natriumacetatlösung (pH ca. 3) und 30-minütiges Nachrühren bei 10 bis 15°C wurde die Kupplungsreaktion zu Ende geführt.
   Der Dispersion des so erhaltenen Dichlortriazinylazofarbstoffes setzte man sodann 34,1 g H-Säure-Mononatriumsalz zu und stellte durch Einstreuen von Natriumhydrogencarbonat einen pH-Wert von 5 bis 5,5 ein, wobei die Temperatur auf 20°C anstieg. Nach zweistündigem Rühren bei 20 bis 25°C und Einhalten des obengenannten pH-Wertes war die Reaktion beendet und der Farbstoff der Formel 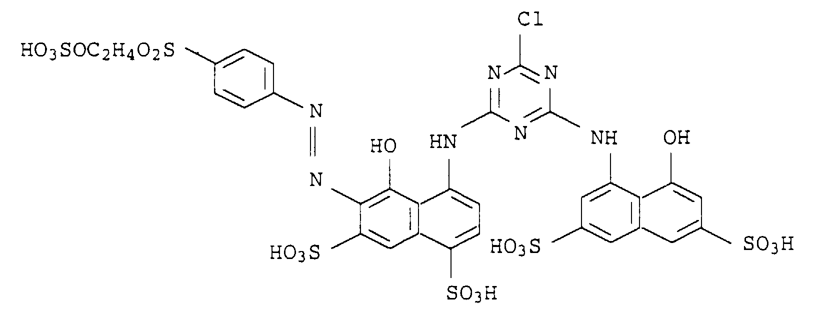 wurde mittels Kaliumchlorid vollständig ausgefällt, abgesaugt und mit 5 gew.-%iger wäßriger Kaliumchloridlösung nachgewaschen.
b) 0,05 mol des unter a) beschriebenen Farbstoffes wurden mit der unter Beispiel 1b) beschriebenen Ankerverbindung versetzt. Pufferung mit Natriumacetat bis zu einem pH-Wert von 5 vervollständigte die Kupplungsreaktion. Der Farbstoff der Formel 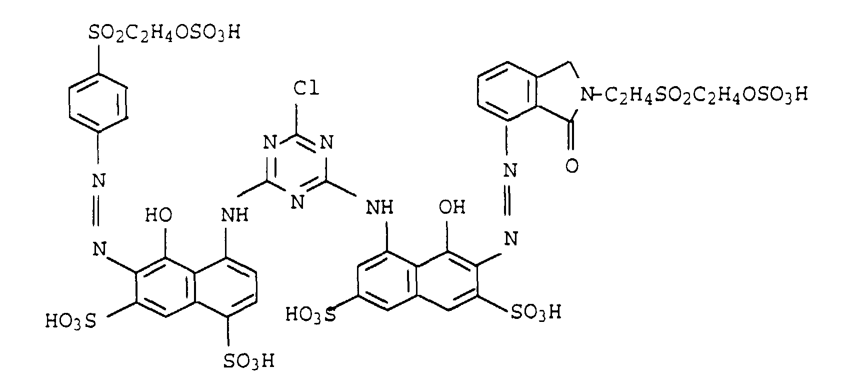 wurde mit 150 g Kaliumchlorid gefällt, abgesaugt, mit 20 gew.-%iger wäßriger Kaliumchloridlösung gewaschen und bei 40°C unter vermindertem Druck getrocknet.
   Der Farbstoff färbt Baumwolle gleichermaßen nach dem Warmauszieh- und Kaltverweilverfahren in klaren, neutralen Rottönen mit sehr hoher Farbstärke und sehr hohem Fixiergrad bei hoher Unempfindlichkeit gegenüber Temperaturschwankungen.

### Beispiel 73

Man ersetzte das in Beispiel 72 a) verwendete Sulfatoethylsulfonylanilin durch eine äquivalente Menge der unter Beispiel 1 b) beschriebene Ankerverbindung und verfuhr dann analog Beispiel 72 b). Man erhielt den Farbstoff der Formel

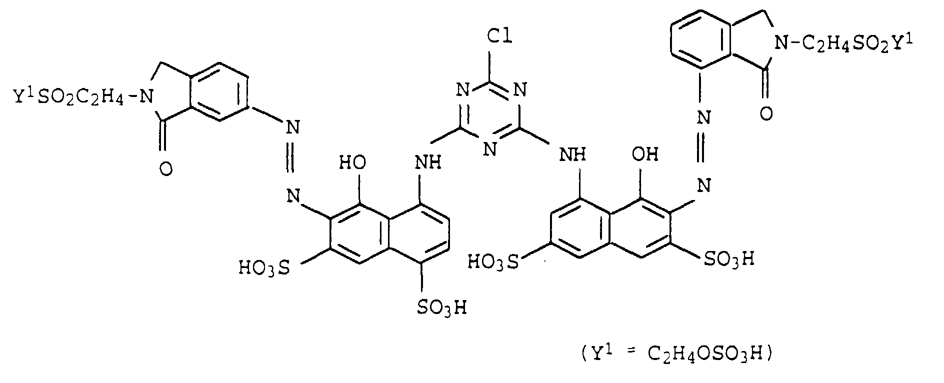

Analog den Beispielen 72 und 73 werden die folgenden Farbstoffe erhalten, wobei Y¹ jeweils den Rest C₂H₄OSO₃H bedeutet.

### Beispiel 74

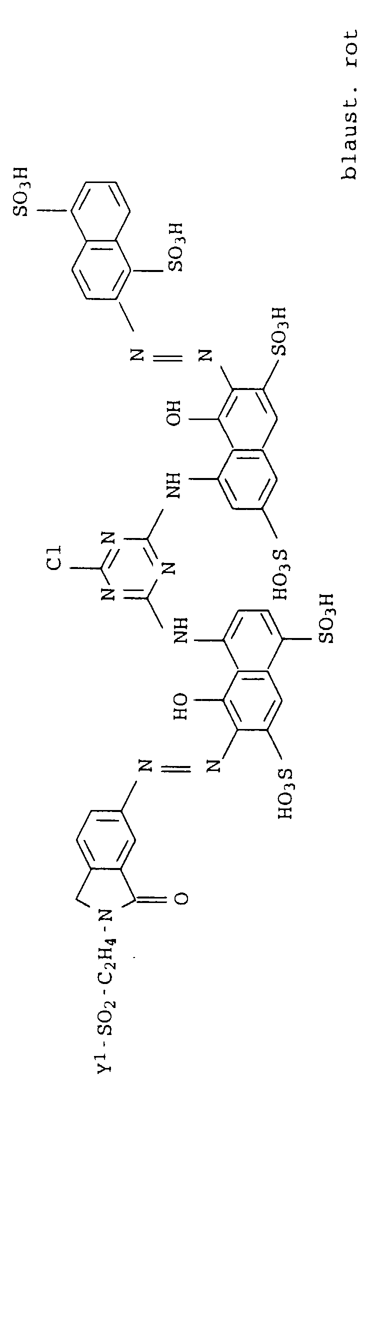

### Beispiel 75

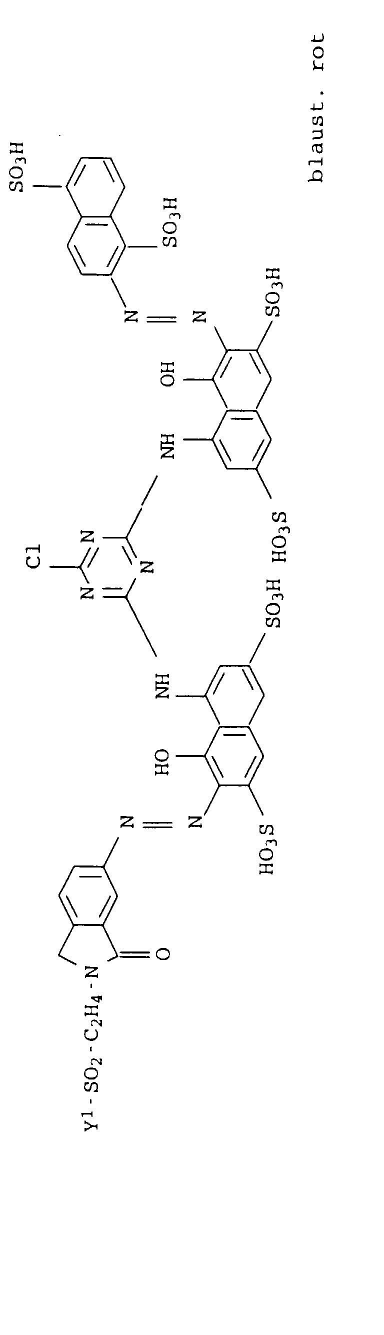

### Beispiel 76

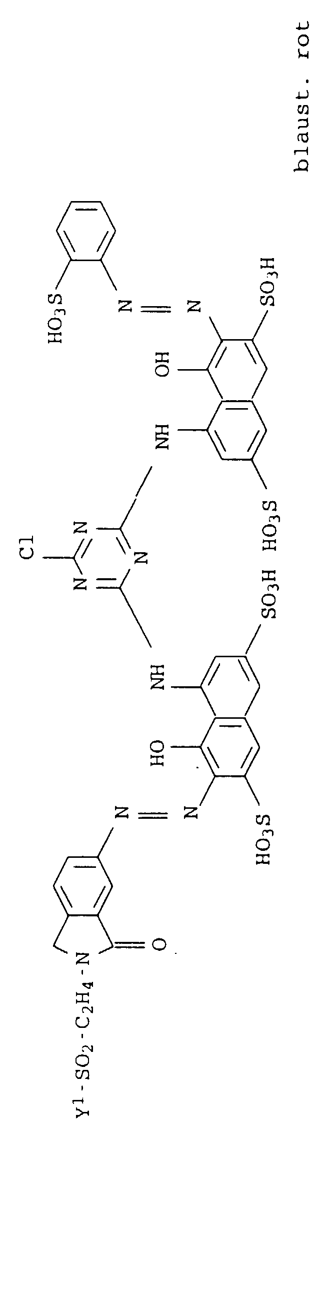

### Beispiel 77

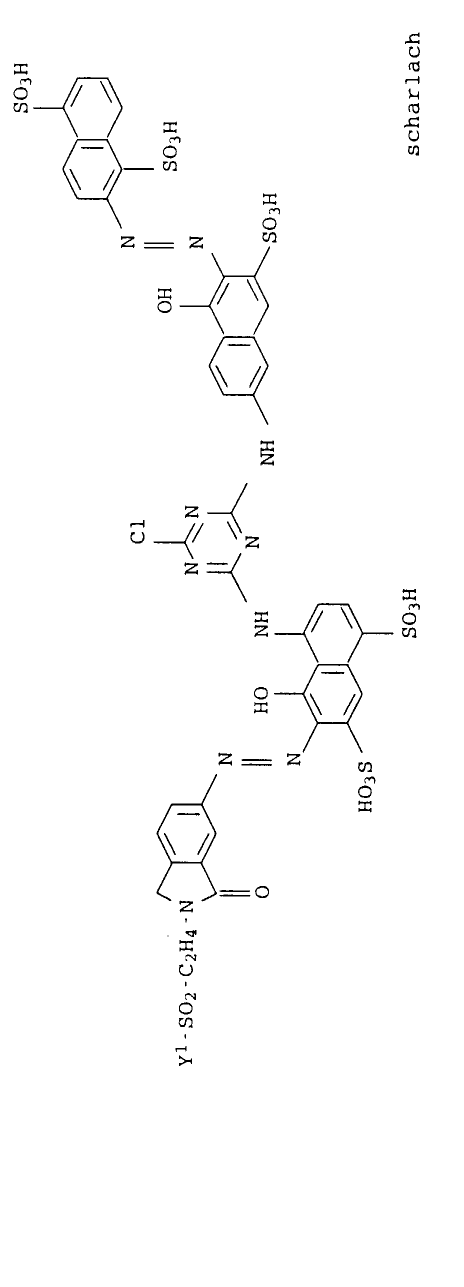

### Beispiel 78

Die unter 1 b) beschriebene Ankerverbindung wurde mit der Dispersion von 16,5 g H-Säure-Mononatrium-salz in 150 ml Wasser vereinigt und bei Raumtemperatur über Nacht gerührt.

Der gebildete Monoazofarbstoff wurde mit einem weiteren Äquivalent der unter Beispiel 1 b) beschriebenen Ankerverbindung vereinigt und mit Natriumacetat bei einem pH-Wert von 5 gekuppelt. Der Farbstoff der Formel 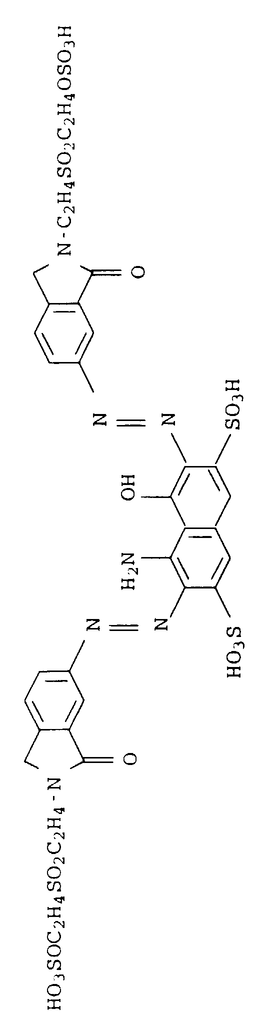 wurde als Kaliumsalz gefällt, abgesaugt, mit 70 gew.-%igem wäßrigen Ethanol gewaschen und bis 40°C unter vermindertem Druck getrocknet.

Er färbt Baumwolle in neutralen Marineblau- und Schwarztönen bei weitgehender Unbhängigkeit von angewandten Färbeverfahren und -temperaturen.

In analoger Weise werden die in der folgenden Tabelle 4 aufgeführten Farbstoffe erhalten.

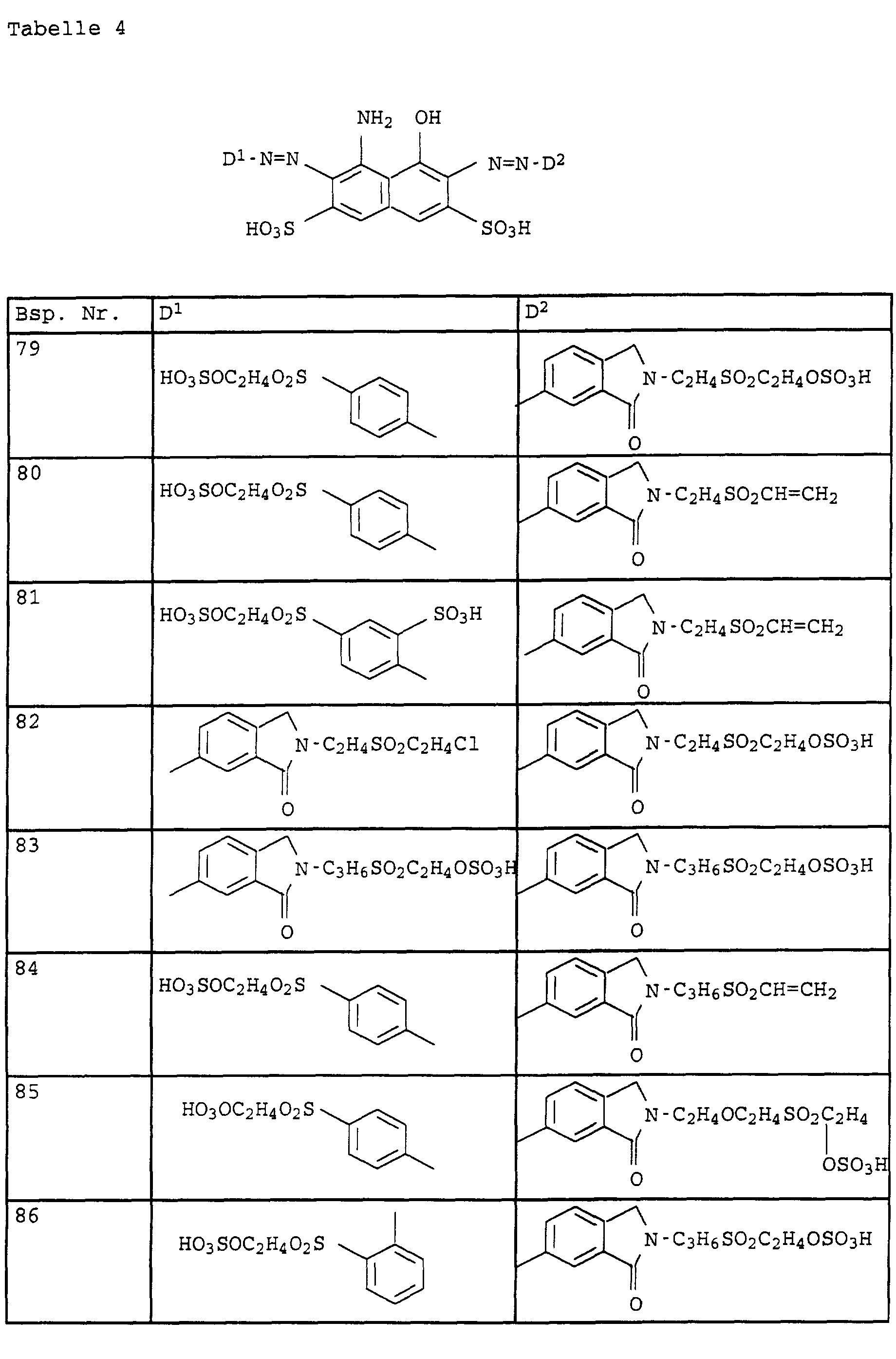

### Beispiel 87

a) 32 g der Verbindung der Formel 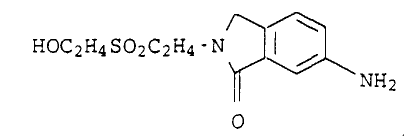 wurden in 300 ml Wasser und 30 ml 30 gew.-%iger Salzsäure angerührt und nach Kühlung mit 100 g Eis mit 30 ml 3,33 N wäßriger Natriumnitritlösung diazotiert. Nach 30-minütigem Rühren bei 0 bis 5 °C wurde der geringe Überschuß an salpetriger Säure mit Amidosulfonsäure zerstört und die Diazoniumsalzlösung mit der Lösung von 10,7 g m-Toluidin in 100 ml Wasser und 10 ml 30 gew.-%iger Salzsäure vereinigt. Mit einer gesättigten Natriumacetatlösung wurde ein pH-Wert von 1,5 eingestellt und bei Raumtemperatur über Nacht gehalten. Das Hydrochlorid des Farbstoffes der Formel 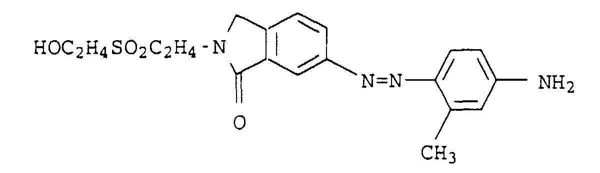 wurde abgesaugt, mit salzsaurem Wasser gewaschen und bei 60°C unter vermindertem Druck getrocknet.
b) 30 g der unter a) beschriebenen Verbindung wurden in 200 g konz. Schwefelsäure eingerührt und 3 h bei 40°C gehalten, bis weitgehend der Schwefelsäureester entstanden war. Durch Zusatz von 90 g 65 gew.-%igem Oleum und zweistündiges Rühren bei 80°C trat Sulfonierung ein. Der resultierende Farbstoff der Formel 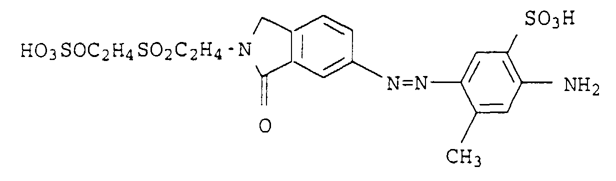 wurde auf 1000 g Eis ausgerührt und abgesaugt.
c) Das wäßrige Schwefelsäure enthaltende Nutschgut aus Beispiel 87b wurde in 150 ml Wasser angerührt und bei 10 bis 15°C mit 11 ml 3,33 N wäßriger Natriumnitritlösung versetzt. Nach 30-minütigem Rühren bei 10 bis 15°C wurde der Überschuß an salpetriger Säure zerstört und die Diazoniumsalzlösung mit der Lösung von 24 g der Verbindung der Formel 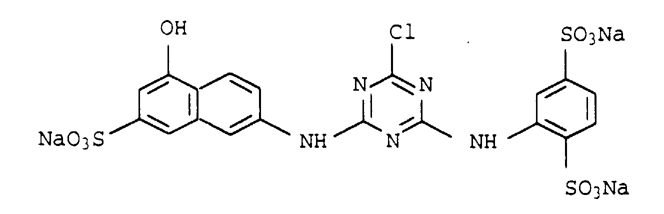 in 150 ml Wasser versetzt. Durch Pufferung mit wäßriger Natriumacetatlösung bis zu einem pH-Wert von 5 erfolgte die Kupplung zum Farbstoff der Formel 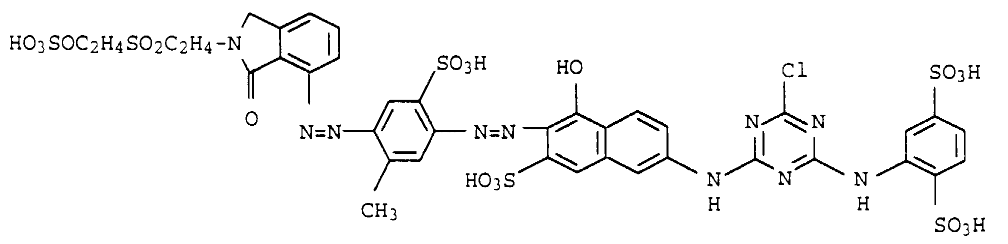 der als Natriumsalz isoliert wurde. Er färbt Baumwolle in klaren Ziegelrottönen mit gutem Echtheitsprofil.

### Beispiel 88

Die Lösung des Reaktionsproduktes von Cyanurchlorid mit H-Säure (wie unter 52a) beschrieben) wurde mit der in Beispiel 1 b) beschriebenen Ankerverbindung unter den dortigen Bedingungen gekuppelt. Nach Zusatz von 5,4 g p-Phenylendiamin wurde ca. 6 Stunden bei Raumtemperatur und bei einem pH-Wert von 6 gerührt.

Nach Erwärmen auf 40°C und einem pH-Wert von 5 war die Kondensation vollendet und es wurde ein Farbstoff der Formel 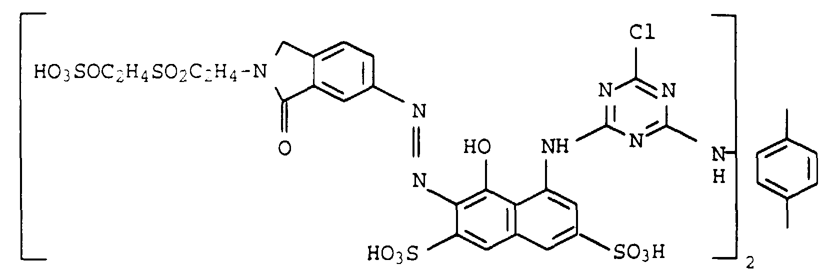 erhalten. Der Farbstoff wurde mit Kochsalz gefällt, abgesaugt, mit 15 gew.-%iger wäßriger Kochsalzlösung gewaschen und bei 40°C unter vermindertem Druck getrocknet. Er färbt Baumwolle in blaustichig rotem Ton mit hoher Brillanz und sehr hoher Farbausbeute.

### Beispiel 89

Man kondensierte zunächst den in Beispiel 88 durch Kupplung erhaltenen Farbstoff der Formel 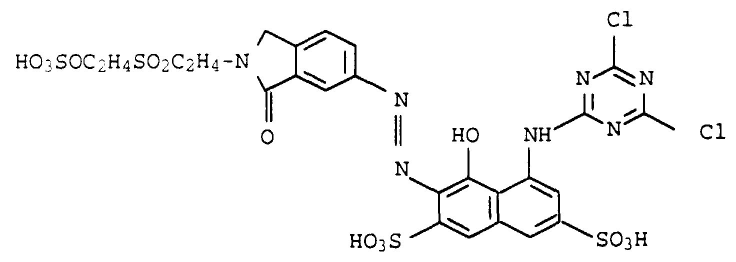 mit der äquivalenten Menge 2-Methylamino-4-aminobenzolsulfonsäure bei einem pH-Wert von 5 und bei 25°C. Danach setzte man ihm unter gleichen Reaktionsbedingungen mit dem in Beispiel 72 a) beschriebenen Farbstoff der Formel 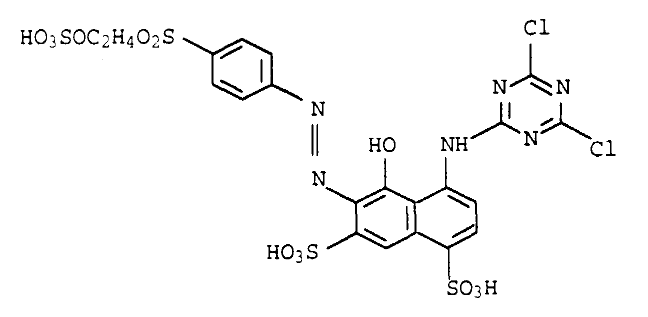 um. Man erhielt den Farbstoff der Formel 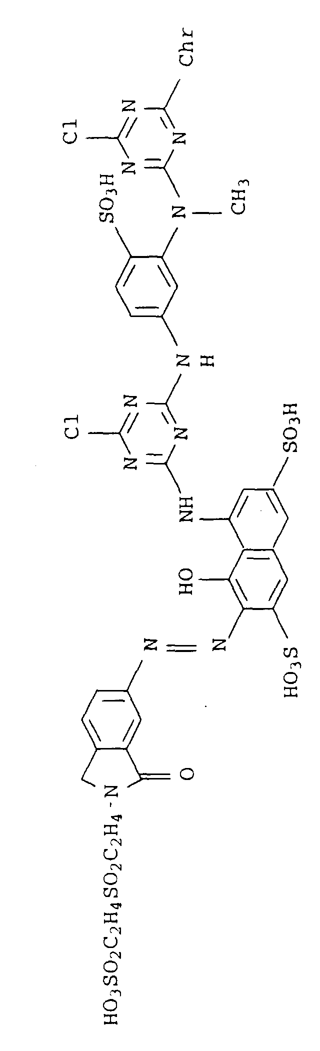 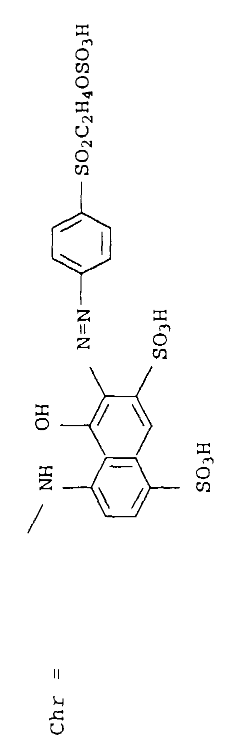 in hoher Ausbeute. Er färbt Baumwolle in neutral rotem Farbton.

In analoger Weise werden die in den folgenden Tabellen 5 und 6 aufgeführten Farbstoffe erhalten.

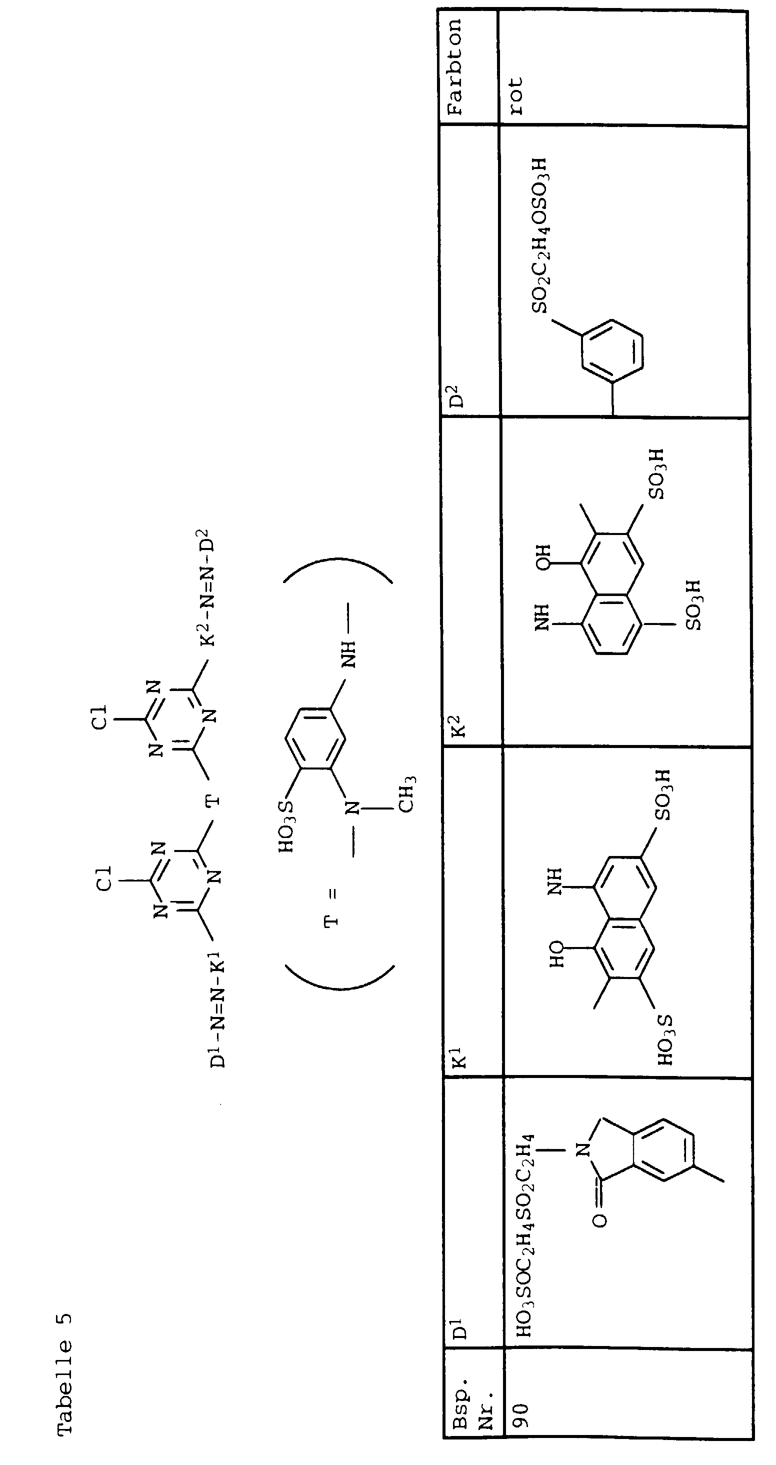

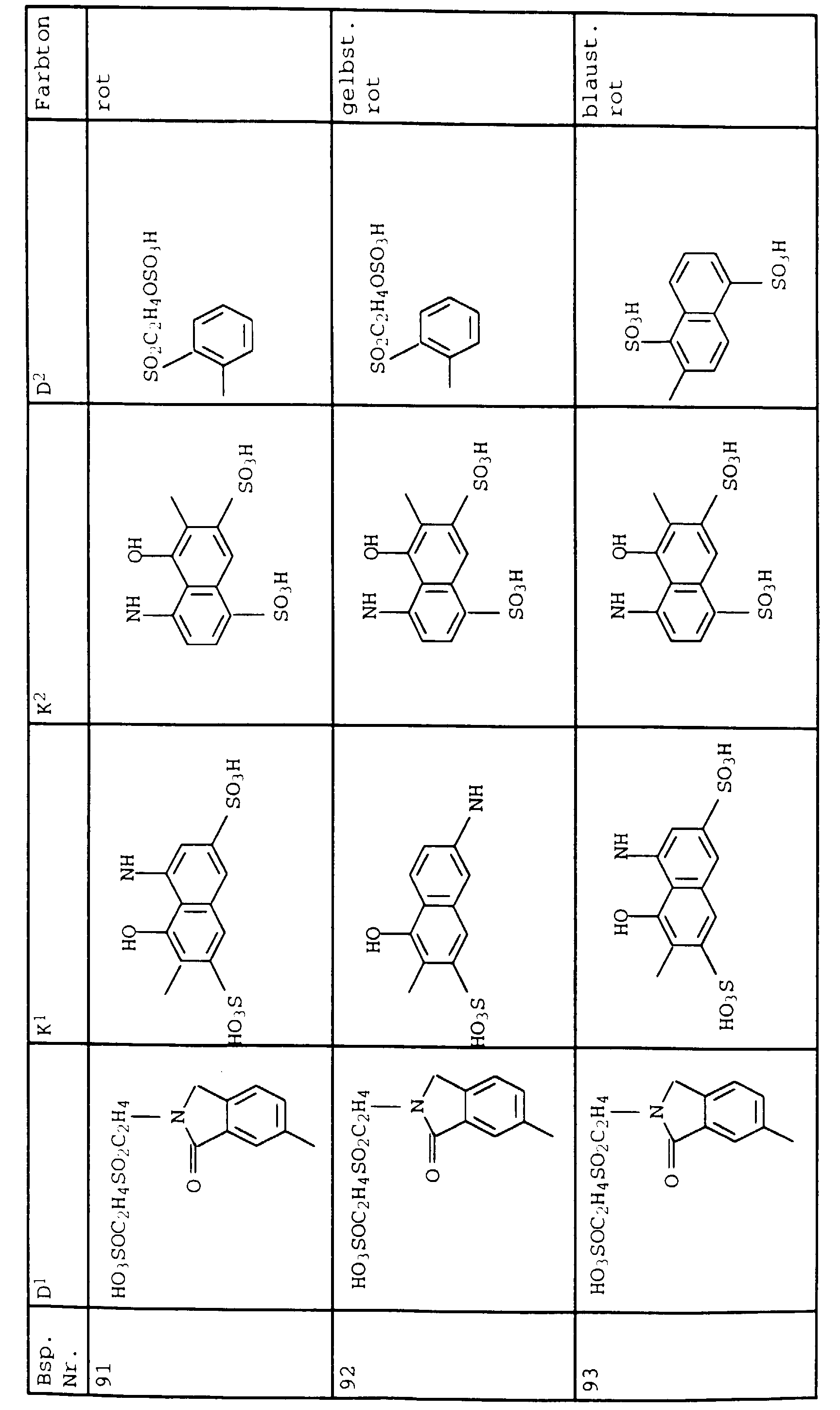

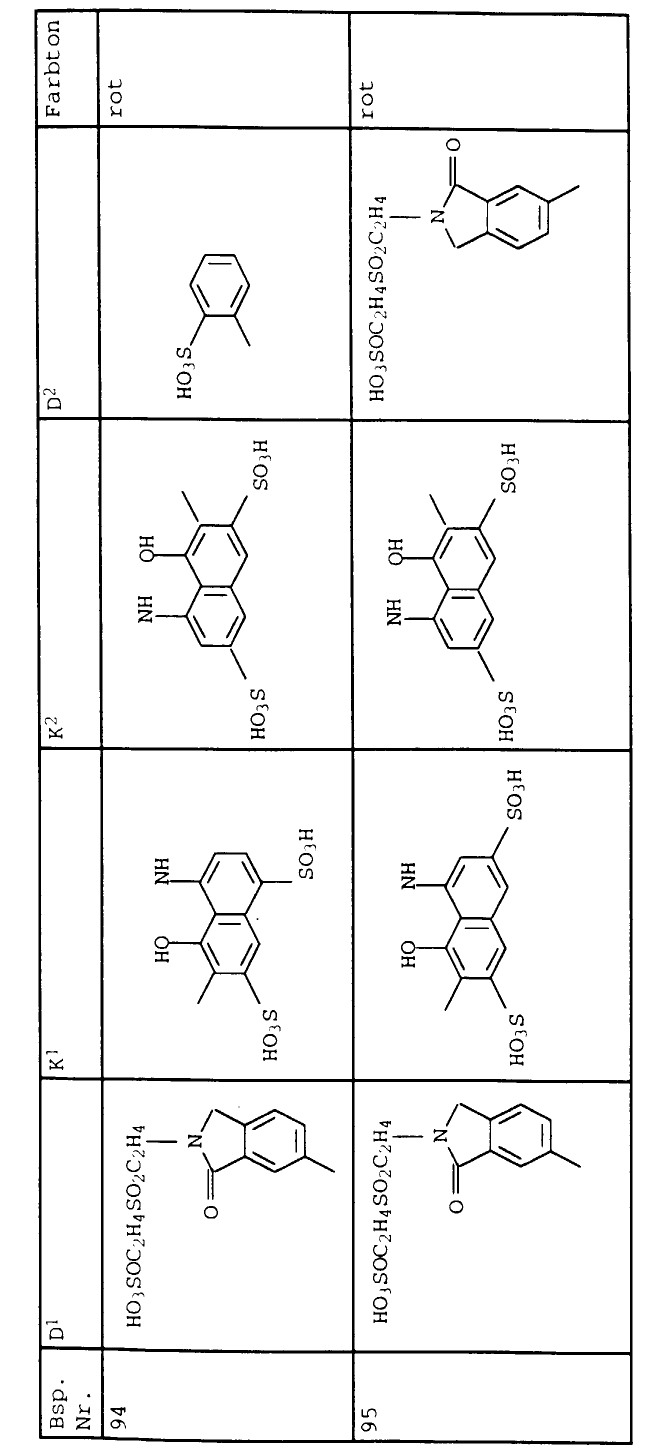

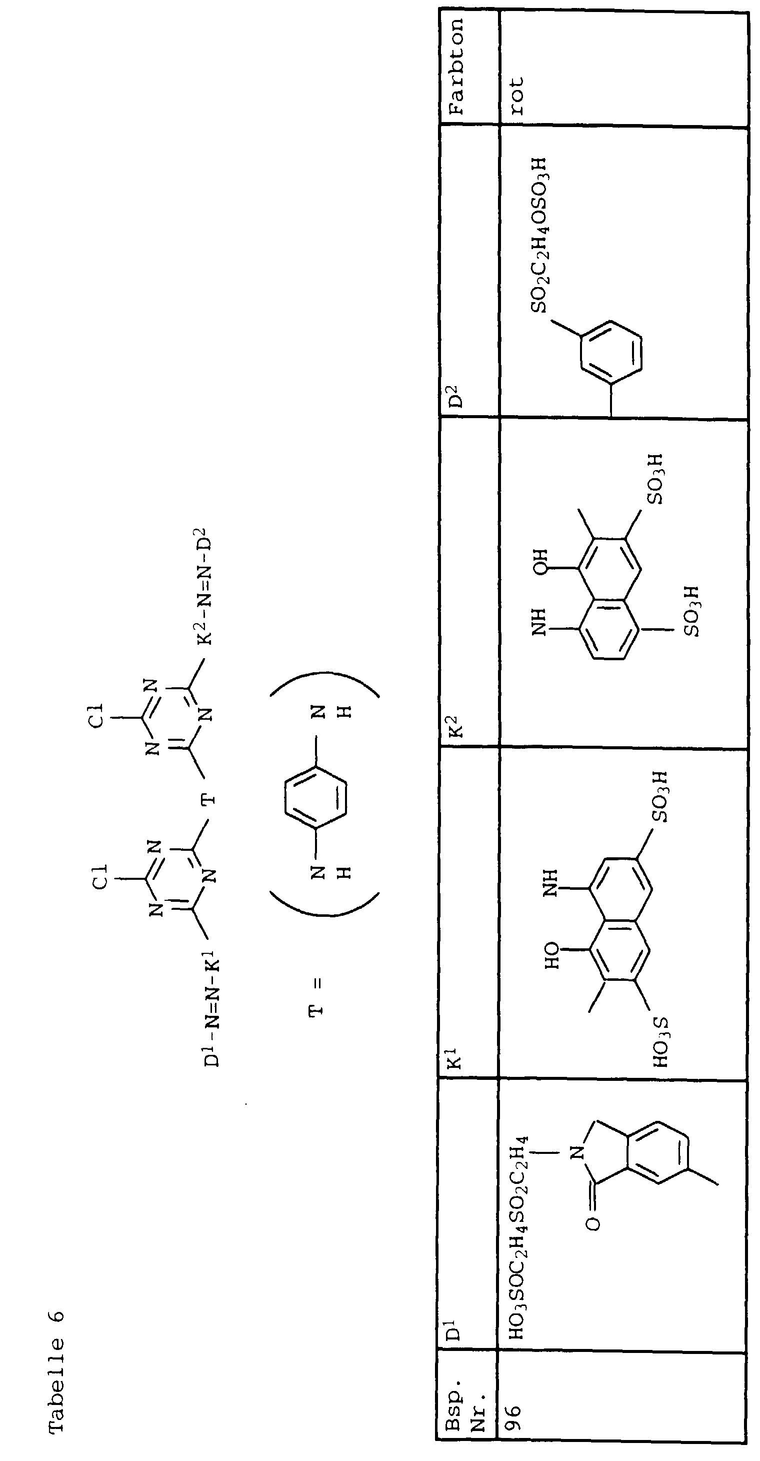

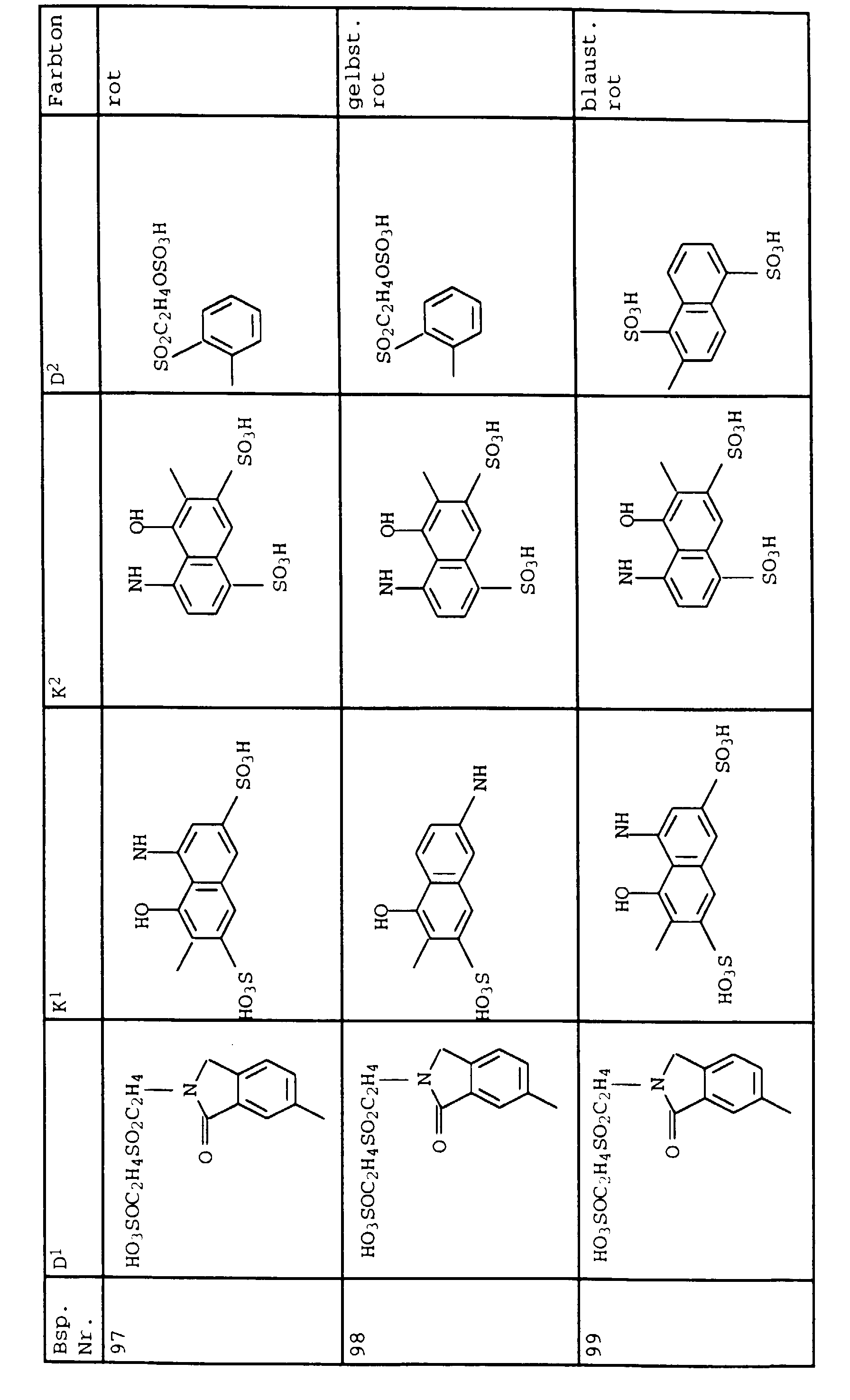

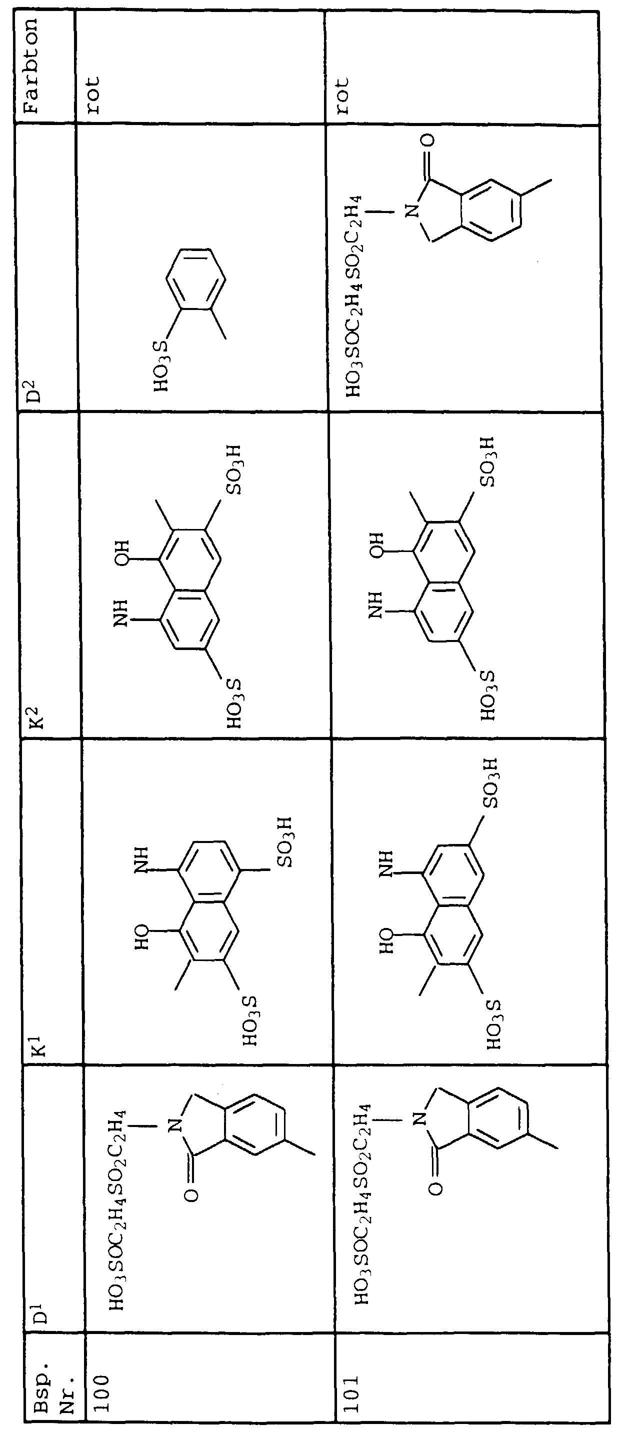

### Beispiel 102

In 400 ml Wasser wurden 42 g Bromaminsäure, 56 g der Verbindung der Formel 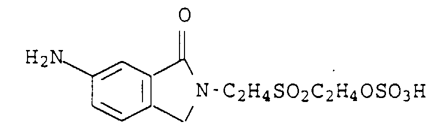 3 g Kupfer(I)chlorid und 21 g Natriumhydrogencarbonat langsam auf 50° erwärmt und weitere 5 h bei 50°C gerührt. Man kühlte dann auf 5°C ab, saugte ab und wusch mit 10 gew.-%iger wäßriger Natriumchloridlösung. Nach Trocknen unter vermindertem Druck isolierte man 57 g des Farbstoffs der Formel 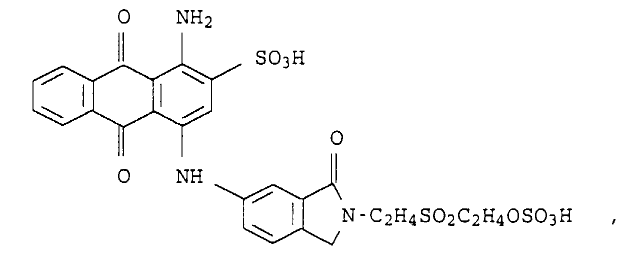 der Baumwolle in brillanten blauem Ton färbt.

### Beispiel 103

25 g Kupferphthalocyanintetrasulfonsäurechlorid wurden in 250 ml Eiswasser suspendiert. Dazu gab man 38 g der Verbindung der Formel 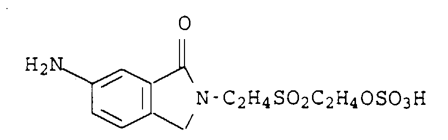 und 0,8 g Nicotinsäureamid zu und stellte den pH-Wert mit 10 gew.-%iger wäßriger Natriumhydrogencarbonatlösung auf 6,0 bis 6,5. Das Reaktionsgemisch rührte 8 h bei Raumtemperatur, wobei der pH-Wert durch weitere Zugabe von wäßriger Natriumhydrogencarbonatlösung bei 6,0 bis 6,5 gehalten wurde. Nach Klärfiltration wurde der Farbstoff durch Zugabe von Aceton ausgefällt, abgesaugt und getrocknet. Man isolierte 52 g des Farbstoffs der Formel 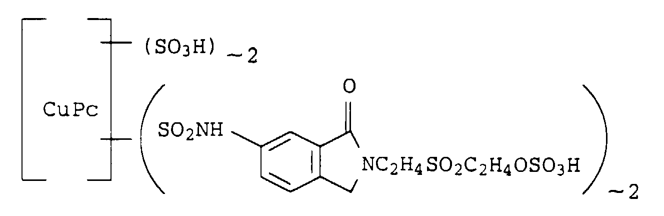 der Baumwolle in brillanten Türkistönen färbt.

### Beispiel 104

40,0 g der Verbindung der Formel 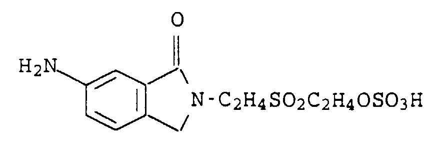 wurden in 250 ml Wasser bei 0 bis 5°C suspendiert. Der pH-Wert wurde mit 18 gew.-%iger Salzsäure auf 1 erniedrigt, dann wurde durch Eintropfen von 30 ml 3,33 N wäßriger Natriumnitritlösung diazotiert.

Nach 30-minütigem Rühren bei 0 bis 5°C wurde der leichte Nitritüberschuß mit Amidosulfonsäure entfernt und die Suspension des Diazoniumsalzes anschließend mit 19,6 g des Pyridons der Formel 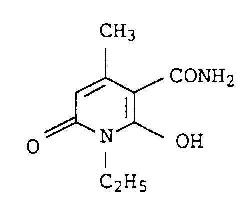 versetzt.

Der pH-Wert wurde durch Zugabe von Natriumcarbonat auf 4,5 bis 5,5 erhöht, dann wurde 4 h bei 0 bis 5°C und dem genannten pH-Wert bis zur vollständigen Kupplung nachgerührt.

Durch Fällung mit 100 g Natriumchlorid, Absaugen und Trocknen unter vermindertem Druck wurden 74 g elektrolythaliger Farbstoff der Formel 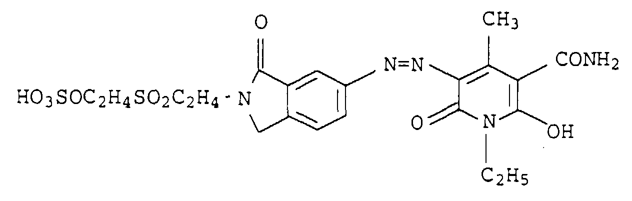 erhalten (λₘₐₓ (Wasser): 418 nm).

Analog Beispiel 104 werden die in der folgenden Tabelle 7 aufgeführten Farbstoffe erhalten.

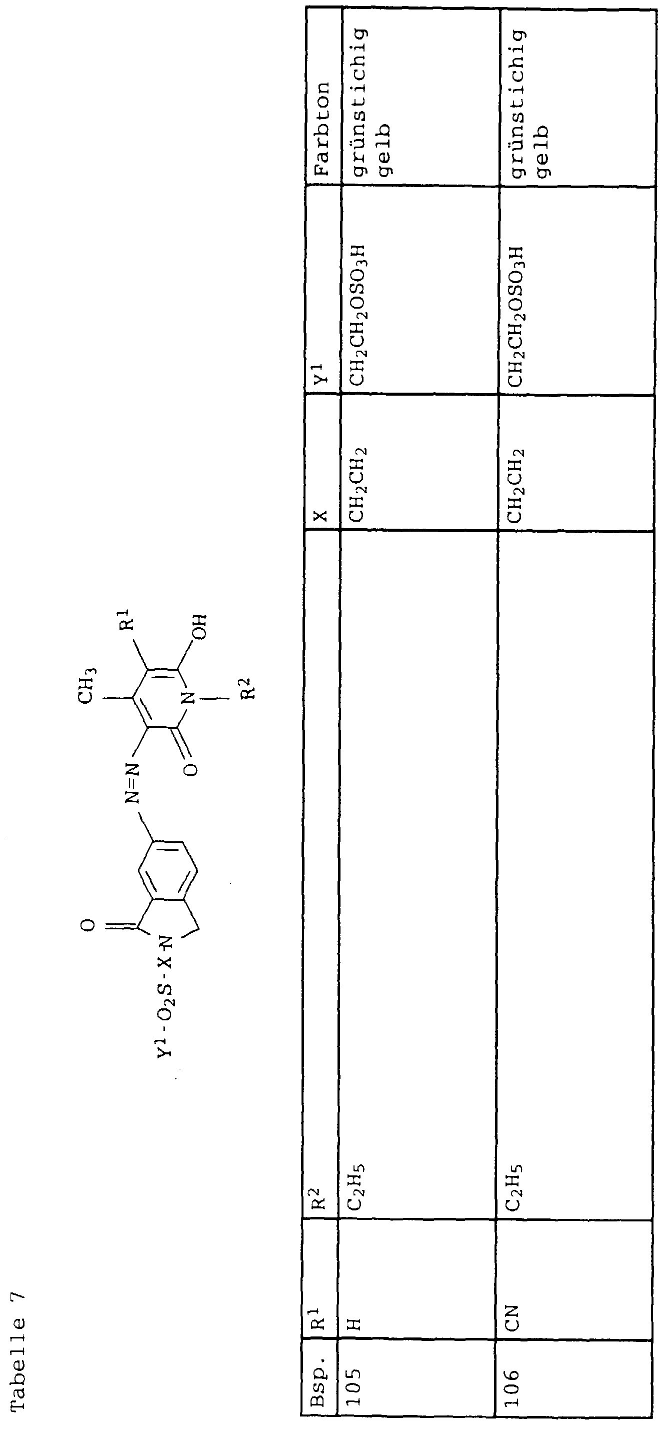

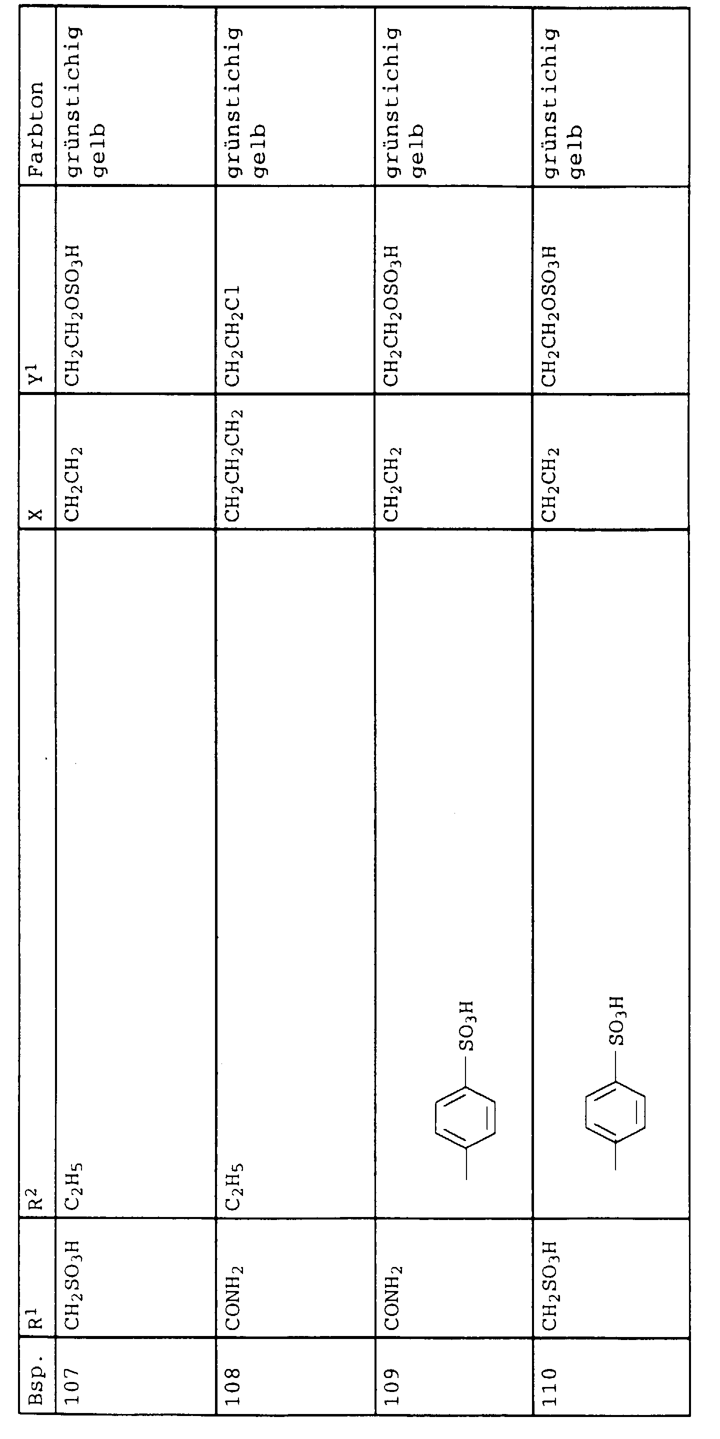

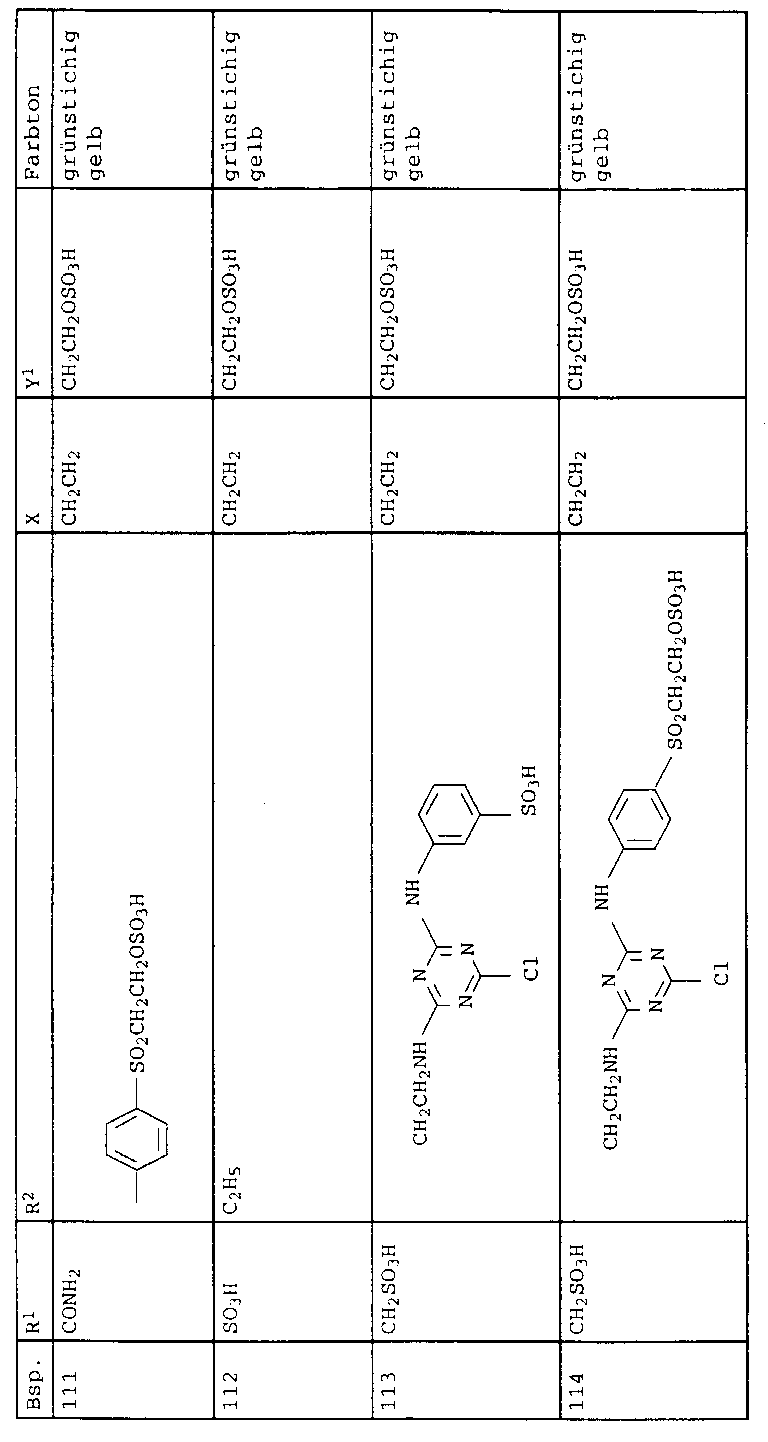

### Beispiel 115

Man verfuhr analog Beispiel 104, verwendete jedoch als Kupplungskomponente die Pyrazolverbindung der Formel 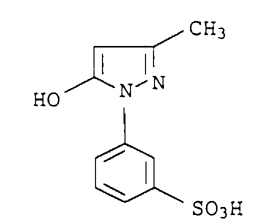 und erhielt nach Fällung mit Natriumchlorid, Absaugen und Trocknen unter vermindertem Druck den Farbstoff der Formel 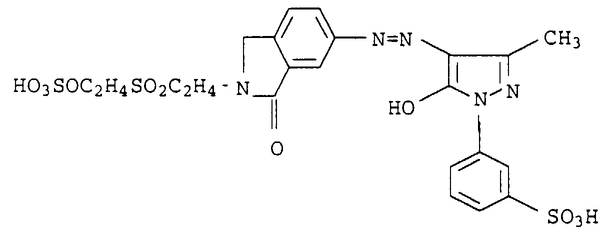 der Baumwolle nach den für Reaktivfarbstoffen üblichen Verfahren in klaren grünstichig gelben Tönen färbt.

Unter Verwendung vergleichbarer Kupplungskomponenten können die in der folgenden Tabelle 8 aufgeführten Farbstoffe erhalten werden.

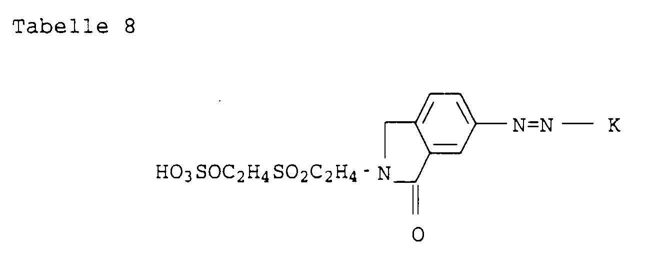

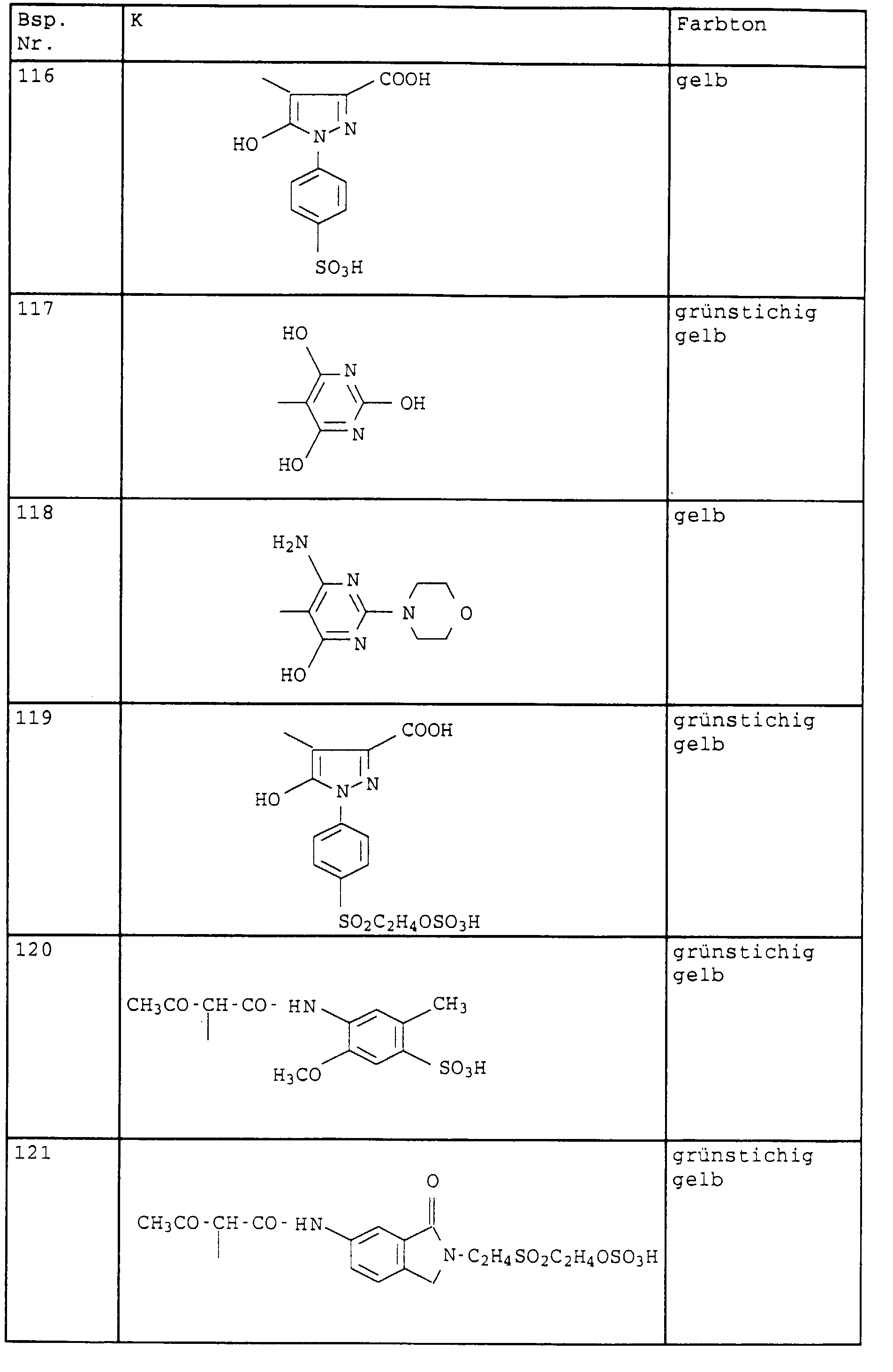

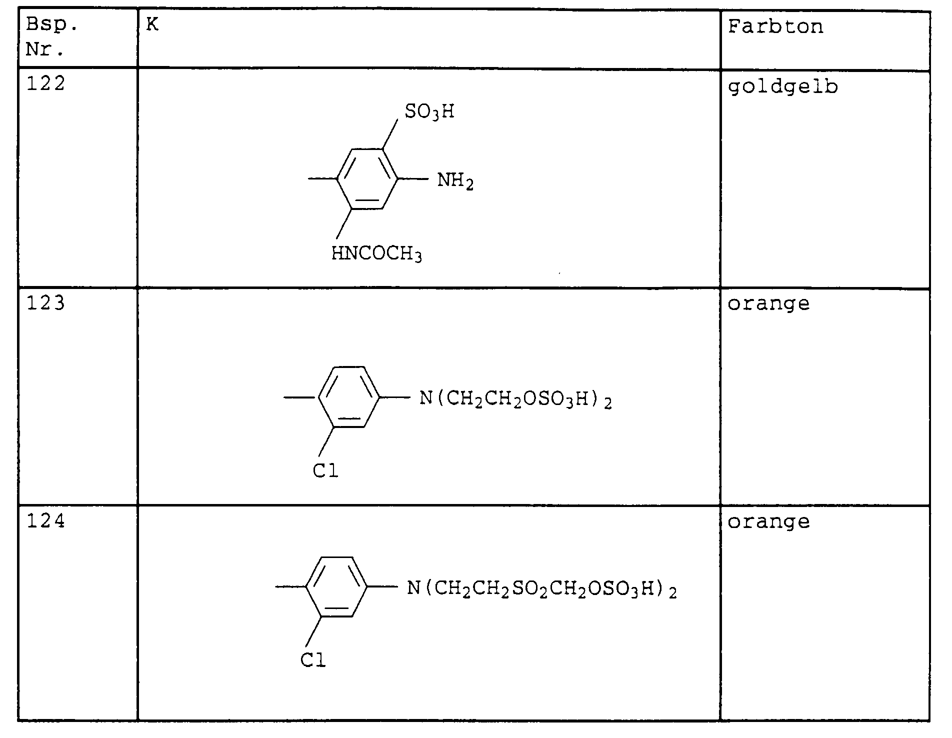

### Beispiel 125

54,8 g des Farbstoffs der Formel 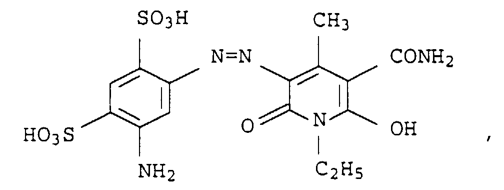 erhalten durch Diazotierung von 2,4-Diaminobenzol-1,5-disulfonsäure und kuppeln bei pH 5 mit 1-Ethyl-2-hydroxy-3-carbamoyl-4-methylpyrid-6-on, wurden in 350 ml Wasser bei einem pH-Wert von 5,5 gelöst und auf 0 bis 5°C abgekühlt. Anschließend wurden 18,4 g Cyanurchlorid, gelöst in 100 ml Aceton, zugetropft und dann 3 h bei 0 bis 5°C bis zur vollständigen Umsetzung nachgerührt. Während des Zutropfens und Nachrührens wurde der pH-Wert durch Zugabe von 5 gew.-%iger wäßriger Natriumhydrogencarbonatlösung zwischen 5,0 und 5,5 gehalten.

Nach Zugabe von 48 g der Verbindung der Formel 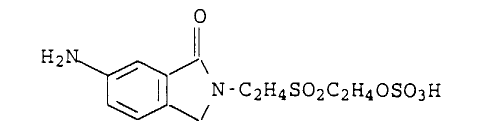 wurde auf 40 bis 45°C erwärmt und 5 h bei dieser Temperatur nachgerührt, wobei der pH-Wert mit 5 gew.-%iger wäßriger Natriumhydrogencarbonatlösung zwischen 5,5 und 6,0 gehalten wurde.

Nach Fällung mit 200 g Natriumchlorid, Absaugen und Trocknen wurde der Farbstoff der Formel 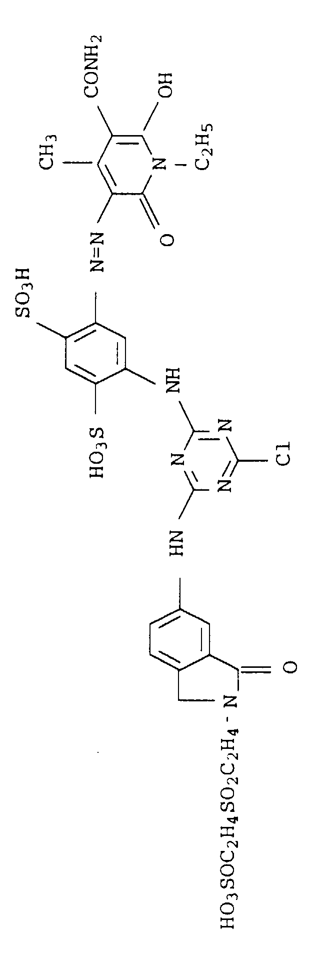 isoliert, der Baumwolle entweder nach dem Ausziehverfahren bei 40 bis 50°C oder dem Kaltverweilverfahren in grünstichigen Gelbtönen färbt.

In analoger Weise werden die in der folgenden Tabelle 9 aufgeführten Farbstoffe erhalten.

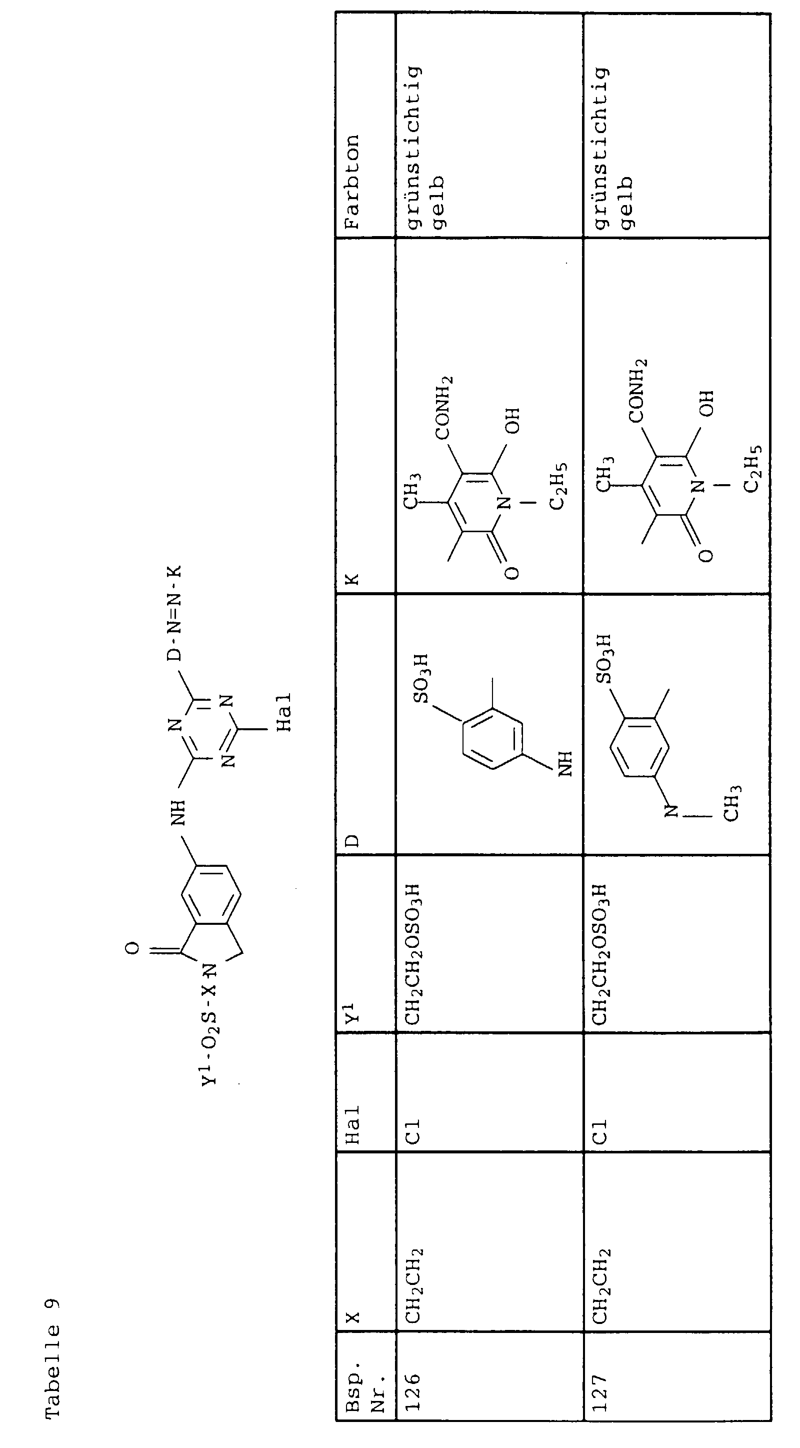

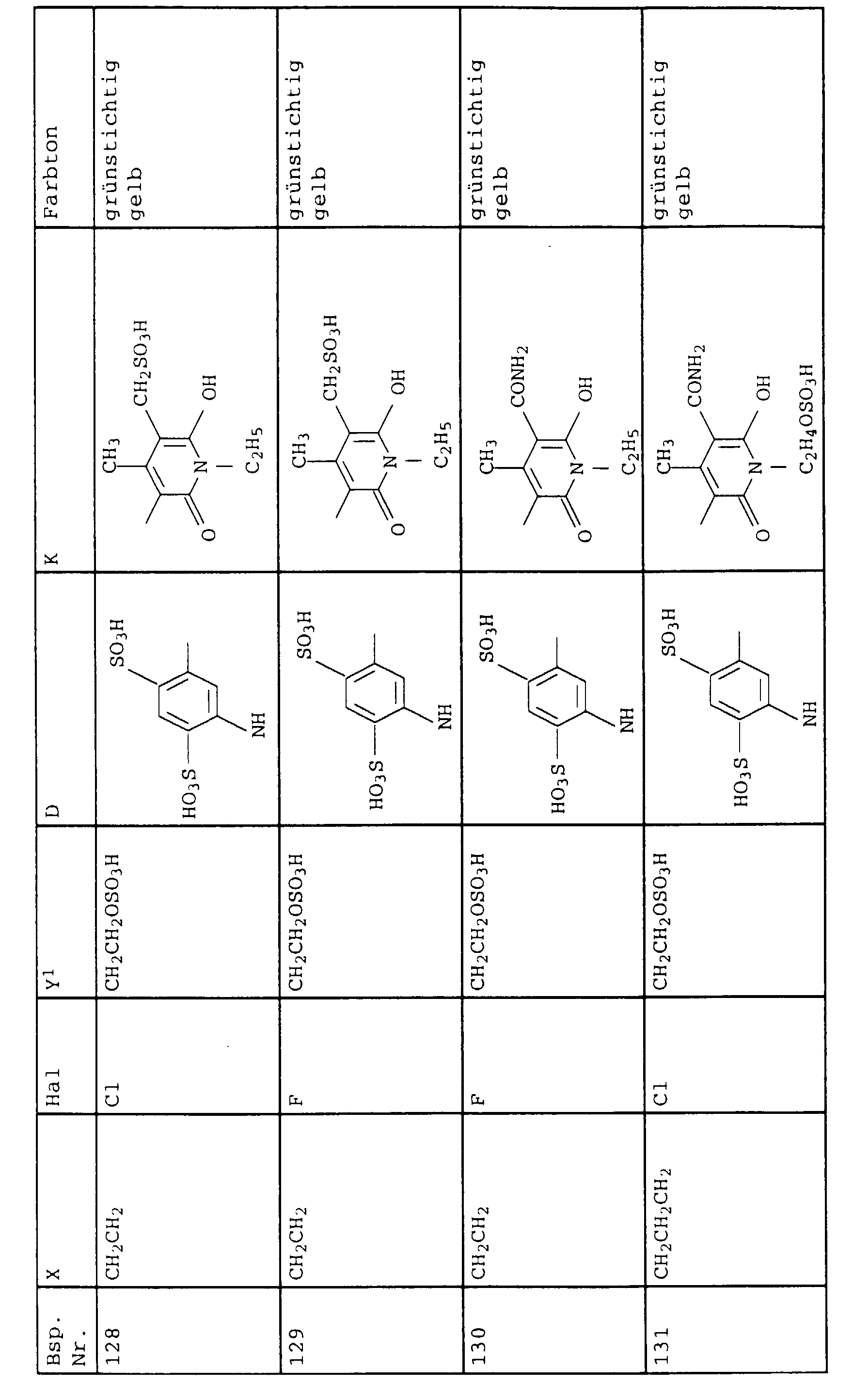

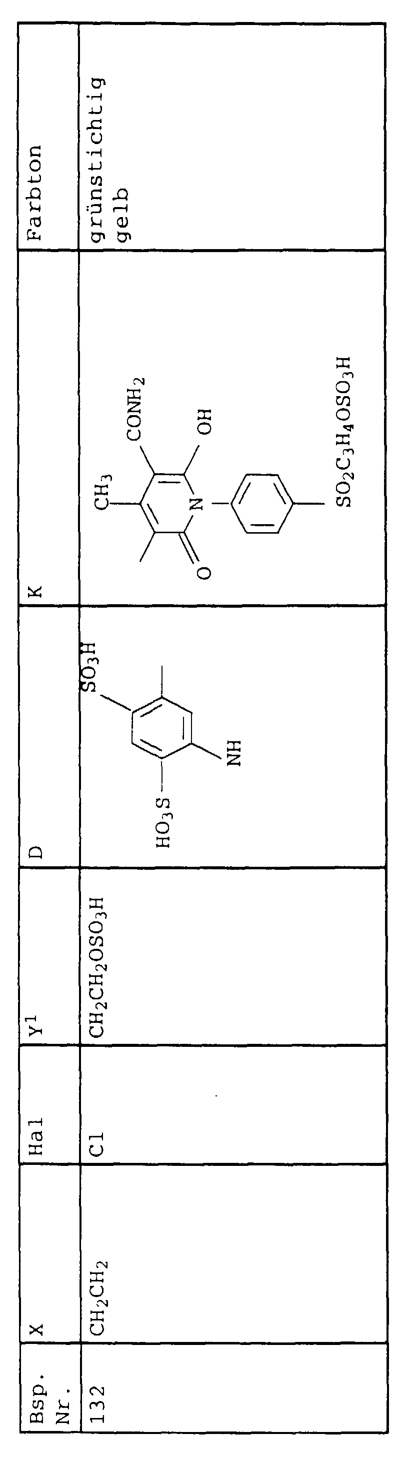

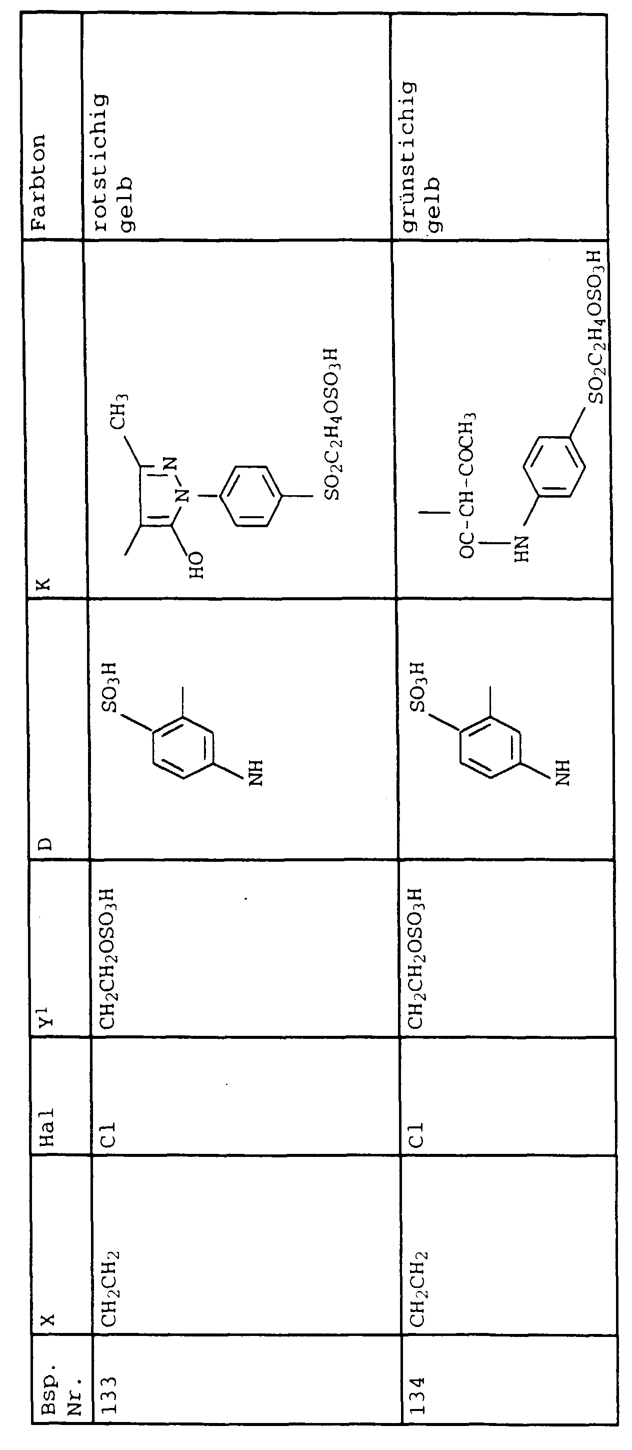

### Beispiel 135

85,1 g des Farbstoffs der Formel 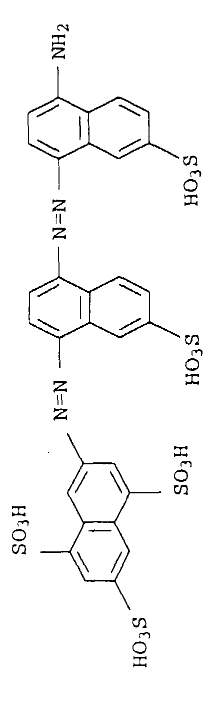 wurden in 750 ml Wasser bei 0 bei 5°C und einem pH-Wert von 5,5 vorgelegt. Anschließend wurden 18,4 g Cyanurchlorid, gelöst in 150 ml Aceton, zugetropft, dabei wurde der pH-Wert durch Zugabe von 5 gew.-%iger wäßriger Natriumhydrogencarbonatlösung zwischen 5,0 und 5,5 gehalten.

Nach vollständiger Umsetzung wurden 50,5 g der Verbindung der Formel 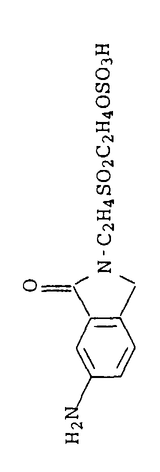 zugegeben und die Temperatur auf 35 bis 40°C erhöht. Es wurde 5 h bei 35 bis 40°C nachgerührt, wobei der pH-Wert mit 5 gew.-%iger wäßriger Natriumhydrogencarbonatlösung zwischen 5,5 und 6,0 gehalten wurde.

Nach Fällung mit 200 g Natriumchlorid, Absaugen und Trocknen wurde der Farbstoff der Formel 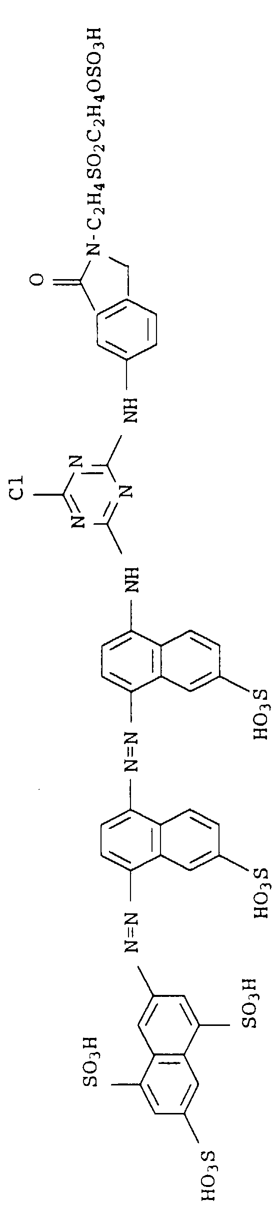 isoliert, der Baumwolle nach den für Reaktivfarbstoffen üblichen Verfahren in rotstichigbraunen Tönen färbt.

In analoger Weise werden die in der folgenden Tabelle 10 aufgeführten Farbstoffe erhalten.

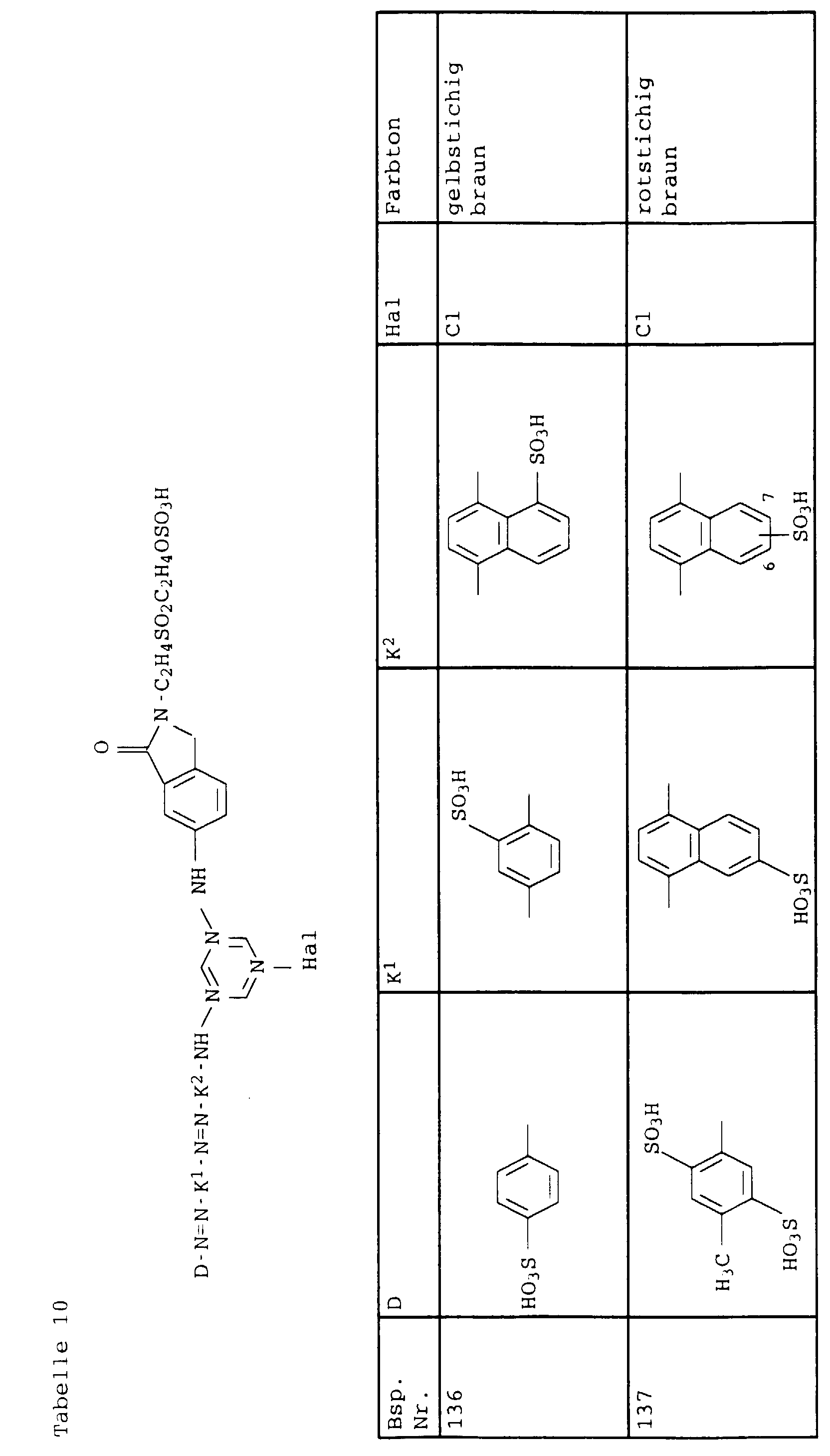

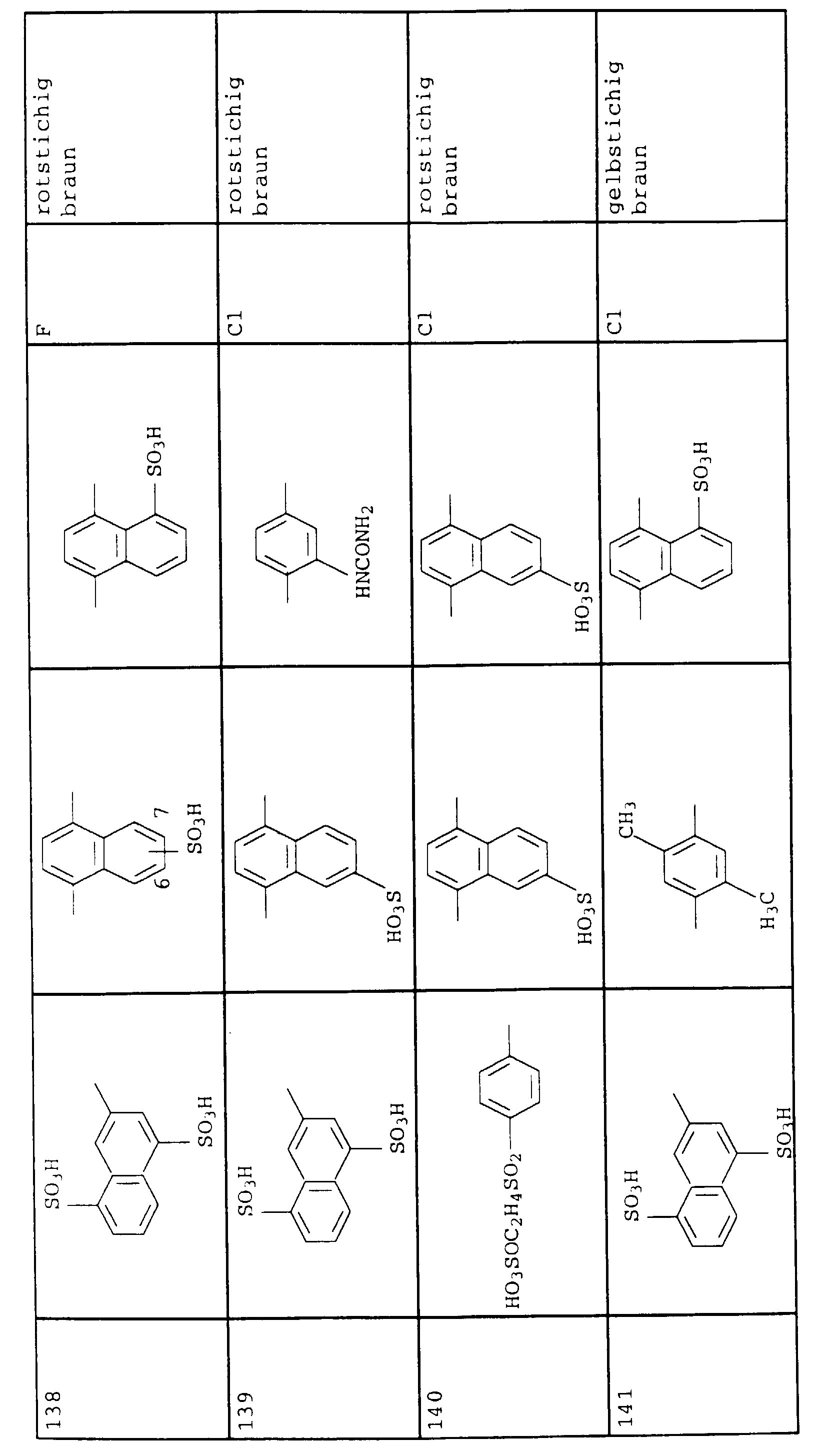

### Beispiel 142

a) 50,6 g der Verbindung der Formel 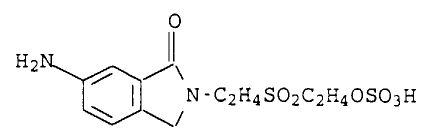 wurden analog Beispiel 104 diazotiert und mit 17,9 g Clevesäure, neutral gelöst in 100 ml Wasser, versetzt. Anschließend wurde der pH-Wert mit gesättigter wäßriger Natriumacetatlösung auf 4,5 bis 5,0 gestellt und 24 h bei Raumtemperatur bis zur vollständigen Kupplung nachgerührt.
   Der pH-Wert wurde mit 18 gew.-%iger Salzsäure erniedrigt. Nach dem Abkühlen auf 0 bis 5°C wurde durch Zugabe von 30 ml 3,33 N wäßriger Natriumnitritlösung, wie in Beispiel 104 beschrieben, diazotiert.
   Nach vollständiger Diazotierung wurden erneut 17,9 g Clevesäure, neutral gelöst in 100 ml Wasser, zugegeben. Anschließend der pH-Wert auf 4,0 erhöht und 25 h bei Raumtemperatur bis zur vollständigen Kupplung nachgerührt.
   Durch Fällung mit Ethanol, Absaugen und Trocknen unter vermindertem Druck wurden 80 g elektrolythaltiger Farbstoff der Formel 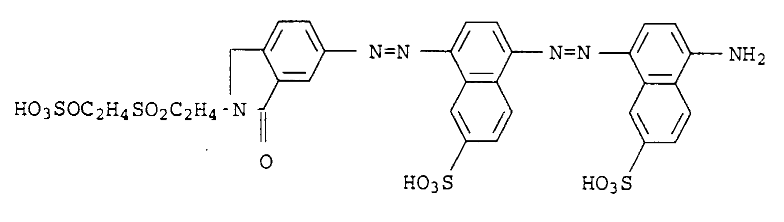 erhalten.
b) 80 g des unter a) beschriebenen Farbstoffs wurden analog Beispiel 125 mit Cyanurchlorid und der Verbindung der Formel 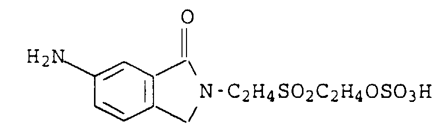 zum Farbstoff der Formel 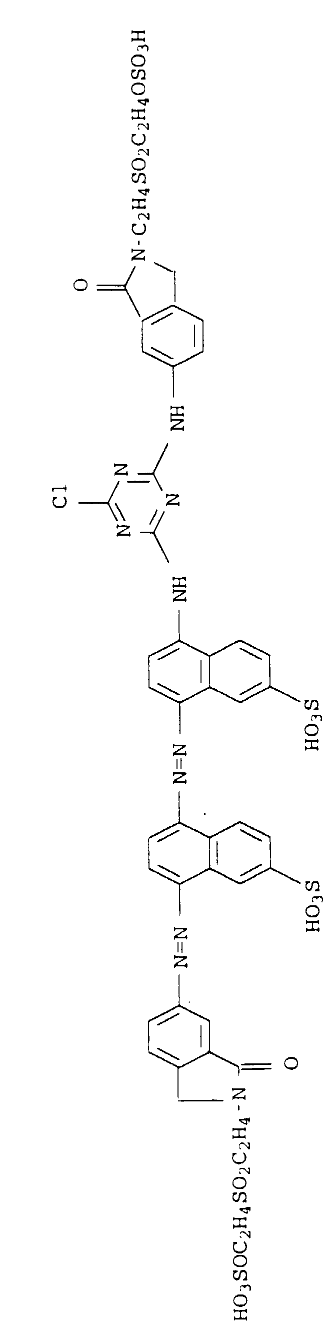 umgesetzt.

Analog Beispiel 142 werden die in der folgenden Tabelle 11 aufgeführten Farbstoffe erhalten.

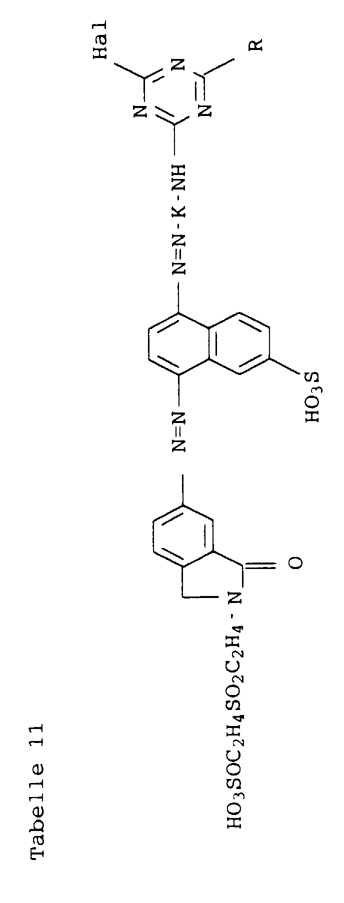

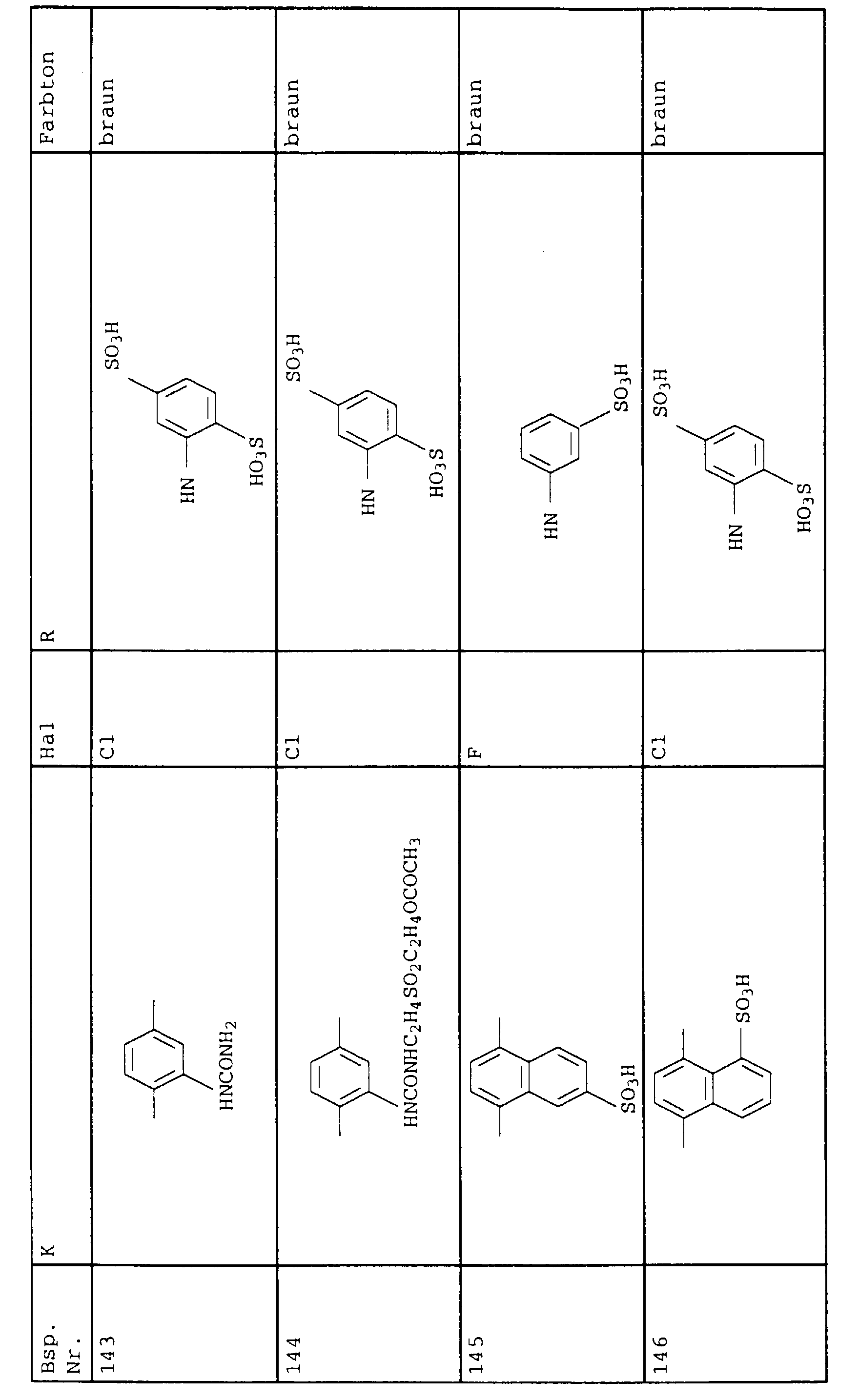

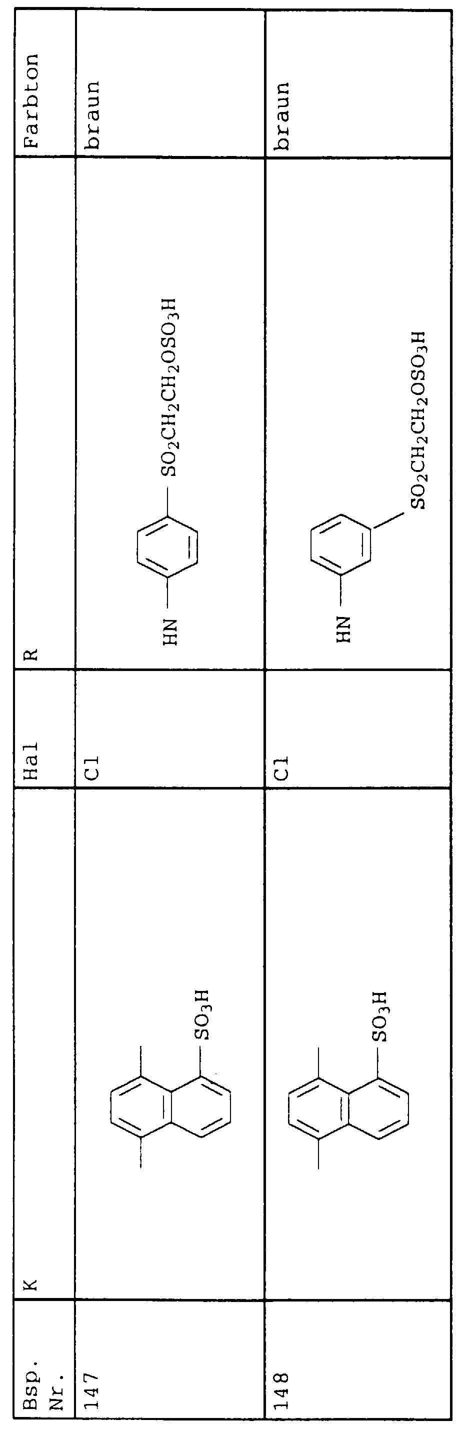

### Beispiel 149

Zu einer Suspension von 75 g des Dichlortriazinfarbstfoffs der Formel 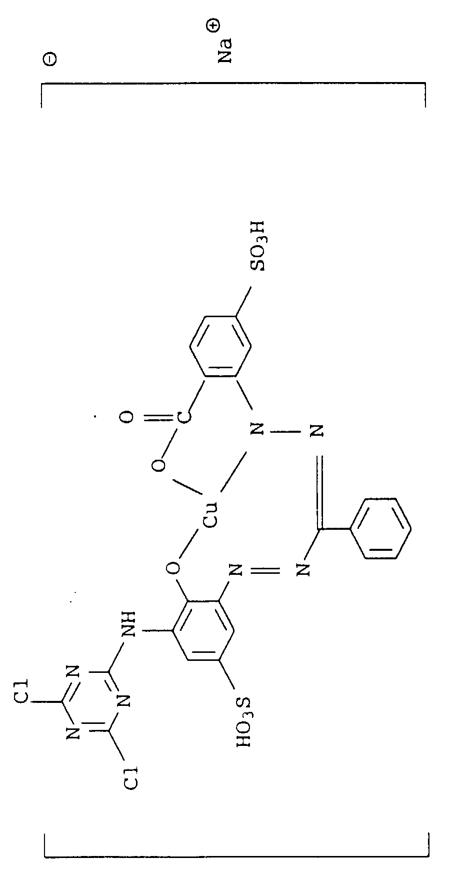 in 600 ml Wasser wurde bei einem pH-Wert von 7 einen Lösung von 40 g der Verbindung der Formel 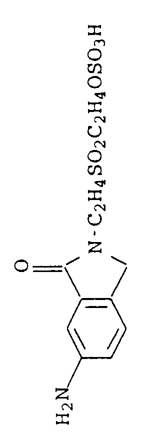 in 600 ml Wasser eingetragen. Die Suspension wurde auf 40 bis 45°C erwärmt und der pH-Wert wurde durch Zusatz von Natriumhydrogencarbonat am Neutralpunkt gehalten. Nach 2,5 h wurde der gebildete Farbstoff der Formel 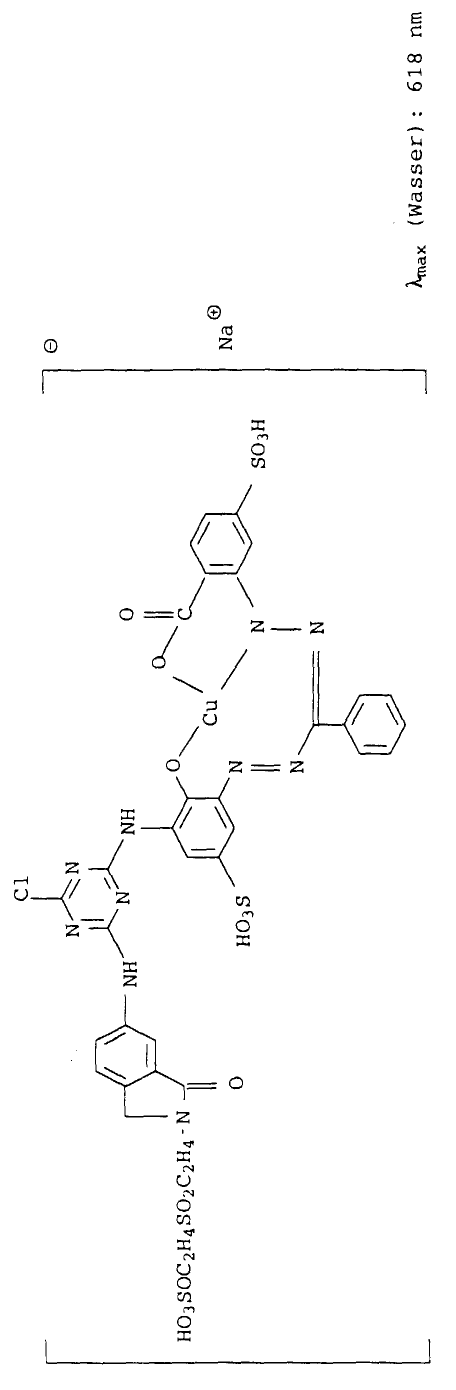 mit 250 g Natriumchlorid ausgesalzen, abfiltriert und getrocknet. Das erhaltene dunkelblaue Farbstoffpulver färbt Baumwolle in klarem blauen Ton. Die Färbungen sind licht- und naßecht, sie weisen eine bemerkenswerte Stabilität gegenüber oxidativen Einflüssen auf.

Analog Beispiel 149 können die in folgender Tabelle 12 aufgeführten Farbstoffe erhalten werden.

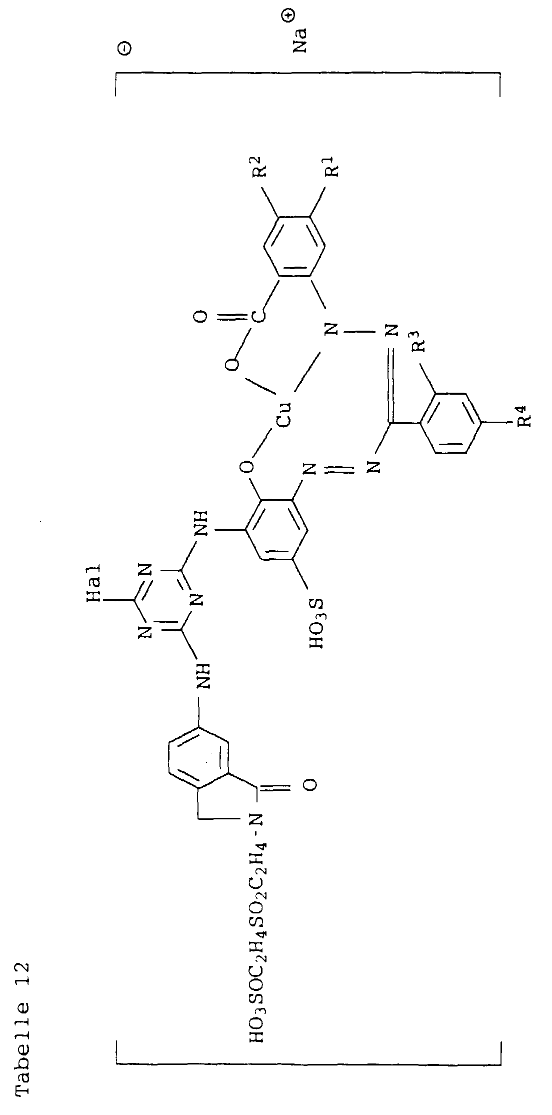

erhalten werden.

| Bsp. Nr. | R¹ | R² | R³ | R⁴ | Hal |
|---|---|---|---|---|---|
| 150 | H | SO₃H | H | H | F |
| 151 | SO₃H | H | H | H | Cl |
| 152 | H | SO₃H | SO₃H | H | Cl |
| 153 | H | SO₃H | H | SO₂CH₂CH₂Cl | Cl |
| 154 | H | NHCOCBr=CH₂ | SO₃H | H | Cl |
| 155 | H | SO₂CH₂CH₂OSO₃H | SO₃H | H | Cl |

### Beispiel 156

19,1 g der Verbindung der Formel 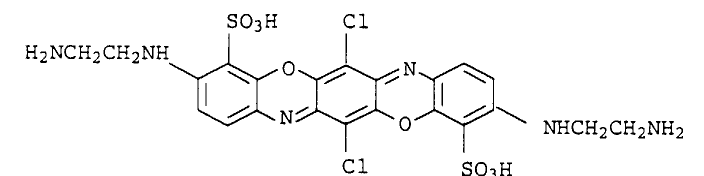 wurden in 1000 ml Wasser eingerührt, wobei mit Natronlauge ein pH-Wert von 10 eingestellt wurde. Diese Lösung wurde in eine 40 bis 50°C warme, auf einen pH-Wert von 6 bis 8 ein gestellte Lösung des Kondensationsproduktes aus 11,1 g Cyanurchlorid mit 24 g der Verbindung der Formel 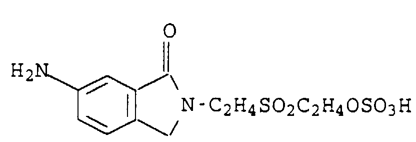 getropft. Unter Aufrechterhaltung eines pH-Wertes von 6,5 bis 7 wurde bei 60°C gerührt bis die Umsetzung beendet war, was etwa 1 h in Anspruch nahm. Nach Abkühlen auf Raumtemperatur wurde mit 500 g Kochsalz ausgesalzen, der ausgefallene Farbstoff abgesaugt und getrocknet. Er färbt Baumwolle in brillantem blauen Ton mit guten Echtheiten und entspricht der Formel

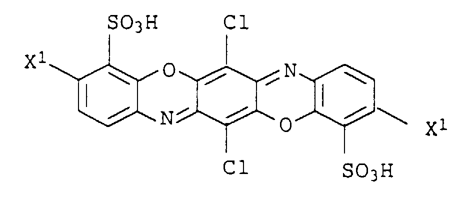

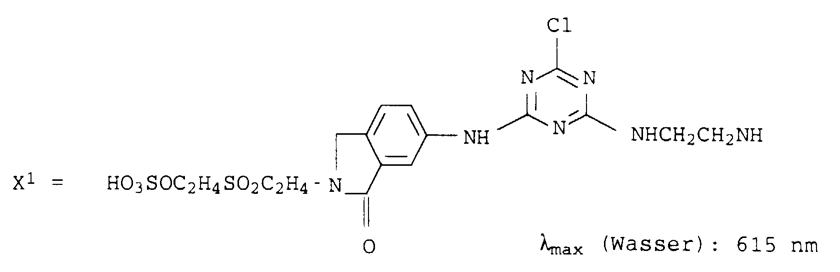

Weitere Farbstoffe, die in analoger Weise erhalten werden, sind in Tabelle 13 aufgeführt.

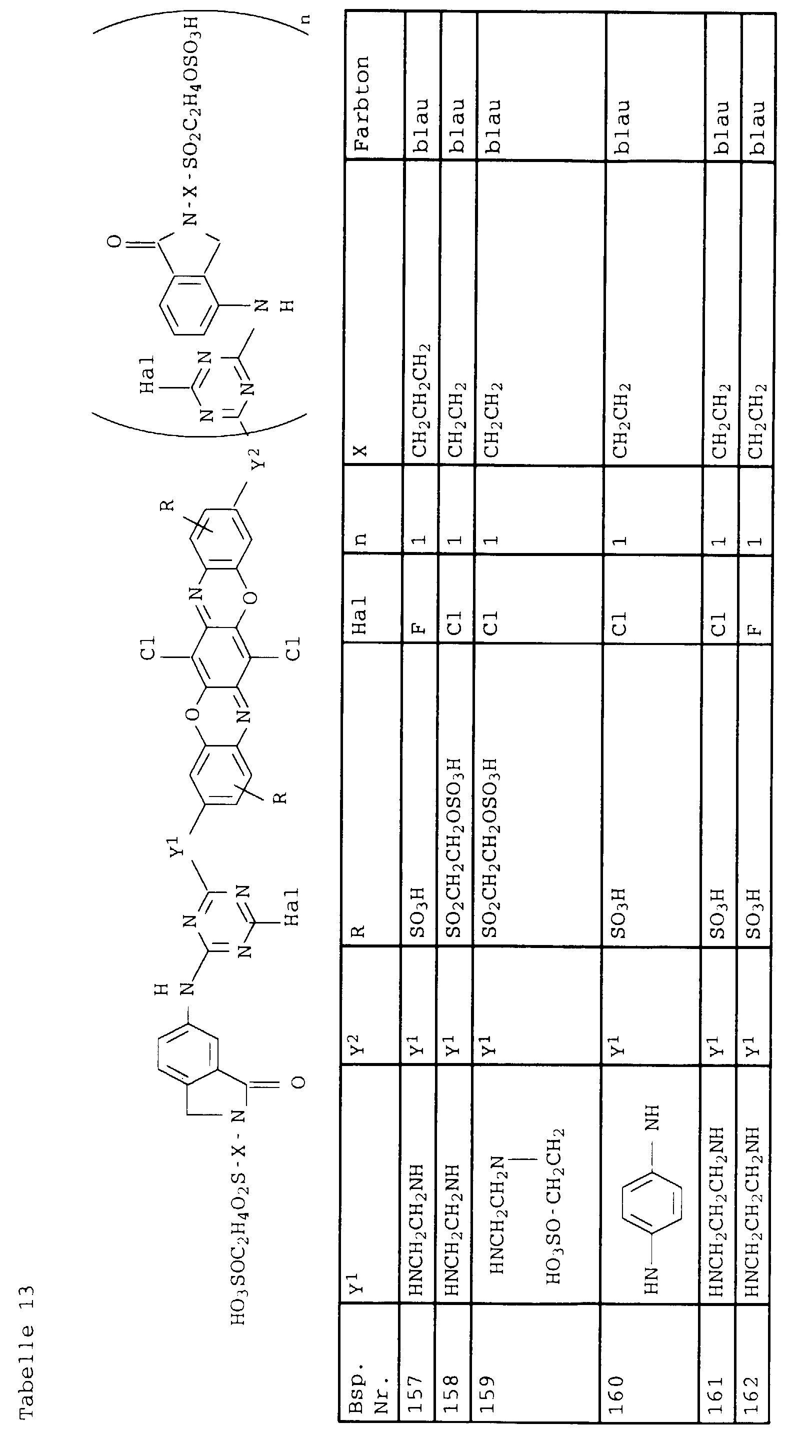

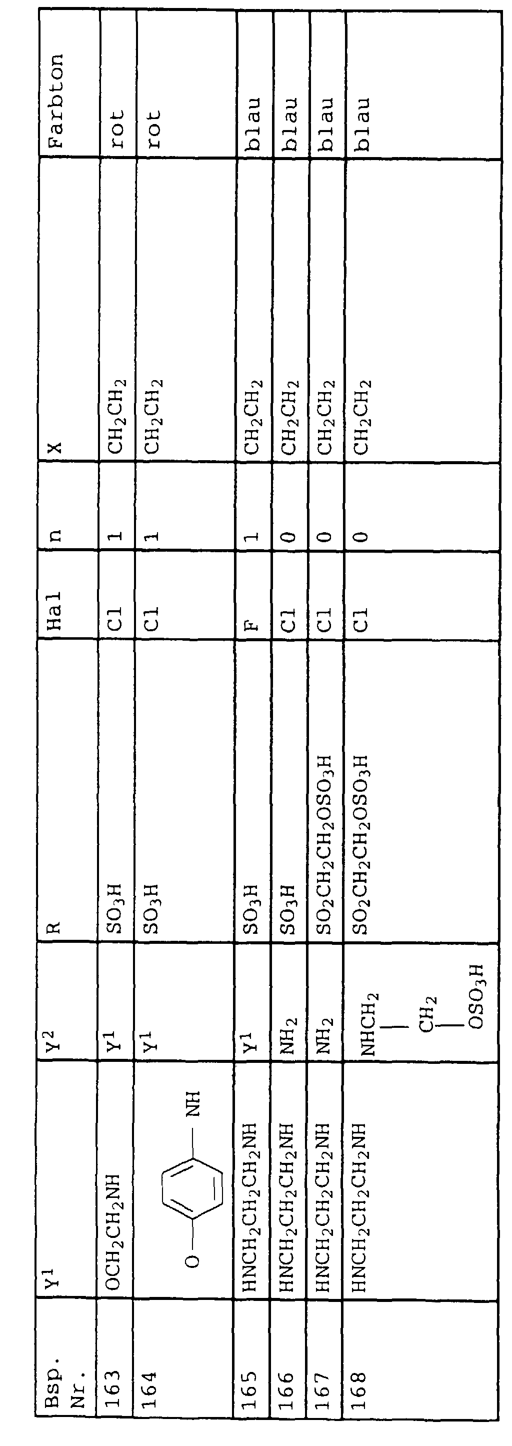

### Beispiel 169

394 g Hydroxyethoxyethylamin und 402 g Phthalid wurden innerhalb 2 h auf 200 bis 210°C erhitzt und das entstandene Wasser abdestilliert. Die Reaktion war nach 3 h beendet. Man destillierte bei 20 bar das überschüssige Amin ab und isolierte 605 g der Verbindung 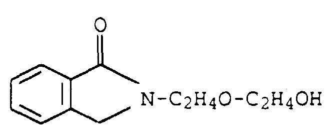 Fp: 72-75°

### Beispiel 170

605 g der Verbindung aus Beispiel 169 wurden in 1000 g Toluol mit 333 g Thionylchlorid auf 60 bis 65°C erhitzt. Man goß nach Abkühlen auf 3000 g Wasser und trennte die organische Phase ab. Nach Abdestillieren des Toluols verblieben 600 g eines Öls. (Cl-Gehalt: 14,5 %; berechnet für 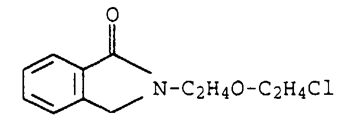 Cl: 14,8 %)

### Beispiel 171

Zu 1000 g n-Brombenzol wurden 292,5 g 2-Mercaptoethanol und 300 g 50%ige Natronlauge 1 h auf 60 bis 70°C erwärmt. Anschließend gab man 718,5 g der Verbindung aus Beispiel 170 zu, wobei die Temperatur auf 90°C anstieg. Nach 3 h bei 95 bis 100°C gab man 1000 g Wasser zu, trennte die Brombenzolphase ab und engte ein. Man isolierte 738 g der Verbindung

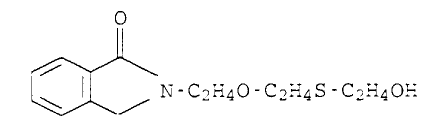

### Beispiel 172

In einer Suspension von 703 g der Verbindung aus Beispiel 171 in 500 g Wasser gab man 2 g Wolframsäure und tropfte bei 65 bis 85°C 567 g einer 30%igen H₂O₂-Lösung zu. Nach beendeter Reaktion wurden 2000 g Ethanol zugegeben und vom Rückstand abfiltriert. Nach Einengen isolierte man 820 g der Verbindung 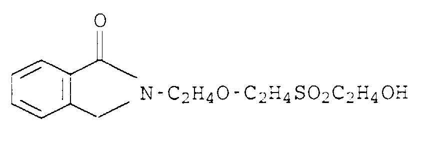 NMR (D₆-DMSO): 3,20 ppm (T,2); 3,40 (M,2); 3,75 (M,8); 4,58 (S,2); 5,07 (T,1); 7,60 (M,4).

### Beispiel 173

485 g aus Beispiel 172 wurden bei 20 bis 40°C in 1519 g 100 %iger Schwefelsäure gelöst. Nach 3 h bei 35 bis 40°C kühlte an auf 0 bis 5°C ab und tropfte eine Lösung aus 107,5 g 100 %ige Salpetersäure in 300 g 96 %iger Schwefelsäure innerhalb 2 h zu. Nach weiteren 5 h wurde der Reaktionsansatz auf 6420 g Eiswasser gegossen. Durch Zugabe von festem Calciumcarbonat wurde der pH-Wert auf 4,0 gestellt. Der Niederschlag wurde abgesaugt und verworfen. Die Mutterlauge wurde bei Temperaturen < 35°C und 14 mbar auf etwa 4000 g eingeengt. Man gab 400 g iso-Propanol, 100 g Eisessig und 5 g eines Pd-Katalysators zu und begaste bei 50 bis 55°C mit Wasserstoff. Nach beendeter Wasserstoffaufnahme wurde vom Katalysator abfiltriert und bei 14 mbar und 35°C auf etwa 1500 ml eingeengt. Die Lösung enthält die Verbindung 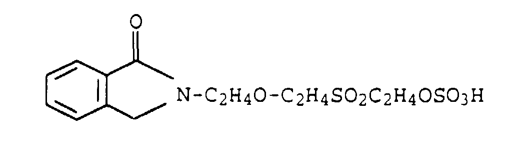 und wird in dieser Form zur Farbstoffsynthese eingesetzt.

## Patentansprüche

1. Reaktivfarbstoffe der Formel I 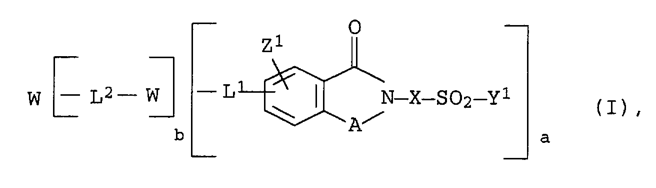 in der
a 1 oder 2,
b 0 oder 1,
Z¹ Wasserstoff, C₁-C₆-Alkyl, C₁-C₆-Alkoxy, Halogen, Nitro, Amino, Hydroxysulfonyl, C₁-C₆-Alkoxycarbonyl, Carbamoyl oder C₁-C₆-Mono- oder Dialkylcarbamoyl,
A Methylen, Sulfonyl oder einen Rest der Formel CH₂-CO oder CH₂-SO₂, wobei die Methylengruppe jeweils an den Benzolring geknüpft ist,
X eine direkte Bindung, C₁-C₈-Alkylen, das gegebenenfalls durch 1 bis 3 Sauerstoffatome in Etherfunktion, 1 bis 3 Iminogruppen oder 1 bis 3 C₁-C₄-Alkyliminogruppen unterbrochen ist, oder einen Rest der Formel L³-CO-NZ²-L⁴, worin L³ und L⁴ unabhängig voneinander jeweils für C₁-C₄-Alkylen und Z² für Wasserstoff, C₁-C₆-Alkyl oder Phenyl stehen, und
Y¹ Vinyl oder einen Rest der Formel C₂H₄Q, worin Q für eine unter alkalischen Reaktionsbedingungen abspaltbare Gruppe steht,
W im Fall 1) den Rest eines Chromophors, der gegebenenfalls weitere reaktive Gruppen aufweist und der sich von einem gegebenenfalls metallisierten Mono- oder Disazofarbstoff, einem Triphendioxazin, einem Anthrachinon, einem metallisierten Formazan oder einem metallisierten Phthalocyanin ableitet, oder
im Fall 2) den Rest einer Kupplungskomponente, an den gegebenenfalls zusätzlich der Rest einer Diazokomponente über eine Azobrücke gebunden ist und der gegebenenfalls zusätzliche reaktive Gruppen aufweist,
L¹ im Fall 1) ein Brückenglied der Formel
O₂S-NZ³, OC-NZ³, Z³N-O₂S, Z³N-OC, Z³N-OC-NZ⁴, NZ³
oder 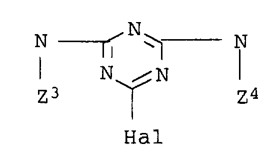 worin Z³ und Z⁴ unabhängig voneinander jeweils für Wasserstoff, C₁-C₆-Alkyl oder Phenyl und Hal für Fluor, Chlor oder Brom oder NZ³ oder NZ⁴ auch für Piperazin-1,4-diyl stehen, oder
im Fall 2) eine Azobrücke und
L² einen Rest der Formel 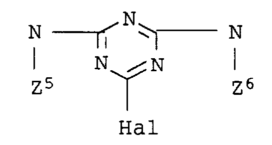 oder 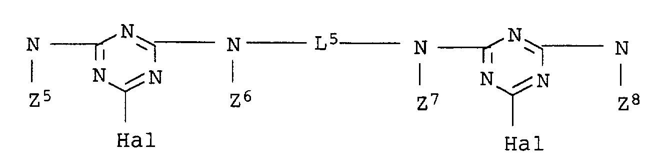 bedeuten, worin Z⁵, Z⁶, Z⁷ und Z⁸ unabhängig voneinander jeweils für Wasserstoff, C₁-C₆-Alkyl oder Phenyl und L⁵ für C₂-C₈-Alkylen oder gegebenenfalls durch C₁-C₄-Alkyl, C₁-C₄-Alkoxy oder Hydroxysulfonyl substituiertes Phenylen stehen und Hal jeweils die obengenannte Bedeutung besitzt.

2. Reaktivfarbstoffe nach Anspruch 1, dadurch gekennzeichnet, daß A Methylen oder den Rest der Formel CH₂-CO bedeutet.

3. Reaktivfarbstoffe nach Anspruch 1, dadurch gekennzeichnet, daß X C₁-C₈-Alkylen, das gegebenenfalls durch 1 bis 3 Sauerstoffatome in Etherfunktion unterbrochen ist, bedeutet.

4. Reaktivfarbstoffe nach Anspruch 1, dadurch gekennzeichnet, daß Z¹ Wasserstoff bedeutet.

5. Schwefelverbindungen der Formel XXVII 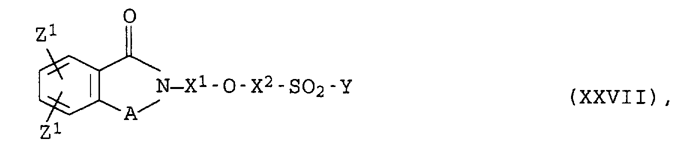 in der
die Reste Z¹ unabhängig voneinander jeweils Wasserstoff, C₁-C₆-Alkyl, C₁-C₆-Alkoxy, Halogen, Nitro, Amino, Hydroxysulfonyl, C₁-C₆-Alkoxycarbonyl, Carbamoyl oder C₁-C₆-Mono- oder Dialkylcarbamoyl,
A Methylen, Sulfonyl oder einen Rest der Formel CH₂-CO oder CH₂-SO₂, wobei die Methylengruppe jeweils an den Benzolring geknüpft ist,
X¹ und X² unabhängig voneinander jeweils C2-C4-Alkylen und
Y Vinyl oder einen Rest der Formel C₂H₄OH oder C₂H₄Q, worin Q für eine unter alkalischen Reaktionsbedingungen abspaltbare Gruppe steht, bedeuten.

6. Schwefelverbindungen nach Anspruch 5, dadurch gekennzeichnet, daß A Methylen oder den Rest der Formel CH₂-CO bedeutet.

7. Schwefelverbindungen nach Anspruch 5, dadurch gekennzeichnet, daß einer der Reste Z¹ Wasserstoff oder Amino und der andere Wasserstoff bedeuten.

8. Verwendung der Reaktivfarbstoffe gemäß Anspruch 1 zum Färben oder Bedrucken von Hydroxygruppen oder Stickstoffatome aufweisenden Substraten.

## Claims

1. A reactive dye of the formula I 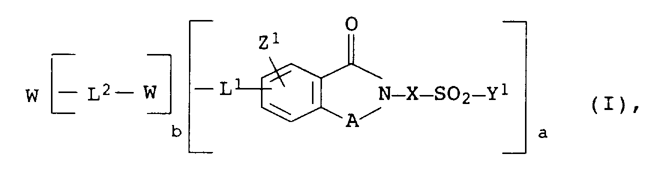 where
a is 1 or 2,
b is 0 or 1,
Z¹ is hydrogen, C₁-C₆-alkyl, C₁-C₆-alkoxy, halogen, nitro, amino, hydroxysulfonyl, C₁-C₆-alkoxycarbonyl, carbamoyl or C₁-C₆-mono- or dialkylcarbamoyl,
A is methylene, sulfonyl or a radical of the formula CH₂-CO or CH₂-SO₂, with the methylene group being linked in each case to the benzene ring,
X is a direct linkage, C₁-C₈-alkylene which may be interrupted via 1 to 3 oxygen atoms in ether functionality, 1 to 3 imino groups or 1 to 3 C₁-C₄-alkylimino groups, or a radical of the formula L³-CO-NZ²-L⁴, where L³ and L⁴ are each, independently of one another, C₁-C₄-alkylene and Z² is hydrogen, C₁-C₆-alkyl or phenyl, and
Y¹ is vinyl or a radical of the formula C₂H₄Q where Q is a group which can be eliminated under alkaline conditions,
W is in case 1) the radical of a chromophore which may have further reactive groups and which is derived from a possibly metallized mono- or disazo dye, a triphendioxazine, an anthraquinone, a metallized formazan or a metallized phthalocyanine, or
in case 2) the radical of a coupling component to which additionally the radical of a diazo component may be linked via an azo bridge and which may have additional reactive groups,
L¹ is in case 1) a linker of the formula
O₂S-NZ³, OC-NZ³, Z³N-O₂S, Z³N-OC, Z³N-OC-NZ⁴, NZ³
or 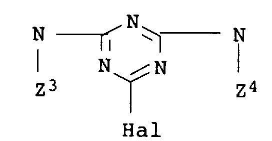 where Z³ and Z⁴ are each, independently of one another, hydrogen, C₁-C₆-alkyl or phenyl and Hal is fluorine, chlorine or bromine or NZ³ or NZ⁴ is also piperazine-1,4-diyl, or
in case 2) an azo bridge and
L² is a radical of the formula 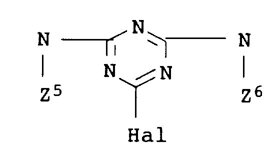 or 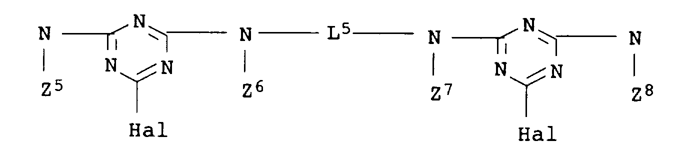 where Z⁵, Z⁶, Z⁷ and Z⁸ are each, independently of one another, hydrogen, C₁-C₆-alkyl or phenyl and L⁵ is C₂-C₈-alkylene or phenylene which is unsubstituted or substituted by C₁-C₄-alkyl, C₁-C₄-alkoxy or hydroxysulfonyl, and Hal has in each case the abovementioned meaning.

2. A reactive dye as claimed in claim 1, wherein A is methylene or the radical of the formula CH₂-CO.

3. A reactive dye as claimed in claim 1, wherein X is C₁-C₈-alkylene which may be interrupted by 1 to 3 oxygen atoms in ether functionality.

4. A reactive dye as claimed in claim 1, wherein Z¹ is hydrogen.

5. A sulfur compound of the formula XXVII 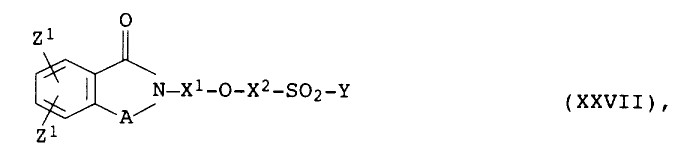 where
the Z¹ radicals are each, independently of one another, hydrogen, C₁-C₆-alkyl, C₁-C₆-alkoxy, halogen, nitro, amino, hydroxysulfonyl, C₁-C₆-alkoxycarbonyl, carbamoyl or C₁-C₆-mono- or dialkylcarbamoyl,
A is methylene, sulfonyl or a radical of the formula CH₂-CO or CH₂-SO₂ where the methylene group is linked in each case to the benzene ring,
X¹ and X² are each, independently of one another, C₂-C₄-alkylene and
Y is vinyl or a radical of the formula C₂H₄OH or C₂H₄Q, where Q is a group which can be eliminated under alkaline conditions.

6. A sulfur compound as claimed in claim 5, wherein A is methylene or the radical of the formula CH₂-CO.

7. A sulfur compound as claimed in claim 5, wherein one of the Z¹ radicals is hydrogen or amino and the other is hydrogen.

8. The use of a reactive dye as claimed in claim 1 for dyeing or printing substrates having hydroxyl groups or nitrogen atoms.

## Revendications

1. Colorants réactifs de formule I 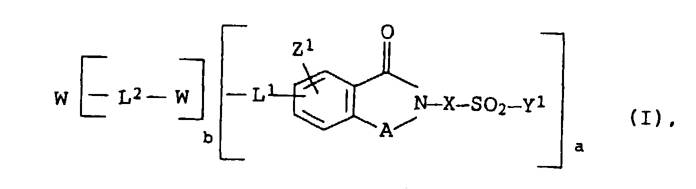 dans laquelle
a vaut 1 ou 2,
b vaut 0 ou 1,
Z¹ représente un atome d'hydrogène, un groupement alkyle en C₁-C₆, alcoxy en C₁-C₆, un atome d'halogène, un groupement nitro, amino, hydroxysulfonyle, (alcoxy en C₁-C₆)carbonyle, carbamoyle ou mono- ou di(alkyle en C₁-C₆)carbamoyle,
A représente un groupement méthylène, sulfonyle ou un reste de formule CH₂-CO ou CH₂-SO₂, où le groupement méthylène est à chaque fois lié au cycle benzénique,
X représente une liaison directe, un groupement alkylène en C₁-C₈, éventuellement interrompu par un à trois atomes d'oxygène en fonction éther, un à trois groupements imino ou un à trois groupements alkylimino en C₁-C₄, ou un reste de formule L³-CO-NZ²-L⁴, où L³ et L⁴ sont mis chacun indépendamment l'un de l'autre pour un groupement alkylène en C₁-C₄ et Z² représente un atome d'hydrogène, un groupement alkyle en C₁-C₆, ou phényle, et
Y¹ représente un groupement vinyle ou un reste de formule C₂H₄Q, où Q est mis pour un groupement séparable dans des conditions réactionnelles alcalines,
W représente
dans le cas 1), le reste d'un chromophore comportant éventuellement d'autres groupements réactifs et dérivant d'un colorant mono- ou disazoïque éventuellement métallé, d'une triphènedioxazine, d'une anthraquinone, d'un formazan métallé ou d'une phtalocyanine métallée, ou
dans le cas 2), le reste d'un composant de couplage, auquel est éventuellement relié en plus le reste d'un composant diazoïque par un pont azo, et qui peut éventuellement comporter des groupements réactifs supplémentaires,
L¹ représente
dans le cas 1), un maillon pontant de formule O₂S-NZ³, OC-NZ³, Z³N-O₂S, Z³N-OC, Z³N-OC-NZ⁴, NZ³ ou 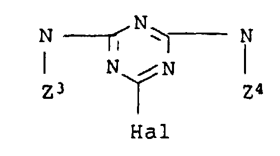 où Z³ et Z⁴ représentent chacun indépendamment de l'autre un atome d'hydrogène, un groupement alkyle en C₁-C₆, ou phényle et Hal est mis pour un atome de fluor, de chlore ou de brome ou NZ³ ou NZ⁴ représentent aussi un groupement pipérazin-1,4-diyle, ou
dans le cas 2), un pont azo et
L² représente un reste de formule 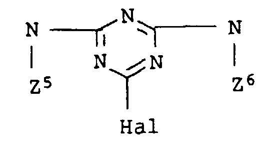 ou 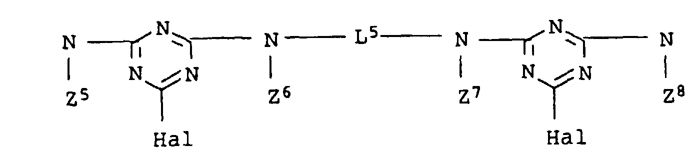 dans lesquelles Z⁵, Z⁶, Z⁷ et Z⁸ représentent chacun indépendamment les uns des autres, un atome d'hydrogène, un groupement alkyle en C₁-C₆, ou phényle, et L⁵ est mis pour un groupement alkylène en C₂-C₈ ou un groupement phénylène éventuellement substitué par des groupements alkyle en C₁-C₄, alcoxy en C₁-C₄, ou hydroxysulfonyle et Hal prend à chaque fois la signification susmentionnée.

2. Colorant réactif selon la revendication 1, caractérisé en ce que A représente un groupement méthylène ou le reste de formule CH₂-CO.

3. Colorant réactif selon la revendication 1, caractérisé en ce que X représente un groupement alkylène en C₁-C₈, éventuellement interrompu par un à trois atomes d'oxygène en fonction éther.

4. Colorant réactif selon la revendication 1, caractérisé en ce que Z¹ représente un atome d'hydrogène.

5. Composés soufrés de formule XXVII 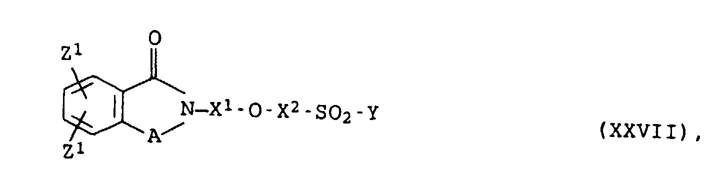 dans laquelle
les restes Z¹ représentent chacun indépendamment les uns des autres, un atome d'hydrogène, un groupement alkyle en C₁-C₆, alcoxy en C₁-C₆, un atome d'halogène, un groupement nitro, amino, hydroxysulfonyle, (alcoxy en C₁-C₆)carbonyle, carbamoyle ou mono- ou di(alkyle en C₁-C₆)carbamoyle,
A représente un groupement méthylène, sulfonyle ou un reste de formule CH₂-CO ou CH₂-SO₂, où le groupement méthylène est à chaque fois lié au cycle benzénique,
X¹ et X² représentent chacun indépendamment l'un de l'autre un groupement alkylène en C2-C4 et
Y représente un groupement vinyle ou un reste de formule C₂H₄OH ou C₂H₄Q, où Q est mis pour un groupement séparable dans des conditions réactionnelles alcalines.

6. Composés soufrés selon la revendication 5, caractérisés en ce que A représente un groupement méthylène ou le reste de formule CH₂-CO.

7. Composés soufrés selon la revendication 5, caractérisés en ce que l'un des restes Z¹ représente un atome d'hydrogène ou un groupement amino et l'autre un atome d'hydrogène.

8. Utilisation de colorants réactifs selon la revendication 1 pour la coloration ou l'impression de substrats comportant des groupements hydroxy ou des atomes d'azote.
